# EUROPEAN PATENT APPLICATION

(11) **EP 4 289 422 A1**
(43) Date of publication of application: **13.12.2023**
(21) Application number: 22749833.4
(22) Date of filing: 04.02.2022
(51) Int. Cl.: A61K 31/192, A61K 31/194, A61K 31/196, A61K 31/4192, A61K 31/421, A61K 31/4745, A61P 17/04, A61P 43/00, C07K 7/08, C12N 15/09

(54) **AGENT FOR SUPPRESSING IL-31 PRODUCTION AND PHARMACEUTICAL COMPOSITION CONTAINING THE SAME**

(30) Priority: 04.02.2021 JP 2021016917
(71) Applicant: Kyushu University, National University Corporation, Nishi-ku Fukuoka-shi Fukuoka 819-0395 (JP); The University of Tokyo, Bunkyo-ku, Tokyo 113-8654 (JP)
(72) Inventor: FUKUI, Yoshinori, Fukuoka-shi, Fukuoka 819-0395 (JP); URUNO, Takehito, Fukuoka-shi, Fukuoka 819-0395 (JP); KANAI, Motomu, Tokyo 113-8654 (JP); OISAKI, Kounosuke, Tokyo 113-8654 (JP); SAIKI, Kuniko, Tokyo 113-8654 (JP)
(74) Representative: Vossius & Partner Patentanwälte Rechtsanwälte mbB
(86) International application number: PCT/JP2022/004558
(87) International publication number: WO 2022/168962

(57) **Abstract**

This invention provides an interleukin-31 (IL-31) production inhibitor and a pharmaceutical composition comprising the inhibitor. The invention also provides a novel compound useful as an active ingredient for an IL-31 production inhibitor. These comprise a compound represented by the following formula (1) or a salt thereof as an active ingredient: 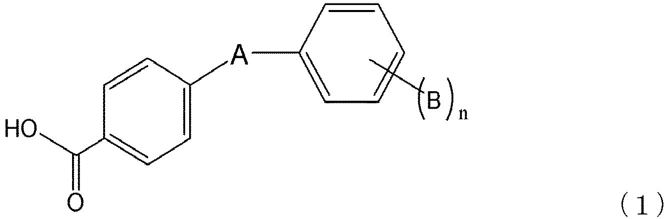 wherein
A is a -NH-N=C(R₁)- group, a divalent N-containing 5-membered heterocyclic group, or a -NH-CO- group, wherein R₁ is a hydrogen atom or a C₁₋₄ alkyl group,
B is the same or different and is a C₁₋₆ alkyl group optionally having one hydroxyl group, a mono or di(C₁₋₆ alkyl)amino group optionally having one hydroxyl group, a halogen atom, a halo C₁₋₆ alkyl group, a C₂₋₆ alkynylene group, or a carboxy group, wherein at least one C₁₋₆ alkyl group in the mono or di (C₁₋₆ alkyl) amino group is optionally attached to a carbon atom adjacent to a carbon atom in the phenyl group to which it is attached, to form a 5- to 6-membered ring, and
n is an integer of 1 to 5.

## Description

### Technical Field

The present invention relates to an interleukin-31 (abbreviated below as "IL-31") production inhibitor and a pharmaceutical composition comprising the inhibitor. The present invention also relates to a novel compound useful as an active ingredient for IL-31 production inhibitors.

### Background Art

Atopic dermatitis (sometimes abbreviated below as "AD") is a chronic inflammatory skin disease characterized by recurrent eczema and intense itch. Itch is a typical symptom observed in many diseases, including AD, and significantly impairs quality of life; thus, addressing this issue is important. Although research on itch has so far focused on histamine, it is known that itch in AD is often not suppressed by H1 histamine receptor blockers.

Itch is induced by a variety of chemical mediators. In particular, IL-31 is known to be a major pruritogen associated with AD (see Non-Patent Literature (NPL) 1). IL-31 is mainly produced by CD4⁺ helper T cells and transmits signals via a heterodimeric receptor composed of IL-31 receptor A (IL-31 RA) and oncostatin M receptor (see NPL 1). Recent clinical studies have found that blocking IL-31 signaling with a specific antibody against IL-31 RA relieves itch in AD patients (see NPL 2). However, a method for treating AD by inhibiting IL-31 production by helper T cells has not yet been established.

Incidentally, it is known that DOCK8 deficiency develops a combined immunodeficiency characterized by AD in humans (see NPL 3). The present inventors previously reported the finding that DOCK8-deficient AND Tg mice (DOCK8-deficient mice expressing AND TCR; sometimes referred to below as "Dock8^{-/-} AND Tg mice") produced by crossing DOCK8-deficient (Dock8^{-/-}) but not Dock8^{+/-} mice with transgenic mice expressing the AND T cell receptor (AND TCR) (referred to as "AND Tg mice") spontaneously develop AD-like skin disease with an increase in serum IL-31 levels (NPL 4). Importantly, CD4⁺ helper T cells from Dock8^{-/-} AND Tg mice produce large amounts of IL-31 when stimulated with an antigen (moth cytochrome C peptide: MCC88-103) in a manner dependent on transcription factor EPAS1 (endothelial PAS domaincontaining protein 1) (NPL 4). Further, it is known that although EPAS1 collaborates with ARNT (aryl hydrocarbon receptor nuclear translocator) to control hypoxic responses, ARNT is not involved in the production of IL-31, and that EPAS1 functions in collaboration with SP1 to control the production of IL-31 (NPL 4). These findings suggest that EPAS1 is a useful drug target for controlling IL-31 production while reducing the risk of adverse effects.

### Citation List

### Non-patent Literature

NPL 1: Furue M, Yamamura K, Kido-Nakahara M, Nakahara T, Fukui Y. Emerging role of interleukin - 31 and interleukin - 31 receptor in pruritus in atopic dermatitis. Allergy 2018;73:29-36.
NPL 2: Kabashima K, Furue M, Hanifin JM, Pulka G, Wollenberg A, Galus R, et al., Nemolizumab in patients with moderate-to-severe atopic dermatitis: Randomized, phase II, long-term extension study. J Allergy Clin Immunol 2018;142:1121-1130.e7.
NPL 3: Zhang Q, Davis JC, Dove CG, Su HC. Genetic, clinical, and laboratory markers for DOCK8 immunodeficiency syndrome. Dis Markers 2010;29:131-9.
NPL 4: Yamamura K, Uruno T, Shiraishi A, Tanaka Y, Ushijima M, Nakahara T, et al. The transcription factor EPAS1 links DOCK8 deficiency to atopic skin inflammation via IL-31 induction. Nat Commun 2017;8:13946.
NPL 5: Kok-Fui L, Kit-Lam C, Chong-Yew L. Eur. J. Med. Chem. 2015, 94, 195.
NPL 6: Tian H, McKnight SL, Russell DW. Endothelial PAS domain protein 1 (EPAS1), a transcription factor selectively expressed in endothelial cells. Genes Dev 1997;11:72-82.
NPL 7: Moreno-Manzano V, Rodríguez-Jiménez FJ, Aceña-Bonilla JL, Fustero-Lardíes S, Erceg S, Dopazo J, et al., FM19G11, a New Hypoxia-inducible Factor (HIF) Modulator, Affects Stem Cell Differentiation Status. J Biol Chem 2010;285:1333-42.
NPL 8: Peng T, Qi B, He J, Ke H, Shi J. Advances in the Development of Phosphodiesterase-4 Inhibitors. J Med Chem 2020;63:10594-10617.

### Summary of Invention

### Technical Problem

An object of the present invention is to provide an IL-31 production inhibitor.

Another object of the present invention is to provide a pharmaceutical composition effective as a therapeutic agent for pruritic diseases, targeting IL-31, which is an itch-inducing substance, in particular, as a therapeutic agent for atopic dermatitis, i.e., an inflammatory disease accompanied by itch. Preferably, an object of the present invention is to provide a pharmaceutical composition having an inhibitory action on IL-31 production by helper T cells and having, based on this action, an effect of reducing itch, which is a characteristic symptom of pruritic diseases. More preferably, an object of the present invention is to provide a pharmaceutical composition having an IL-31 production inhibitory action, and having, based on this action, an itch-reducing effect with fewer adverse effects.

Still another object of the present invention is to provide a novel compound useful as an active ingredient for the pharmaceutical composition, and provide a medicinal use thereof.

### Solution to Problem

As stated above, EPAS1 is a useful drug target for controlling IL-31 production. Therefore, in order to develop a small-molecule compound that can serve as an active ingredient for a therapeutic agent for atopic dermatitis, the present inventors focused on EPAS1-mediated activation of the promoter of IL-31 gene, produced reporter cells that reflect transcriptional activation of IL-31 gene in an Epas1-dependent manner, and constructed an assay system using the cells (luciferase reporter assay).

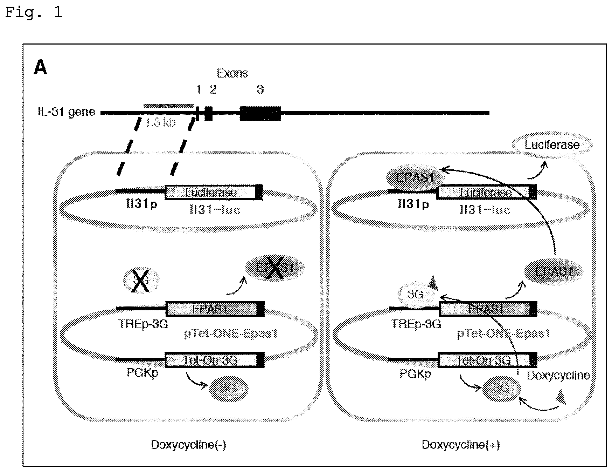

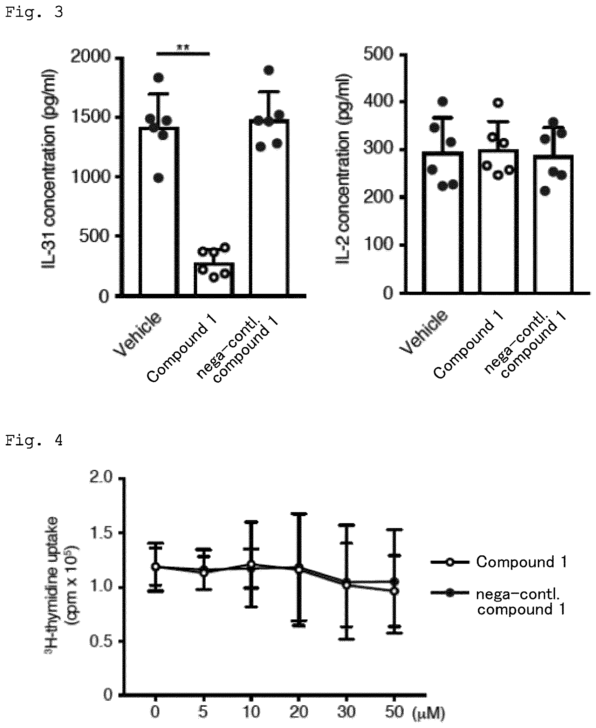

More specifically, the inventors created mouse embryonic fibroblasts (MEFs) into which a construct (Il31-luc) in which a promoter region (approximately 1.3 kb) upstream of Exon 1 of mouse IL-31 gene is connected to a photoprotein luciferase gene, and a pTet-one system (pTet-ONE-Epas1), which can express Epas1 under antibiotic (doxycycline) induction, were incorporated (see Fig. 1). In Fig. 1, 3G refers to a transcription factor that is activated by binding to doxycycline. As shown in Fig. 1, in the absence of doxycycline (Doxycycline(-)), 3G cannot bind to the promoter region that expresses Epas1 (TREp-3G), and Epas1 is not expressed. On the other hand, in the presence of doxycycline (Doxycycline(+)), doxycycline binds to 3G, which then binds to the TREp-3G, inducing Epas1 expression and activating the promoter of IL-31 gene.

Using this assay system, compounds obtained from primary screening of a chemical library consisting of a group of approximately 10,000 compounds (including unpublished compounds) were further subjected to secondary screening using helper T cells from Dock8^{-/-} AND Tg mice. The results confirmed that a specific hydrazone compound (4-(2-(4-isopropylbenzylidene)hydrazineyl)benzoic acid) (which is also referred to below as "compound 1") selectively inhibits IL-31 production at low concentrations without affecting the production of interleukin-2 (IL-2) or interleukin-4 (IL-4).

Based on these findings, the inventors conducted further research to preferably find compounds that selectively suppress IL-31 production based on an IL-31 gene expression inhibitory action, like compound 1. As a result, the present invention, which encompasses the following embodiments, has been completed.

### (1) IL-31 Production Inhibitor

(I-1) An IL-31 production inhibitor comprising a compound represented by the following formula (1) or a salt thereof as an active ingredient: 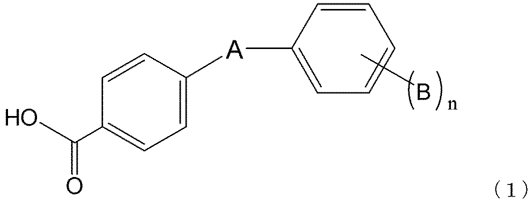 wherein
   A is a -NH-N=C(R₁)- group, a divalent N-containing 5-membered heterocyclic group, a -NH-CO- group, or a group in which a divalent N-containing 5-membered heterocyclic group is linked to a -NH-CO- group, wherein R₁ is a hydrogen atom or a C₁₋₄ alkyl group,
   B is the same or different and is a C₁₋₆ alkyl group optionally having one hydroxyl group, a mono or di(C₁₋₆ alkyl)amino group optionally having one hydroxyl group, a halogen atom, a halo C₁₋₆ alkyl group, a C₂₋₆ alkynylene group, or a carboxy group, wherein at least one C₁₋₆ alkyl group in the mono or di(C₁₋₆ alkyl)amino group is optionally attached to a carbon atom adjacent to a carbon atom in the phenyl group to which it is attached, to form a 5- to 6-membered ring, and
   n is an integer of 1 to 5.
(I-2) The IL-31 production inhibitor according to (I-1), wherein in the compound (1), the phenyl group with a substituent represented by B in the formula (1) has an octanol-water partition coefficient (KLogP) of 1.5 or more.
(I-3) The IL-31 production inhibitor according to (I-1) or (I-2), wherein the compound (1) is a compound represented by the formula (1) in which
   A is a -NH-N=C(R₁)- group, wherein R₁ is a hydrogen atom,
   B is the same or different and is a C₁₋₆ alkyl group, a di(C₁₋₆ alkyl)amino group optionally having one hydroxyl group, a halogen atom, or a halo C₁₋₆ alkyl group, wherein at least one C₁₋₆ alkyl group in the di(C₁₋₆ alkyl)amino group is optionally attached to a carbon atom adjacent to a carbon atom in the phenyl group to which it is attached, to form a 6-membered ring, and n is an integer of 1 to 5.
(I-4) The IL-31 production inhibitor according to (I-1) or (I-2), wherein the compound (1) is a compound represented by the formula (1) in which
   A is a divalent N-containing 5-membered heterocyclic group,
   B is the same or different and is a C₁₋₆ alkyl group, a di (C₁₋₆ alkyl) amino group, a halogen atom, or a halo C₁₋₆ alkyl group, and
   n is an integer of 1 to 5.

### (II) Pharmaceutical Composition with Pharmacological Action Based on IL-31 Production Inhibitory Action

(II-1) A pharmaceutical composition comprising the IL-31 production inhibitor of any one of (I-1) to (I-4) and a pharmaceutically acceptable carrier or excipient.
(II-2) The pharmaceutical composition according to (II-1), which is in an oral administration form.
(II-3) The pharmaceutical composition according to (II-1) or (II-2), which is an anti-pruritic agent.
(II-4) The pharmaceutical composition according to (II-1) or (II-2), which is a therapeutic agent for an IL-31-mediated pruritic disease, preferably an inflammatory disease accompanied by itch, and more preferably atopic dermatitis.

### (III) Hydrazone Compound and Use Thereof

(III-1) A compound represented by the following formula (1) or a salt thereof: 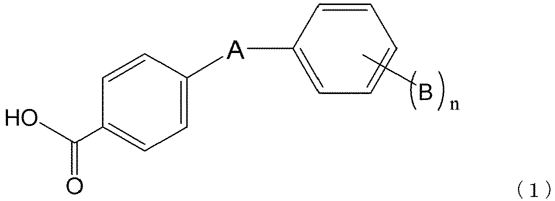 wherein
   A is a -NH-N=C(R₁)- group, a divalent N-containing 5-membered heterocyclic group, a -NH-CO- group, or a group in which a divalent N-containing 5-membered heterocyclic group is linked to a -NH-CO- group, wherein R₁ is a hydrogen atom or a C₁₋₄ alkyl group,
   B is the same or different and is a C₁₋₆ alkyl group optionally having one hydroxyl group, a mono or di(C₁₋₆ alkyl)amino group optionally having one hydroxyl group, a halogen atom, a halo C₁₋₆ alkyl group, a C₂₋₆ alkynylene group, or a carboxy group, wherein at least one C₁₋₆ alkyl group in the mono or di(C₁₋₆ alkyl)amino group is optionally attached to a carbon atom adjacent to a carbon atom in the phenyl group to which it is attached, to form a 5- to 6-membered ring, and
   n is an integer of 1 to 5.
(III-2) The compound or a salt thereof according to (III-1), wherein in the formula (1),
   A is a -NH-N=C(R₁)- group, wherein R₁ is a hydrogen atom,
   B is the same or different and is a C₁₋₆ alkyl group, a di(C₁₋₆ alkyl)amino group optionally having one hydroxyl group, a halogen atom, or a halo C₁₋₆ alkyl group, wherein at least one C₁₋₆ alkyl group in the di(C₁₋₆ alkyl)amino group is optionally attached to a carbon atom adjacent to a carbon atom in the phenyl group to which it is attached, to form a 6-membered ring, and
   n is an integer of 1 to 5.
(III-3) The compound or a salt thereof according to (III-1), wherein in the formula (1),
   A is a divalent N-containing 5-membered heterocyclic group,
   B is the same or different and is a C₁₋₆ alkyl group, a di (C₁₋₆ alkyl) amino group, a halogen atom, or a halo C₁₋₆ alkyl group, and
   n is an integer of 1 to 5.

### (IV) Screening System

A screening system for an IL-31 production inhibitor showing suppression of expression of a reporter protein, wherein a system of expression of the reporter protein induced by an IL-31 gene promoter (Il31p) is introduced in a mouse MEF cell or human cell line (e.g., human HCT116 cell line) in which EPAS1 expression is induced in the presence of doxycycline (doxycycline(+)) .

### Advantageous Effects of Invention

The present invention can provide an IL-31 production inhibitor that has an action of selectively inhibiting IL-31 gene expression and specifically suppressing IL-31 production without affecting the gene expression or production of cytokines, such as IL-2 and IL-4. The IL-31 production inhibitor of the present invention has low cytotoxicity and can thus be usefully used as an active ingredient for a pharmaceutical composition that is applied to mammals, including humans. More specifically, the IL-31 production inhibitor of the present invention is useful as an active ingredient for a therapeutic agent for IL-31-mediated pruritic diseases, in particular, for a therapeutic agent having an effect of relieving or reducing itch. Accordingly, a pharmaceutical composition comprising the IL-31 production inhibitor of the present invention as an active ingredient is useful as a highly safe therapeutic agent for an IL-31-mediated pruritic disease, in particular, as a highly safe therapeutic agent having an effect of relieving or reducing itch.

Furthermore, a novel compound provided by the present invention has an action of selectively inhibiting IL-31 gene expression and specifically suppressing IL-31 production. In addition, the novel compound provided by the present invention is highly safe for mammals, including humans, and is thus useful as an active ingredient for the IL-31 production inhibitor and pharmaceutical composition.

### Brief Description of Drawings

Fig. 1 is a schematic view of the reporter cell (MEF) system used for primary screening of a chemical library (Experimental Example 1). In the presence of doxycycline (Doxycycline(+)), the expression of EPAS1 is induced in MEFs, and the IL-31 gene promoter (Il31 p) is activated.

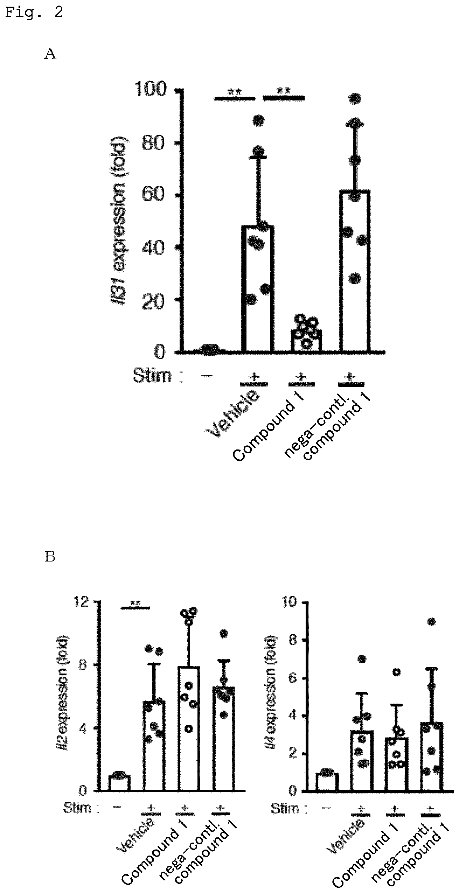

Fig. 2 shows the results of measuring the expression level of the IL-31 gene (Il31 expression) (Fig. 2A) and the expression levels of the IL-2 gene (Il2) and the IL-4 gene (Il4) (Fig. 2B) after culturing CD4⁺ T cells (3 × 10⁵ cells) from Dock8^{-/-} AND Tg mice with T cell-depleted irradiated spleen cells (5 × 10⁶ cells) containing a test compound (compound 1; 4-(2-(4-isopropylbenzylidene)hydrazineyl)benzoic acid), negative control compound 1 (referred to in Fig. 2 as "nega-contl.compound 1"; the same applies to the following figures; 4-(2-(4-(2- (dimethylamino)ethoxy)benzylidene)hydrazineyl)benzoic acid; 2.5 µM), or a vehicle alone (0.1% DMSO), in the presence of MCC88-103 (3 µg/ml) for 24 hours (Experimental Example 1).
Fig. 3 shows the results of treating the CD4⁺ T cells with compound 1 and negative control compound 1 (20 µM) in the presence of MCC88-103 and measuring the amounts of IL-31 and IL-2 produced (pg/ml) by an ELISA assay (Experimental Example 1).
Fig. 4 shows the results of a non-specific T cell proliferation assay using PMA and ionomycin (Experimental Example 1). The horizontal axis represents the amount of each compound (compound 1 and negative control compound 1) added (µM), and the vertical axis represents the amount of radioactivity (cpm × 10⁵) due to ³H-thymidine incorporated in CD4⁺ T cells (Experimental Example 1).
Fig. 5 shows the results of orally administering compound 1 to mice and measuring the concentration (µM) of compound 1 in blood for 24 hours (Experimental Example 2(1)).
Fig. 6 shows the results of scratch behavior measurement (Experimental Example 2(2)). The vertical axis represents the number of scratching bouts during 2 hours.
Fig. 7 shows the results of treating human cancer cell line HT1080 with FM19G11 or compound 1 and evaluating the effect on VEGFA expression and GLUT1 expression induced by hypoxia (Experimental Example 3(1)).
Fig. 8 shows the results of a chromatin immunoprecipitation (ChIP) assay (Experimental Example 3(2)). The vertical axis represents the percentage (%) relative to total DNA.
Fig. 9 shows the results of Experimental Example 4. CD4⁺ T cells of atopic dermatitis (AD) patients produced larger amounts of IL-31 than CD4⁺ T cells of healthy subjects (control), and treatment with compound 1 reduced IL-31 production in a dosedependent manner (Fig. 9A). On the other hand, IL-2 production was not affected (Fig. 9B).

### Description of Embodiments

### (I) IL-31 Production Inhibitor

The IL-31 production inhibitor of the present invention is characterized by comprising a compound represented by the following formula (1) or a salt thereof as an active ingredient.

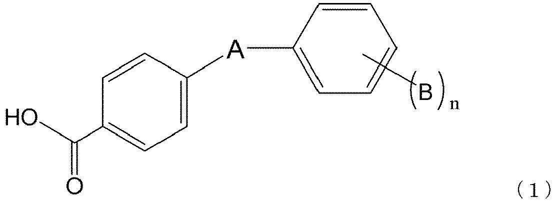

A in formula (1) is a -NH-N=C(R₁)- group, a divalent N-containing 5-membered heterocyclic group, a -NH-CO- group, or a group in which a divalent N-containing 5-membered heterocyclic group is linked to a -NH-CO- group. A is preferably a -NH-N=C(R₁)-group or a divalent N-containing 5-membered heterocyclic group, and more preferably a -NH-N=C(R₁)- group.

In the -NH-N=C(R₁)- group, R₁ may be a hydrogen atom or an alkyl group. The alkyl group is not limited and is preferably a linear or branched C₁₋₆ alkyl group, more preferably a C₁₋₃ alkyl group, and particularly preferably a methyl group. R₁ is preferably a hydrogen atom.

The divalent N-containing 5-membered heterocyclic group may be any divalent 5-membered heterocyclic group containing at least one nitrogen atom in the ring, and is preferably an N-containing 5-membered unsaturated heterocyclic group. The N-containing 5-membered heterocyclic group may also contain a heteroatom, such as an oxygen atom or a sulfur atom, in addition to a nitrogen atom. Examples of 5-membered unsaturated heterocycles containing one nitrogen atom include, but are not limited to, oxazole, isoxazole, pyrrole, and thiazole. Examples of 5-membered unsaturated heterocycles containing two nitrogen atoms include imidazole, pyrazole, oxadiazole, thiadiazole, and furazan. Examples of 5-membered unsaturated heterocycles containing three nitrogen atoms include triazole. The N-containing 5-membered heterocyclic group is preferably one containing only nitrogen as a heteroatom, or containing nitrogen and oxygen as heteroatoms. More preferred are, for example, oxazole and isoxazole with one nitrogen atom and one oxygen atom, imidazole and pyrazole with two nitrogen atoms, triazole with three nitrogen atoms, and oxadiazole with two nitrogen atoms and one oxygen atom.

The two bonds in the divalent N-containing 5-membered heterocyclic group are preferably located at the most distant positions. For example, it is preferable that one bond of the N-containing 5-membered heterocyclic group attached to the p-carboxyphenyl group, which is part of the main skeleton of compound (1), is located at position 1, while the other bond is located at position 3 or 4.

B in formula (1) is the same or different and is an alkyl group optionally having one hydroxyl group, a mono or di(alkyl)amino group optionally having one hydroxyl group, a halogen atom, a haloalkyl group, an alkynylene group, or a carboxy group.

Although not so limited, these substituents are preferably set so that the phenyl group with the substituent has an octanol-water partition coefficient (KLogP) of 1.5 or more. In order for compound (1) to have IL-31 production inhibitory action, the phenyl group with a substituent represented by B preferably has a KLogP of 2 or more, more preferably 2.5 or more, still more preferably 3 or more, and particularly preferably 3.5 or more.

The alkyl group represented by B is not limited and is preferably a linear or branched alkyl group having 1 to 6 carbon atoms (C₁₋₆ alkyl group), and more preferably a C₁₋₄ alkyl group. These alkyl groups include methyl, ethyl, n-propyl, isopropyl, n-butyl, isobutyl, tert-butyl, n-pentyl, and n-hexyl. Preferred examples include, but are not limited to, methyl, isopropyl, and tert-butyl. As long as the phenyl group with an alkyl group as a substituent has a KLogP of 1.5 or more, the alkyl group can optionally have a hydroxyl group. The number of the hydroxyl groups is 1 or less, and preferably 0.

The same as described above applies to the alkyl group in the mono or di(alkyl)amino group represented by B. Preferred is a linear C₁₋₄ alkyl group. The two alkyl groups in the dialkylamino group may be the same or different. The one or two alkyl groups in the mono or di(alkyl)amino group may be each optionally attached to a carbon atom adjacent to a carbon atom in the phenyl group to which the amino group is attached, to form a 5- to 6-membered ring. As long as the phenyl group with the mono or di(alkyl)amino group as a substituent has a KLogP of 1.5 or more, the alkylamino group can optionally have a hydroxyl group. The number of the hydroxyl groups is 1 or less, and preferably 0. Examples of the dialkylamino group optionally having a hydroxyl group include, but are not limited to, a hydroxyethyl(methyl)amino group, a hydroxypropyl(methyl) amino group, a hydroxybutyl(ethyl)amino group, a dimethyl amino group, a diethyl amino group, and a methyl(ethyl)amino group.

The halogen atom represented by B includes fluorine, chlorine, bromine, and iodine. The halogen atom is preferably a fluorine atom or a chlorine atom.

The haloalkyl group represented by B includes a linear or branched C₁₋₆ alkyl group having 1 to 3 halogen atoms. The haloalkyl group is preferably a trihalo C₁₋₆ alkyl group with three halogen atoms. Examples of trihalo C₁₋₆ alkyl groups include, but are not limited to, a trifluoromethyl group, a trichloromethyl group, a 2,2,2-trifluoroethyl group, a 2,2,2-trichloroethyl group, a 3,3,3 trifluoropropyl group, and a 4,4,4-trichlorobutyl group.

The alkynylene group represented by B is preferably, but is not limited to, a linear or branched C₂₋₆ alkynylene group with one triple bond, and preferably a C₂₋₃ alkynylene group. Examples of alkynylene groups include, but are not limited to, ethynyl, 1-propynyl, and 2-propynyl.

In formula (1), n is an integer of 1 to 5. In other words, compound (1) is one in which at least one of the five hydrogen atoms of the phenyl group is substituted with substituent B, which is the same or different. Although not so limited, substituent B on the phenyl group may be located in the ortho-, meta-, or para-position, with respect to group A attached to the benzene ring, as long as the phenyl group with substituent B has a KLogP of 1.5 or more. Although not so limited, for example, when n is 1, substituent B is preferably located in the para-position, and when n is 2, substituents B are preferably located in the meta-position and ortho-position. For example, when n is 2, one of substituents B is preferably a halogen atom while the other is preferably a trihalo C₁₋₆ alkyl group.

In terms of compound (1), the following compounds (i) to (viii) are included as preferred compounds.
(i) A compound represented by formula (1) in which
   A is a -NH-N=C(R₁)- group, wherein R₁ is the same or different and is a hydrogen atom or a C₁₋₄ alkyl group,
   B is the same or different and is a C₁₋₆ alkyl group, a di (C₁₋₆ alkyl)amino group optionally having one hydroxyl group, a halogen atom, or a halo C₁₋₆ alkyl group, wherein at least one C₁₋₆ alkyl group in the di(C₁₋₆ alkyl)amino group is optionally attached to a carbon atom adjacent to a carbon atom in the phenyl group to which it is attached, to form a 5 to 6 member ring, and n is an integer of 1 to 5.
(ii) A compound represented by formula (1) in which
   A is a -NH-N=C(R₁)- group, wherein R₁ is a hydrogen atom,
   B is the same or different and is a C₁₋₄ alkyl group, a di (C₁₋₃ alkyl)amino group optionally having one hydroxyl group, a halogen atom, or a trihalo-C₁₋₃ alkyl group, wherein at least one C₁₋₃ alkyl group in the di(C₁₋₃ alkyl)amino group is optionally attached to a carbon atom adjacent to a carbon atom in the phenyl group to which it is attached, to form a 6-membered ring, and n is an integer of 1 to 5.

   The compounds belonging to groups (i) and (ii) above may be collectively referred to below as "compound A." For compound A, preferred is a compound belonging to group (ii).
(iii) A compound represented by formula (1) in which
   A is a divalent N-containing 5-membered unsaturated heterocyclic group,
   B is the same or different and is a C₁₋₆ alkyl group, a di (C₁₋₆ alkyl)amino group, a halogen atom, or a halo C₁₋₆ alkyl group, and n is an integer of 1 to 5.
(iv) A compound represented by formula (1) in which
   A is a divalent N-containing 5-membered unsaturated heterocyclic group, and preferably oxazole, isoxazole, triazole, imidazole, pyrazole, or oxadiazole,
   B is the same or different and is a C₁₋₄ alkyl group, a di (C₁₋₃ alkyl)amino group, a halogen atom, or a halo C₁₋₃ alkyl group, and n is an integer of 1 to 5.

   The compounds belonging to groups (iii) and (iv) above may be collectively referred to below as "compound B."
(v) A compound represented by formula (1) in which
   A is a -NH-CO- group,
   B is the same or different and is a C₁₋₆ alkyl group, a di (C₁₋₆ alkyl)amino group, a halogen atom, or a halo C₁₋₆ alkyl group, wherein at least one C₁₋₆ alkyl group in the di (C₁₋₆ alkyl) amino group is optionally attached to a carbon atom adjacent to a carbon atom in the phenyl group to which it is attached, to form a 6-membered ring, and
   n is an integer of 1 to 5.
(vi) A compound represented by formula (1) in which
   A is a -NH-CO- group,
   B is the same or different and is a C₁₋₄ alkyl group, a di (C₁₋₃ alkyl)amino group, a halogen atom, or a halo C₁₋₃ alkyl group, and n is an integer of 1 to 5.

   The compounds belonging to groups (v) and (vi) above may be collectively referred to below as "compound C."
(vii) A compound represented by formula (1) in which
   A is a group in which a divalent N-containing 5-membered unsaturated heterocyclic group is linked to a -NH-CO- group,
   B is the same or different and is a C₁₋₆ alkyl group, a di (C₁₋₆ alkyl)amino group, a halogen atom, or a halo C₁₋₆ alkyl group, and n is an integer of 1 to 5.
(viii) A compound represented by formula (1) in which
   A is a -NH-CO- group,
   B is a C₁₋₄ alkyl group, and
   n is 1.

The compounds belonging to groups (vii) and (viii) above may be collectively referred to below as "compound D."

In terms of compound (1), the following compounds are included as particularly preferred compounds.

Compound A:
Compound 1: 4-(2-(4-isopropylbenzylidene)hydrazinyl)benzoic acid,
Compound 2: 4-(2-(4-(tert-butyl)benzylidene)hydrazinyl)benzoic acid,
Compound 3: 4-(2-(4-(dimethylamino)benzylidene)hydrazinyl)benzoic acid,
Compound 4: 4-(2-(4-((2-hydroxyethyl)(methyl)amino)benzylidene)hydrazinyl)benzoic acid,
Compound 5: 4-(2-(4-((3-hydroxypropyl)(methyl)amino)benzylidene)hydrazinyl)benzoic acid,
Compound 6: 4-(2-(4-(ethyl(4-hydroxybutyl)amino)benzylidene)hydrazinyl)benzoic acid,
Compound 7: 4-(2-((2,3,6,7-tetrahydro-1H,5H-pyrido[3,2,1-ij]quinolin-9-yl)methylene)hydrazinyl)benzoic acid,
Compound 8: 4-(2-(4-fluoro-2-methylbenzylidene)hydrazinyl)benzoic acid,
Compound 9: 4-(2-((perfluorophenyl)methylene)hydrazinyl)benzoic acid,
Compound 10: 4-(2-(2-chloro-5-(trifluoromethyl)benzylidene)hydrazinyl)benzoic acid,
Compound 11: 4-(2-(2-fluoro-5-(trifluoromethyl)benzylidene)hydrazinyl)benzoic acid,
Compound 12: 4-(2-(4-ethynylbenzylidene)hydrazinyl)benzoic acid,
Compound 13: 3-((2-(4-carboxyphenyl)hydrazono)methyl)benzoic acid, and
Compound 18: 4-(2-(1-(4-isopropylphenyl)ethylidene)hydrazinyl)benzoic acid.

### Compound B:

Compound 14: 4-(5-(4-(tert-butyl)phenyl)oxazol-2-yl)benzoic acid,
Compound 15: 4-(5-(2-chloro-5-(trifluoromethyl)phenyl)oxazol-2-yl)benzoic acid,
Compound 16: 4-(1-(4-(tert-butyl)phenyl)-1H-1,2,3-triazol-4-yl)benzoic acid,
Compound 19: 4-(5-(4-(dimethylamino)phenyl)oxazol-2-yl)benzoic acid,
Compound 20: 4-(2-(2-chloro-5-(trifluoromethyl)phenyl)oxazol-5-yl)benzoic acid,
Compound 21: 4-(3-(2-chloro-5-(trifluoromethyl)phenyl)isoxazol-5-yl)benzoic acid,
Compound 22: 4-(1-(4-isopropylphenyl)-1H-1,2,3-triazol-4-yl)benzoic acid,
Compound 23: 4-(1-(2-chloro-5-(trifluoromethyl)phenyl)-1H-1,2,3-triazol-4-yl)benzoic acid,
Compound 24: 4-(1-(2-fluoro-5-(trifluoromethyl)phenyl)-1H-1,2,3-triazol-4-yl)benzoic acid,
Compound 25: 4-(3-(2-chloro-5-(trifluoromethyl)phenyl)-1H-1,2,4-triazol-5-yl)benzoic acid,
Compound 26: 4-(5-(4-isopropylphenyl)-1H-imidazol-2-yl)benzoic acid,
Compound 27: 4-(5-(2-chloro-5-(trifluoromethyl)phenyl)-1H-imidazol-2-yl)benzoic acid
Compound 28: 4-(5-(4-isopropylphenyl)-1H-pyrazol-3-yl)benzoic acid,
Compound 29: 4-(5-(2-chloro-5-(trifluoromethyl)phenyl)-1H-pyrazol-3-yl)benzoic acid,
Compound 30: 4-(3-(2-chloro-5-(trifluoromethyl)phenyl)-1H-pyrazol-1-yl)benzoic acid,
Compound 31: 4-(5-(4-isopropylphenyl)-1,3,4-oxadiazol-2-yl)benzoic acid,
Compound 32: 4-(5-(2-chloro-5-(trifluoromethyl)phenyl)-1,3,4-oxadiazol-2-yl)benzoic acid, and
Compound 33: 4-(3-(2-chloro-5-(trifluoromethyl)phenyl)-1,2,4-oxadiazol-5-yl)benzoic acid.

### Compound C:

Compound 17: 4-(4-isopropylbenzamido)benzoic acid.

### Compound D:

Compound 34: 4-(5-(4-isopropylbenzamido)-1,3,4-thiadiazol-2-yl)benzoic acid.

Compound (1) mentioned above can be in the form of a pharmaceutically acceptable salt, which can be used in the same manner as with compound (1) in a free form. Pharmaceutically acceptable salts include, but are not limited to, alkali metal salts, such as sodium and potassium; alkaline earth metal salts, such as calcium and magnesium; and salts with cations of nontoxic ammonium, quaternary ammonium, and amine, such as ammonium, tetramethylammonium, tetraethylammonium, methylamine, dimethylamine, trimethylamine, triethylamine, and ethylamine. When compound (1) above includes isomers, such as stereoisomers and optical isomers, these isomers are also encompassed by an embodiment of compound (1) of the present invention. Compound (1) can also be in the form of a solvate, and the solvate is also encompassed by an embodiment of compound (1) of the present invention.

Compound (1) above or a salt thereof can be produced according to Production Examples described below. In particular, Production Example 1, which is a production method for compound 1, is a typical method for synthesizing a hydrazone compound, typified by compound 1. Therefore, hydrazone compounds belonging to the compound A group, in which A in formula (1) is a -NH-N=C(R₁)- group as in compound 1, can be produced with reference to this production method. Further, Production Example 14, which is a production method for compound 14, is a typical method for synthesizing a compound having an N-containing heterocyclic group, typified by compound 14. Therefore, compounds belonging to the compound B group, in which A in formula (1) is a divalent N-containing heterocyclic group as in compound 14, can be produced with reference to this production method. Further, compounds belonging to the compound C group, in which A in formula (1) is a -NH-CO- group as in compound 17, can be produced with reference to Production Example 17, which is a production method for compound 17. Furthermore, compounds belonging to the compound D group, in which A in formula (1) is a group in which a divalent N-containing heterocyclic group is linked to a -NH-CO- group as in compound 34, can be produced with reference to Production Example 41, which is a production method for compound 34.

Compound (1) obtained by these production methods is isolated and/or purified from the reaction mixture by using known isolation and/or purification techniques. Examples of such separation and/or purification techniques include distillation, recrystallization, solvent extraction, column chromatography, ion exchange chromatography, gel chromatography, affinity chromatography, and preparative thin layer chromatography.

Compound (1) or a salt thereof has an action of selectively suppressing EPAS1-induced IL-31 gene expression and IL-31 production. On the other hand, compound (1) or a salt thereof does not have an action of suppressing the gene expression or production of other cytokines, such as IL-2 and IL-4. Accordingly, compound (1) or a salt thereof is useful as a selective IL-31 production inhibitor.

The term "IL-31 production inhibitor" as used in the present invention encompasses the meanings of suppression of IL-31 gene expression and suppression of IL-31 production. The IL-31 production inhibitor, which is the subject of the present invention, preferably has an IL-31 gene expression inhibitory action and consequently has an IL-31 production inhibitory effect.

The IL-31 production inhibitor according to the present invention may consist only of compound (1) described above or a salt thereof, or may comprise other components, such as additives usually used in the related fields (e.g., the fields of biochemistry and pharmaceuticals). The specific additives and the proportions of the additives in the IL-31 production inhibitor are not limited as long as the IL-31 production inhibitor has an IL-31 production inhibitory action, based on compound (1) or a salt thereof. For example, additives and their proportions may be suitably selected with reference to those described for the pharmaceutical composition described below.

### (II) Pharmaceutical Composition

The pharmaceutical composition according to the present invention comprises compound (1) described above or a pharmaceutically acceptable salt thereof. The pharmaceutically acceptable salt can be suitably selected with reference to the detailed descriptions above for compound (1).

The pharmaceutical composition according to the present invention may consist only of compound (1) or a pharmaceutically acceptable salt thereof, or may be prepared in combination with any carrier or additive to be in a form suitable for desired use, such as administration route and administration method, by known methods. The pharmaceutical composition according to the present invention is preferably in a form suitable for intravenous or oral administration, and more preferably oral administration.

Specific dosage forms include tablets, pills, powders, liquids, suspensions, emulsions, granules, capsules, suppositories, injections (e.g., solutions and suspensions), and drops. Preferred are dosage forms suitable for oral administration, such as tablets, pills, powders, liquids, suspensions, emulsions, granules, and capsules.

The amount of compound (1) or a pharmaceutically acceptable salt thereof incorporated in the pharmaceutical composition according to the present invention is not limited as long as the IL-31 production inhibitory action is achieved based on compound (1) or a salt thereof. For example, the amount can be suitably adjusted within the range of about 0.001 to 99 mass% or less, preferably about 0.01 to 50 mass%, and more preferably about 0.05 to 10 mass%, per 100 mass% of the pharmaceutical composition.

The diseases targeted for treatment by the pharmaceutical composition according to the present invention are IL-31-mediated pruritic diseases. Preferred are inflammatory diseases accompanied by itch, in particular, skin diseases accompanied by itch. Specific examples include atopic dermatitis, dermatomyositis, chronic dermatitis, allergic contact dermatitis, dermatitis herpetiformis, pruritus in cutaneous T-cell lymphoma, prurigo nodularis, prurigo chronica multiformis, urticaria pigmentosa, and bullous pemphigoid. Preferred is atopic dermatitis. Atopic dermatitis is typically classified into infant atopic dermatitis, pediatric atopic dermatitis, adult atopic dermatitis, and maternal atopic dermatitis according to the time of onset or according to the subject developing the disease. The atopic dermatitis targeted by the present invention includes all these types of atopic dermatitis.

The pharmaceutical composition of the present invention can be suitably used for the treatment of these diseases. In the present invention, the term "treatment" means, in particular, relieving and curing itch among the symptoms of the diseases mentioned above. The meanings of the term also include not only complete elimination but also partial elimination (relief, reduction, etc.) of itch. The subject of the treatment by the pharmaceutical composition of the present invention is a human with a disease mentioned above. As stated above, the human subject is not limited and may be an infant, a child, an adult, or a pregnant woman.

The daily administration amount, time of administration, and frequency of administration of the pharmaceutical composition according to the present invention are not limited as long as the amount, time, and frequency are effective for the treatment of the diseases mentioned above. For example, although not so limited, the administration may be usually performed in an amount in the range of about 0.1 mg to 1 mg/person per day in terms of compound (1) or a pharmaceutically acceptable salt thereof, once to multiple times per day.

In the present specification, the terms "comprise," "include," and "contain" include the meanings of consisting of and consisting essentially of.

### Examples

The present invention is described below with reference to Experimental Examples in order to facilitate understanding of the configuration and effects of the present invention. However, the present invention is not limited by these Experimental Examples. The following experiments were performed at room temperature (25±5°C) under atmospheric pressure conditions, unless otherwise specified. Below, "%" means "mass%," and "part" means "parts by mass" unless otherwise specified.

### Production Examples

### Introduction

¹H NMR spectra were recorded on a JEOL ECX500 spectrometer (500 MHz for ¹H NMR and 125.65 MHz for ¹³C NMR) and a JEOL ECS400 spectrometer (400 MHz for ¹H NMR, 100 MHz for ¹³C NMR, and 370 MHz for ¹⁹F NMR) (JEOL Ltd.) . ¹H NMR and ¹³C NMR chemical shifts were reported in the scale relative to CDCl₃ (¹H NMR: δ = 7.26 ppm, ¹³C NMR: δ = 77.16 ppm), DMSO-d₆ (¹H NMR: δ = 2.50 ppm, ¹³C NMR: δ = 39.52), CD₃OD (¹H NMR: δ = 3.31 ppm, ¹³C NMR: δ = 49.00 ppm), and acetone-d₆ (¹H NMR: δ = 2.05 ppm, ¹³C NMR: δ = 29.84 ppm) as internal standards. ¹⁹F NMR chemical shifts were reported relative to hexafluorobenzene (δ = -164.90 ppm) as an external standard. Electrospray ionization (ESI)-mass spectra were recorded on a Bruker ESI-TOF MS microTOFII spectrometer for HRMS. Infrared (IR) spectra were recorded on a JASCO FT/IR410 Fourier transform infrared spectrophotometer. Column chromatography was performed using Biotage (registered trademark) Isolera (trademark) One 3.0 with a Biotage (registered trademark) SNAP Ultra (Uppsala, Sweden) prepacked column.

Commercially available products were used for chemicals and solvents. Humidity-sensitive reactions were all performed in an argon atmosphere. Compounds for use were dissolved in 100% dimethyl sulfoxide (DMSO) (produced by FUJIFILM Wako Pure Chemical Corporation) and promptly diluted with an appropriate buffer.

### Production Example 1

### 4-(2-(4-Isopropylbenzylidene)hydrazinyl)benzoic acid (compound 1)

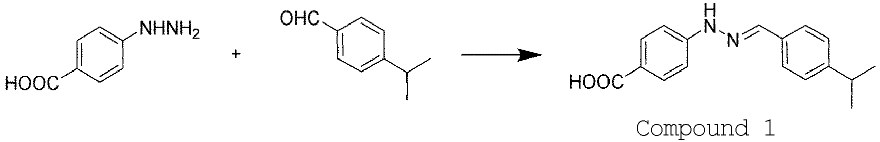

A solution of 4-hydrazineylbenzoic acid (1.00 g, 6.56 mmol, 1.00 equiv) and 4-isopropylbenzaldehyde (1.02 g, 6.90 mmol, 1.05 equiv) in acetic acid (20 mL) was stirred at room temperature overnight. The resulting liquid mixture was cooled, and the resulting precipitate was collected by filtration. The solid was washed with cold acetic acid to give 4-(2-(4-isopropylbenzylidene)hydrazinyl)benzoic acid as a beige solid (1.22 g, 4.34 mmol, 66% yield).

¹H NMR (400 MHz, DMSO-d₆) δ 12.30 (1H, br), 10.74 (1H, s), 7.92 (1H, s), 7.81 (2H, d, J = 8.6 Hz), 7.59 (2H, d, J = 8.2 Hz), 7.26 (2H, d, J = 8.2 Hz), 7.09 (2H, d, J = 8.6 Hz), 2.88 (1H, m), 1.19 (6H, d, J = 7.4 Hz);
¹³C NMR (500 MHz, DMSO-d₆) δ 167.3, 149.1, 148.9, 139.0, 133.0, 131.2, 126.7, 126.1, 120.1, 111.1, 33.3, 23.8;
FT-IR (KBr) 3313, 2957, 1671, 1597, 1428, 1266, 1168 cm⁻¹;
HRMS (ESI-TOF) m/z: [M + Na]⁺ Calcd for C₁₇H₁₈N₂O₂Na 305.1260; Found 305.1262.

The production method described above is a typical method for synthesizing a hydrazone compound. The following hydrazone compounds were produced according to the procedure described in Production Example 1.

### Production Example 2

### 4-(2-(4-(tert-Butyl)benzylidene)hydrazinyl)benzoic acid (compound 2)

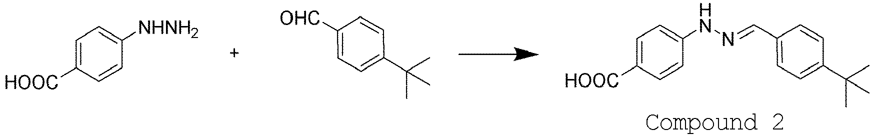

4-(2-(4-(tert-Butyl)benzylidene)hydrazinyl)benzoic acid was obtained as a beige solid (45.5 mg, 0.153 mmol, 82% yield) after purification by column chromatography (SiO₂, SNAP ultra (10 g), EtOAc/hexane = 50% → 100%) in the same manner as in the procedure described in Production Example 1, except that 4-(tert-butyl)benzaldehyde (30.1 mg, 0.186 mmol) was used in place of 4-isopropylbenzaldehyde.

¹H NMR (400 MHz, CD₃OD) δ 7.89 (2H, d, J = 8.7 Hz), 7.82 (1H, s), 7.59 (2H, d, J = 8.5 Hz), 7.42 (2H, d, J = 8.5 Hz), 7.10 (2H, d, J = 8.7 Hz), 1.33 (9H, s);
LRMS (ESI+) m/z 297 [M+H]⁺.

### Production Example 3

### 4-(2-(4-(Dimethylamino)benzylidene)hydrazinyl)benzoic acid (compound 3)

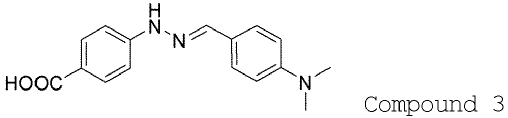

4-(2-(4-(Dimethylamino)benzylidene)hydrazinyl)benzoic acid was obtained as a yellow solid (23.4 mg, 0.082 mmol, 44% yield) in the same manner as in the procedure described in Production Example 1, except that 4-(dimethylamino)benzaldehyde (27.75 mg, 0.186 mmol) was used in place of 4-isopropylbenzaldehyde.

¹H NMR (500 MHz, DMSO-d₆) δ 12.19 (1H, br), 10.48 (1H, s), 7.84 (1H, s), 7.79 (2H, d, J = 8.5 Hz), 7.50 (2H, d, J = 8.5 Hz), 7.04 (2H, d, J = 8.5 Hz), 6.72 (2H, d, J = 8.5 Hz), 2.93 (6H, s);
¹³C NMR (500 MHz, DMSO-d₆) δ 167.6, 150.8, 149.4, 140.4, 131.4, 127.5, 123.1, 119.5, 112.2, 110.9, 40.0; FT-IR (KBr) 3299, 2886, 1669, 1612, 1521, 1290, 1167 cm⁻¹;
HRMS (ESI-TOF) m/z: [M + Na]⁺ Calcd for C₁₆H₁₇N₃O₂Na 306.1213; Found 306.1207.

### Production Example 4

### 4-(2-(4-((2-Hydroxyethyl)(methyl)amino)benzylidene)hydrazinyl)benzoic acid (compound 4)

### (1) Production of 4-((2-hydroxyethyl)(methyl)amino)benzaldehyde

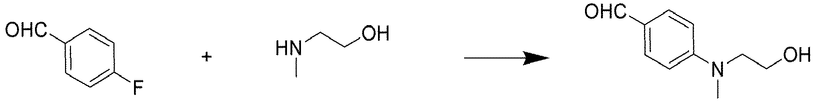

4-Fluorobenzaldehyde (862 mg, 11.48 mmol, 1.5 equiv), sodium carbonate (1.2 g, 11.48 mmol, 1.5 equiv), and 18-crown-6 (80.6 mg, 0.3 mmol, 0.04 equiv) were added to a solution of 2-(methylamino)ethanol (948 mg, 7.64 mmol, 1.0 equiv) in DMSO (1 mL). The mixture was heated at 100°C for 24 hours and then poured into ice water. The resulting product was extracted with dichloromethane, and the solvent was removed under vacuum. The residue was purified by column chromatography (SiO₂, SNAP ultra (10 g), EtOAc/hexane = 4% → 26%) to give 4-((2-hydroxyethyl)(methyl)amino)benzaldehyde as a yellow solid (1.09 g, 6.12 mmol, 80% yield). The spectroscopic properties of this compound were consistent with those reported in the literature.
¹H NMR (400 MHz, CDCl₃) δ 9.61 (1H, s), 7.65 (2H, d, J = 8.7 Hz), 6.72 (2H, d, J = 8.7 Hz), 3.85 (dt, J = 4.5 Hz, J = 5.8 Hz, 2H), 3.61 (t, J = 5.8 Hz, 2H), 3.37 (m, 1H), 3.11 (s, 3H)_{∘}

The method described above is a typical method for synthesizing an aminobenzaldehyde compound. In the following production methods, aminobenzaldehyde compounds were produced according to the procedure described in Production Example 4(1).

### (2) Production of 4-(2-(4-((2-hydroxyethyl)(methyl)amino)benzylidene)hydrazinyl)benzoic acid

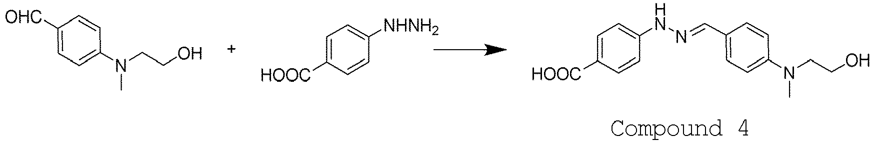

4-(2-(4-((2-Hydroxyethyl)(methyl)amino)benzylidene)hydrazinyl)benzoic acid was obtained as a yellow solid (104.6 mg, 0.334 mmol, 53% yield) after purification by column chromatography (SiO₂, SNAP ultra (10 g), MeOH/CH₂Cl₂ = 1% → 12%) in the same manner as in the procedure described in Production Example 1, except that 4-((2-hydroxyethyl)(methyl)amino)benzaldehyde (112.7 mg, 0.628 mmol) produced in (1) above was used in place of 4-isopropylbenzaldehyde.

¹H NMR (500 MHz, DMSO-d₆) δ 10.45 (1H, s), 7.82 (1H, s), 7.78 (2H, d, J = 8.3 Hz), 7.47 (2H, d, J = 8.5 Hz), 7.02 (2H, d, J = 8.3 Hz), 6.71 (2H, d, J = 8.5 Hz), 4.73 (1H, br), 3.53 (2H, t, J = 5.7 Hz), 3.43 (2H, t, J = 5.7 Hz), 2.96 (3H, s);
¹³C NMR (500 MHz, DMSO-d₆) δ 167.5, 149.8, 149.4, 140.5, 131.4, 127.6, 122.5, 119.4, 111.7, 110.8, 58.3, 54.2, 21.2; FT-IR (KBr) 3422, 3238, 2871, 1655, 1602, 1519, 1278, 1167 cm⁻¹;
HRMS (ESI-TOF) m/z: [M + Na]⁺Calcd for C₁₇H₁₉N₃O₃Na 336.1319; Found 336.1318.

### Production Example 5

### 4-(2-(4-((3-Hydroxypropyl)(methyl)amino)benzylidene)hydrazinyl)benzoic acid (compound 5)

### (1) Production of 4-((3-hydroxypropyl)(methyl)amino)benzaldehyde

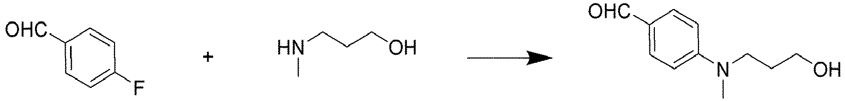

4-((3-Hydroxypropyl)(methyl)amino)benzaldehyde was obtained as a yellow solid (414.7 mg, 2.14 mmol, 56% yield) after purification by column chromatography (SiO₂, SNAP ultra (10 g), EtOAc/CH₂Cl₂ = 1% → 34%) in the same manner as in the procedure described in Production Example 4(1), except that 3-(methylamino)propan-1-ol (511.3 mg, 5.74 mmol) was used in place of 2-(methylamino)ethanol.
¹H NMR (400 MHz, CDCl₃) δ 9.63 (1H, s), 7.66 (2H, d, J = 8.9 Hz), 6.68 (2H, d, J = 8.9 Hz), 3.67 (2H, t, J = 5.8 Hz), 3.54 (2H, t, J = 7.1 Hz), 3.02 (3H, s), 2.79 (1H, brs), 1.83 (2H, m)

### (2) Production of 4-(2-(4-((3-hydroxypropyl)(methyl)amino)benzylidene)hydrazinyl)benzoic acid

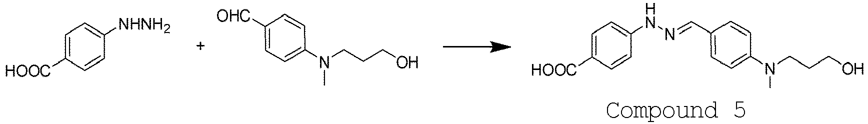

4-(2-(4-((3-Hydroxypropyl)(methyl)amino)benzylidene)hydrazinyl)benzoic acid was obtained as a yellow solid (45.1 mg, 0.137 mmol, 74% yield) after purification by column chromatography (SiO₂, SNAP ultra (10 g), MeOH/CH₂Cl₂ = 1% → 12%) in the same manner as in the procedure described in Production Example 1, except that 4-((3-hydroxypropyl)(methyl)amino)benzaldehyde (36 mg, 0.186 mmol) produced in (1) above was used in place of 4-isopropylbenzaldehyde.
¹H NMR (500 MHz, CD₃OD) δ 7.86 (2H, d, J = 8.8 Hz),7.74 (1H, s), 7.50 (2H, d, J = 8.5 Hz),7.05 (2H, d, J = 8.8 Hz), 6.75 (2H, d, J = 8.5 Hz), 3.62 (2H, m), 3.48 (2H, m), 2.98 (3H, s), 1.80 (2H, m)

### Production Example 6

### 4-(2-(4-(Ethyl(4-hydroxybutyl)amino)benzylidene)hydrazinyl)benzoic acid (compound 6)

### (1) Production of 4-(ethyl(4-hydroxybutyl)amino)benzaldehyde

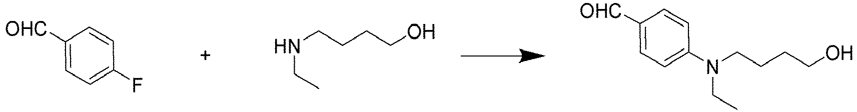

4-(Ethyl(4-hydroxybutyl)amino)benzaldehyde was obtained as a yellow solid (630.7 mg, 2.84 mmol, 74% yield) after purification by column chromatography (SiO₂, SNAP ultra (10 g), EtOAc/CH₂Cl₂ = 4% → 34%) in the same manner as in the procedure described in Production Example 4(1), except that 4-(ethylamino)butan-1-ol (672.4 mg, 5.74 mmol) was used in place of 2-(methylamino)ethanol.
¹H NMR (400 MHz, CDCl₃) δ 9.68 (1H, s), 7.67 (2H, d, J = 8.7 Hz), 6.65 (2H, d, J = 8.7 Hz), 3.69 (2H, m), 3.44 (2H, m), 3.37 (2H, m), 1.71 (2H, m), 1.62 (2H, m), 1.18 (3H, m)

### (2) Production of 4-(2-(4-(ethyl(4-hydroxybutyl)amino)benzylidene)hydrazinyl)benzoic acid

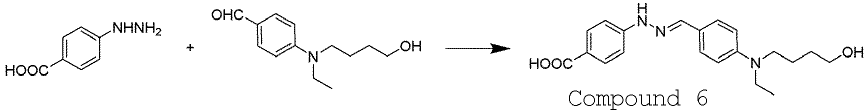

4-(2-(4-(Ethyl(4-hydroxybutyl)amino)benzylidene)hydrazinyl)benzoic acid was obtained as a yellow amorphous solid (54.0 mg, 0.151 mmol, 81% yield) after purification by column chromatography (SiO₂, SNAP ultra (10 g), MeOH/CH₂Cl₂ = 1% → 12%) in the same manner as in the procedure described in Production Example 1, except that 4-(ethyl(4-hydroxybutyl)amino)benzaldehyde (41 mg, 0.186 mmol) produced in (1) was used in place of 4-isopropylbenzaldehyde.
¹H NMR (400 MHz, CD₃OD) δ 7.85 (2H, d, J = 8.5 Hz),7.74 (1H, s), 7.48 (2H, d, J = 8.7 Hz),7.04 (2H, d, J = 8.5 Hz), 6.70 (2H, d, J = 8.7 Hz), 3.59 (2H, m), 3.42 (2H, m), 3.35 (2H, m) 1.68 (2H, m), 1.60 (2H, m), 1.16 (3H, m)

### Production Example 7

### 4-(2-((2,3,6,7-Tetrahydro-1H,5H-pyrido[3,2,1-ij]quinolin-9-yl)methylene)hydrazinyl)benzoic acid (compound 7)

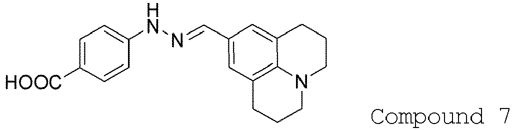

4-(2-((2,3,6,7-Tetrahydro-1H,5H-pyrido[3,2,1-ij]quinolin-9-yl)methylene)hydrazinyl)benzoic acid was obtained as a yellow solid (37.2 mg, 0.110 mmol, 59% yield) after purification by column chromatography (SiO₂, SNAP ultra (10 g), MeOH/CH₂Cl₂ = 5%) in the same manner as in the procedure described in Production Example 1, except that 2,3,6,7-tetrahydro-1H,5H-pyrido[3,2,1-ij]quinoline-9-carbaldehyde (37.4 mg, 0.186 mmol) was used in place of 4-isopropylbenzaldehyde.

¹H NMR (500 MHz, DMSO-d₆) δ 10.38 (1H, s), 7.76 (2H, d, J = 8.5 Hz), 7.71 (1H, s), 7.02 (2H, s), 7.00 (2H, d, J = 8.5 Hz), 3.14 (4H, m), 2.69 (4H, m), 1.85 (4H, m);
¹³C NMR (500 MHz, DMSO-d₆) δ 167.6, 149.4, 143.4, 140.9, 131.4, 125.1, 121.9, 120.9, 119.2, 110.7, 49.4, 27.3, 21.5; FT-IR (KBr) 3304, 2926, 1659, 1596, 1514, 1265, 1157 cm⁻¹;
HRMS (ESI-TOF) m/z: [M + H]⁺ Calcd for C₂₀H₂₂N₃O₂ 336.1707; Found 336.1694

### Production Example 8

### 4-(2-(4-Fluoro-2-methylbenzylidene)hydrazinyl)benzoic acid (compound 8)

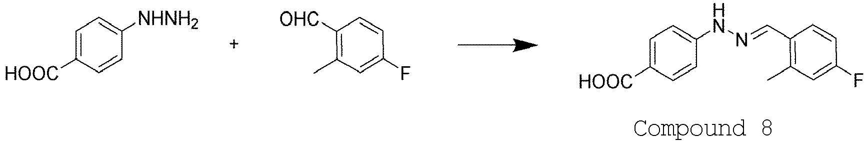

4-(2-(4-Fluoro-2-methylbenzylidene)hydrazinyl)benzoic acid was obtained as an off-white solid (11.0 mg, 0.040 mmol, 21% yield) after purification by column chromatography (SiO₂, SNAP ultra (10 g), MeOH/CH₂Cl₂ = 5%) in the same manner as in the procedure described in Production Example 1, except that 4-fluoro-2-methylbenzaldehyde (25.6 mg, 0.186 mmol) was used in place of 4-isopropylbenzaldehyde.

¹H NMR (400 MHz, CD₃OD) δ 8.09 (s, 1H), 7.89 (2H, d, J = 8.9 Hz), 7.83 (1H, dd, J = 9.4 Hz J = 9.4 Hz), 7.09 (2H, d, J = 8.9 Hz), 6.94 (2H, m), 2.49 (s, 3H);
LRMS (ESI+) m/z 273 [M+H]⁺

### Production Example 9

### 4-(2-((Perfluorophenyl)methylene)hydrazinyl)benzoic acid (compound 9)

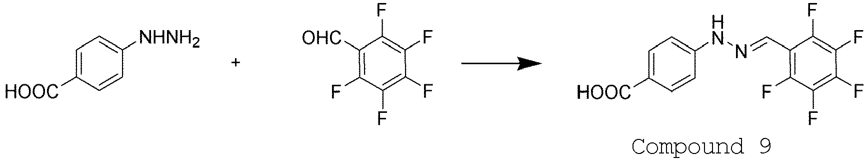

A reaction was performed in the same manner as in the procedure described in Production Example 1, except that 2,3,4,5,6-pentafluorobenzaldehyde (61.3 mg, 0.186 mmol) was used in place of 4-isopropylbenzaldehyde, and the resulting precipitate was collected by filtration. The solid was washed with Et₂O to give 4-(2-((perfluorophenyl)methylene)hydrazinyl)benzoic acid as a white solid (2.8 mg, 0.008 mmol, 4% yield).
¹H NMR (400 MHz, CD₃OD) δ 7.92 (2H, d, J = 8.9 Hz), 7.89 (1H, s), 7.13 (2H, d, J = 8.9 Hz)

### Production Example 10

### 4-(2-(2-Chloro-5-(trifluoromethyl)benzylidene)hydrazinyl)benzoic acid (compound 10)

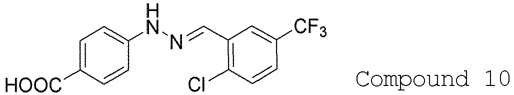

4-(2-(2-Chloro-5-(trifluoromethyl)benzylidene)hydrazinyl)benzoic acid was obtained as a beige solid (44.8 mg, 0.131 mmol, 70% yield) in the same manner as in the procedure described in Production Example 1, except that 2-chloro-5-(trifluoromethyl)benzaldehyde (38.70 mg, 0.186 mmol) was used in place of 4-isopropylbenzaldehyde.

¹H NMR (500 MHz, DMSO-d₆) δ 12.19 (1H, br), 11.29 (1H, s), 8.29 (1H, s), 8.23 (1H, s), 7.85 (2H, d, J = 8.8 Hz), 7.70 (1H, d, J = 8.3 Hz), 7.64 (1H, d, J = 8.3 Hz), 7.15 (2H, d, J = 8.8 Hz);
¹³C NMR (500 MHz, DMSO-d₆) δ 167.4, 148.1, 135.4, 133.7, 133.2, 131.4, 128.6, 128.4, 125.8, 125.0, 122.4, 121.7, 111.9; ¹⁹F NMR (400 MHz, DMSO-d₆) δ -63.67; FT-IR (KBr) 3280, 3077, 2973, 1677, 1607, 1271, 1165 cm⁻¹;
HRMS (ESI-TOF) m/z: [M + Na]⁺ Calcd for C₁₅H₁₀ClF₃N₂O₂Na 365.0275; Found 365.0283.

### Production Example 11

### 4-(2-(2-Fluoro-5-(trifluoromethyl)benzylidene)hydrazinyl)benzoic acid (compound 11)

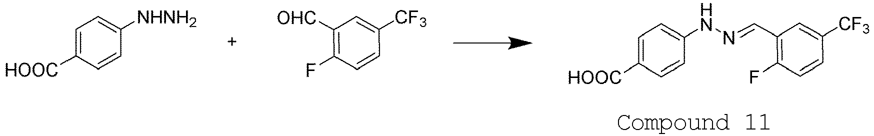

4-(2-(2-Fluoro-5-(trifluoromethyl)benzylidene)hydrazinyl)benzoic acid was obtained as a beige solid (4.8 mg, 0.014 mmol, 7% yield) after purification by column chromatography (SiO₂, SNAP ultra (10 g), EtOAc/hexane = 30% → 100%) in the same manner as in the procedure described in Production Example 1, except that 2-fluoro-5-(trifluoromethyl)benzaldehyde (35.7 mg, 0.186 mmol) was used in place of 4-isopropylbenzaldehyde.
¹H NMR (400 MHz, DMSO-d₆) δ 12.40 (1H, brs), 11.20 (1H, s), 8.22 (1H, d, ³J_{H-H} = 7.5 Hz), 8.12 (1H, s), 7.84 (2H, d, ³J_{H-H} = 8.5 Hz), 7.75 (1H, s), 7.51 (1H, dd, ³J_{H-F} = 9.8 Hz, ³J_{H-H} = 7.5 Hz), 7.15 (2H, d, ³J_{H-H} = 8.5 Hz)

### Production Example 12

### 4-(2-(4-Ethynylbenzylidene)hydrazinyl)benzoic acid (compound 12)

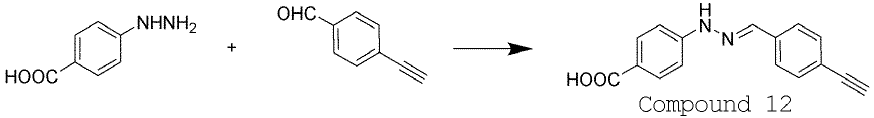

4-(2-(4-Ethynylbenzylidene)hydrazinyl)benzoic acid was obtained as a yellow solid (37.8 mg, 0.143 mmol, 76% yield) after purification by column chromatography (SiO₂, SNAP ultra (10 g), EtOAc/hexane = 30% → 100%) in the same manner as in the procedure described in Production Example 1, except that 4-ethynylbenzaldehyde (24.1 mg, 0.186 mmol) was used in place of 4-isopropylbenzaldehyde.
¹H NMR (400 MHz, CD₃OD) δ 7.90 (2H, d, J = 8.9 Hz), 7.83 (1H, s), 7.65 (2H, d, J = 8.5 Hz), 7.46 (2H, d, J = 8.5 Hz), 7.13 (2H, d, J = 8.9 Hz), 3.56 (1H, s); LRMS (ESI+) m/z 265 [M+H]⁺

### Production Example 13

### 3-((2-(4-Carboxyphenyl)hydrazono)methyl)benzoic acid (compound 13)

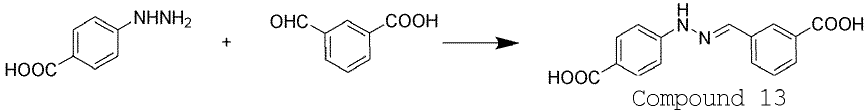

3-((2-(4-Carboxyphenyl)hydrazono)methyl)benzoic acid was obtained as a light yellow solid (45.5 mg, 0.160 mmol, 86% yield) in the same manner as in the procedure described in Production Example 1, except that 3-formylbenzoic acid (27.9 mg, 0.186 mmol) was used in place of 4-isopropylbenzaldehyde.
¹H NMR (400 MHz, DMSO-d₆) δ10.93 (1H, s), 8.22 (1H, s), 8.01 (1H, s), 7.93 (1H, d, J = 7.6 Hz), 7.88 (1H, d, J = 7.6 Hz), 7.83 (2H, d, J = 8.9 Hz), 7.53 (1H, dd, J = 7.6, 7.6 Hz), 7.12 (2H, d, J = 8.9 Hz)

### Production Example 14

### 4-(5-(4-(tert-Butyl)phenyl)oxazol-2-yl)benzoic acid (compound 14) (1) Production of 5-(4-(tert-butyl)phenyl)oxazole

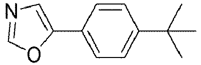

4-tert-Butylbenzaldehyde (227 mg, 1.4 mmol, 1 equiv) and Tos MIC (287 mg 1.47 mmol, 1.05 equiv) were dissolved in methanol (5 mL), followed by the addition of anhydrous potassium carbonate (380 mg, 2.8 mmol, 2 equiv). The reaction mixture was heated under reflux for 2 hours. The reaction mixture was cooled to room temperature and poured into water and diethyl ether. The organic phase was separated, washed with water, and dried over sodium sulfate. After filtration, the solvent was removed under vacuum, and the crude product was purified by column chromatography (SiO₂, SNAP ultra (10 g), EtOAc/hexane = 5% → 30%) to give 5-(4-(tert-butyl)phenyl)oxazole as a yellow solid (219.0 mg, 1.08 mmol, 77% yield). The spectral data were consistent with those reported in the literature.
¹H NMR (400 MHz, CDCl₃) δ 7.89 (1H, s), 7.58 (2H, d, J = 8.5 Hz), 7.44 (2H, d, J = 8.5 Hz), 7.30 (1H, s), 1.33 (9H, s).

The method described above is a typical method for synthesizing an aryloxazole compound. In the following production methods, aryloxazole compounds were produced according to the procedure described in Production Example 14(1).

### (2) Production of methyl 4-(5-(4-(tert-butyl)phenyl)oxazol-2-yl)benzoate

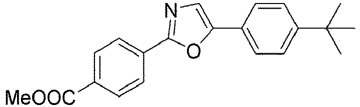

DMF (0.5 mL) was added to a mixture of 5-(4-(tert-butyl)phenyl)oxazole (50.30 mg, 0.25 mmol, 1 equiv) produced in (1), methyl 4-iodobenzoate (78.6 mg, 0.3 mmol, 1.2 equiv), CuI (47.6 mg, 0.25 mmol, 1 equiv), PPh₃ (13.1 mg, 0.05 mmol, 0.2 equiv), and Na₂CO₃ (53 mg, 0.5 mmol, 2 equiv) . The resulting mixture was stirred at 150°C for 2 hours. After cooling, the mixture was poured into water, extracted with dichloromethane, and dried over sodium sulfate. After filtration, the solvent was removed under vacuum, and the crude product was purified by column chromatography (SiO₂, SNAP ultra (10 g), EtOAc/hexane = 5% → 30%) to give methyl 4-(5-(4-(tert-butyl)phenyl)oxazol-2-yl)benzoate (66.80 mg, 0.199 mmol, 79% yield) as a white solid. The spectral data were consistent with those reported in the literature.

¹H NMR (500 MHz, acetone-d₆) δ 8.24 (2H, d, J = 8.3 Hz), 8.15 (2H, d, J = 8.3 Hz), 7.80 (2H, d, J = 8.5 Hz), 7.70 (1H, s), 7.56 (2H, d, J = 8.5 Hz), 3.92 (3H, s), 1.34 (9H, s);
¹³C NMR (500 MHz, acetone-d₆) δ 166.8, 153.2, 153.0, 132.4, 132.3, 130.9, 127.0, 126.0, 125.2, 124.8, 52.7, 35.4, 31.5; FT-IR (KBr) 3122, 2961, 2860, 1715, 1274, 1106 cm⁻¹;
HRMS (ESI-TOF) m/z: [M + Na]⁺ Calcd for C₂₁H₂₁NO₃Na 358.1414; Found 358.1401.

### (3) 4-(5-(4-(tert-Butyl)phenyl)oxazol-2-yl)benzoic acid

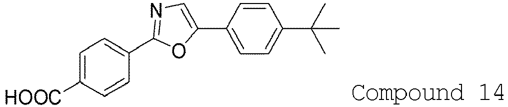

A solution of methyl 4-(5-(4-(tert-butyl)phenyl)oxazol-2-yl)benzoate (33.5 mg, 0.1 mmol, 1 equiv) in acetone (0.5 mL) was stirred under reflux for 15 minutes. Next, a solution of NaOH (8 mg, 0.2 mmol, 2 equiv) in water (5 mL) was added. The mixture was stirred under reflux for 3 hours. After the mixture was cooled to room temperature, the solvent was removed under vacuum. A 1M HCl aqueous solution was added, the resulting product was extracted with EtOAc, and the combined EtOAc layers were dried over Na₂SO₄ and filtered, and the solvent was removed under vacuum. The residue was purified by column chromatography (SiO₂, SNAP ultra (10 g), EtOAc/hexane = 50) to give 4-(5-(4-(tert-butyl)phenyl)oxazol-2-yl)benzoic acid as a pale white solid (13.7 mg, 0.042 mmol, 42% yield).

¹H NMR (500 MHz, DMSO-d₆) δ 8.16 (2H, d, J = 8.3 Hz), 8.09 (2H, d, J = 8.3 Hz), 7.82 (1H, s), 7.76 (2H, d, J = 8.5 Hz), 7.50 (2H, d, J = 8.5 Hz), 1.29 (9H, s);
¹³C NMR (500 MHz, DMSO-d₆) δ 166.7, 159.1, 151.6, 132.2, 130.3, 130.1, 126.0, 125.9, 124.5, 124.1, 34.5, 30.9; FT-IR (KBr) 3069, 2954, 2864, 1681, 1425, 1289 cm⁻¹;
HRMS (ESI-TOF) m/z: [M + H]⁺ Calcd for C₂₀H₂₀NO₃ 322.1438; Found 322.1438.

### Production Example 15

### 4-(5-(2-Chloro-5-(trifluoromethyl)phenyl)oxazol-2-yl)benzoic acid (compound 15)

### (1) Production of 5-(2-chloro-5-(trifluoromethyl)phenyl)oxazole

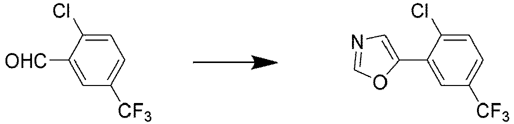

2-Chloro-5-(trifluoromethyl)benzaldehyde (292 mg, 1.4 mmol, 1 equiv) and TosMIC (287 mg, 1.47 mmol, 1.05 equiv) were dissolved in methanol (5 mL), followed by the addition of anhydrous potassium carbonate (380 mg, 2.8 mmol, 2 equiv). The reaction mixture was heated under reflux for 2 hours. The reaction mixture was cooled to room temperature and poured into water and diethyl ether. The organic phase was separated, washed with water, and dried over sodium sulfate. The solvent was filtered and removed under vacuum, and the crude product was purified by column chromatography (SiO₂, SNAP ultra (10 g), EtOAc/hexane = 5% → 30%) to give 5-(2-chloro-5-(trifluoromethyl)phenyl)oxazole as a white solid (244.6 mg, 0.987 mmol, 70% yield).
¹H NMR (400 MHz, CDCl₃) δ8.07 (1H, d, J = 1.7 Hz), 8.00 (1H, s), 7.86 (1H, s), 7.59 (1H, d, J = 8.5 Hz), 7.50 (1H, dd, J = 1.7 Hz, J = 8.5 Hz)

### (2) Production of methyl 4-(5-(2-chloro-5-(trifluoromethyl)phenyl)oxazol-2-yl)benzoate

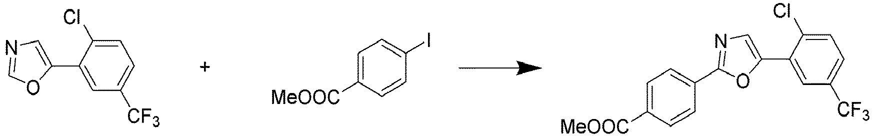

DMF (0.5 mL) was added to a mixture of 5-(2-chloro-5-(trifluoromethyl)phenyl)oxazole (61.9 mg, 0.25 mmol, 1 equiv) produced in (1), methyl 4-iodobenzoate (78.6 mg, 0.3 mmol, 1.2 equiv), CuI (47.6 mg, 0.25 mmol, 1 equiv), PPh₃ (13.1 mg, 0.05 mmol, 0.2 equiv), and Na₂CO₃ (53 mg, 0.5 mmol, 2 equiv). The resulting liquid mixture was stirred at 150°C for 2 hours. After cooling, the mixture was poured into water, extracted with dichloromethane, and dried over sodium sulfate. After the solvent was filtered and removed under vacuum, the crude product was purified by column chromatography (SiO₃, SNAP ultra (10 g), EtOAc/hexane = 5% → 30%) to give methyl 4-(5-(2-chloro-5-(trifluoromethyl)phenyl)oxazol-2-yl)benzoate as a white solid (40.8 mg, 0.106 mmol, 42% yield).
¹H NMR (400 MHz, CDCl₃) δ 8.21 (1H, d, J = 8.5 Hz), 8.16-8.20 (4H, m), 8.00 (1H, s), 7.64 (1H, d, J = 8.0 Hz), 7.53 (1H, d, J = 8.5 Hz), 3.96 (3H, s)

### (3) Production of 4-(5-(2-chloro-5-(trifluoromethyl)phenyl)oxazol-2-yl)benzoic acid

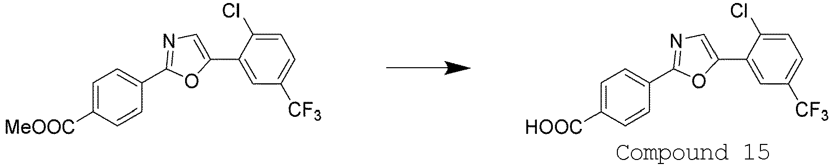

Methyl 4-(5-(2-chloro-5-(trifluoromethyl)phenyl)oxazol-2-yl)benzoate (33.5 mg, 0.1 mmol, 1 equiv) in acetone (0.5 mL) was stirred under reflux for 15 minutes, followed by the addition of NaOH (8 mg, 0.2 mmol, 2 equiv) dissolved in water (5 mL). The resulting liquid mixture was stirred under reflux for 3 hours. After the mixture was cooled to room temperature, the solvent was removed under vacuum. A 1M HCl aqueous solution was added, the resulting product was extracted with EtOA, the combined EtOAc layers were dried over Na₂SO₄ and filtered, and the solvent was removed under vacuum. The residue was purified by column chromatography (SiO₂, SN AP ultra (10 g), EtOAc/hexane = 50% → MeOH = 100%) to give 4-(5-(2-chloro-5-(trifluoromethyl)phenyl)oxazol-2-yl)benzoic acid as a pale white solid (4.8 mg, 0.013 mmol, 12% yield).
¹H NMR (400 MHz, CD₃OD) δ 8.26 (1H, s), 8.15 (2H, d, J = 8.2 Hz), 8.11 (2H, d, J = 8.2 Hz), 8.04 (1H, s), 7.80 (1H, d, J = 8.5 Hz), 7.68 (1H, d, J = 8.5 Hz)

### Production Example 16

### 4-(1-(4-(tert-Butyl)phenyl)-1H-1,2,3-triazol-4-yl)benzoic acid (compound 16)

### (1) Production of 1-azido-4-(tert-butyl)benzene

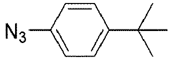

4-(tert-Butyl)aniline (149.2 mg, 1 mmol, 1 equiv) was dissolved in a 10N HCl aqueous solution (10 mL) and EtOH (5 mL) at room temperature. After the mixture was cooled to 0°C, and NaNO₂ (69.0 mg, 1 mmol, 1 equiv) in H₂O (0.1 mL) was added, the reaction mixture was stirred at 0 to 5°C for 10 minutes. Sodium azide (78.0 mg, 1.2 mmol, 1.2 equiv) was added, and the mixture was stirred at room temperature for 2 hours. The reaction was post-treated by dilution with EtOAc. The organic layer was washed with brine and dried over Na₂SO₄. After filtration and evaporation of the solvent, 1-azido-4-(tert-butyl)benzene was obtained as a brown oil, which was sufficiently pure for further reaction (142.30 mg, 0.812 mmol, 81% yield). The spectral data were consistent with those reported in the literature.
¹H NMR (400 MHz, CDCl₃) δ 7.37 (2H, d, J = 8.7 Hz), 6.96 (2H, d, J = 8.7 Hz), 1.31 (9H, s).

### (2) Production of 4-(1-(4-(tert-butyl)phenyl)-1H-1,2,3-triazol-4-yl)benzoic acid

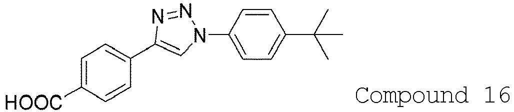

THF (2.5 mL) and Et₃N (1.25 mL) were added to a mixture of 4-ethynylbenzoic acid (20 mg, 0.136 mmol, 1 equiv), 1-azido-4-(tert-butyl)benzene (23.8 mg, 0.136 mmol, 1 equiv) produced in (1) above, and CuI (5.2 mg, 0.136 mmol, 0.2 equiv). The reaction mixture was stirred at 60°C overnight. The solvent was removed under vacuum, and the residue was purified by column chromatography (SiO₂, SNAP ultra (10 g), MeOH/CH₂Cl₂ = 10% → 30%) to give 4-(1-(4-(tert-butyl)phenyl)-1H-1,2,3-triazol-4-yl)benzoic acid as a beige solid (28.6 mg, 0.088 mmol, 65% yield).

¹H NMR (400 MHz, CD₃OD) δ 8.98 (1H, s), 8.09 (2H, d, J = 8.5 Hz), 8.01 (2H, d, J = 8.5 Hz), 7.80 (2H, d, J = 8.8 Hz), 7.62 (2H, d, J = 8.8 Hz), 1.35 (9H, s);
¹³C NMR (500 MHz, DMSO-d₆) δ 151.6, 146.3, 142.5, 134.3, 134.2, 130.1, 126.7, 125.3, 120.6, 119.8, 34.5, 31.0; FT-IR (KBr) 3126, 3056, 2956, 1674, 1519, 1277 cm⁻¹;
HRMS (ESI-TOF) m/z: [M + H]⁺ Calcd for C₁₉H₂₀N₃O₂ 322.1550; Found 322.1541

### Production Example 17

### 4-(4-Isopropylbenzamido)benzoic acid (compound 17)

### (1) Production of ethyl 4-(4-isopropylbenzamido)benzoate

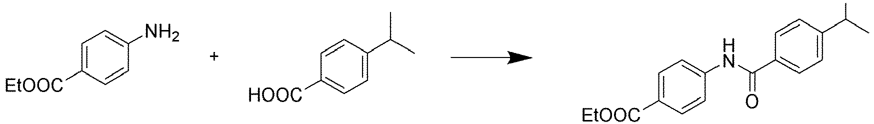

Thionyl chloride (1.00 g, 8.41 mmol, 6.4 equiv) was added to a dichloromethane solution (40 mL) of 4-isopropylbenzoic acid (215.1 mg, 1.31 mmol, 1 equiv), and the reaction mixture was stirred under reflux for 1 hour. Ethyl 4-aminobenzoate (240 mg, 1.45 mmol, 1.1 equiv) and triethylamine (150 mg, 1.49 mmol, 1.1 equiv) were then added. After stirring under reflux for 8 hours, the reaction mixture was washed with 10% HCl and saturated NaHCO₃, dried over Na₂SO₄, and filtered, and the solvent was removed under vacuum. The residue was purified by column chromatography (SiO₂, SNAP ultra (10 g), EtOAc/hexane = 5% → 45%) to give ethyl 4-(4-isopropylbenzamido)benzoate as a pale yellow solid (154.5 mg, 0.496 mmol, 37% yield).
¹H NMR (400 MHz, CDCl₃) δ8.09 (1H, s), 8.03 (2H, d, J = 8.5 Hz), 7.79 (2H, d, J = 7.5 Hz), 7.73 (2H, d, J = 8.5 Hz), 7.32 (2H, d, J = 7.5 Hz), 4.36 (2H,m), 2.97 (1H, m), 1.38 (3H, m), 1.27 (6H, m)

### (2) Production of 4-(4-isopropylbenzamido)benzoic acid

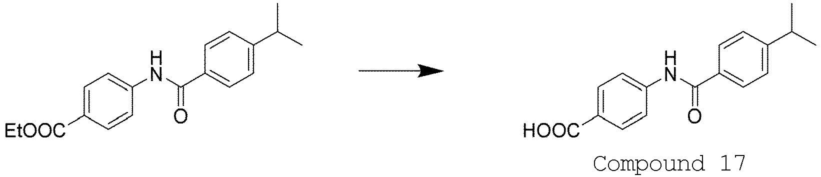

A 3M NaOH aqueous solution (0.2 mL) was added to a solution of ethyl 4-(4-isopropylbenzamido)benzoate (84.1 mg, 0.27 mmol, 1 equiv) in EtOH (1 mL) and THF (1 mL). The mixture was stirred at room temperature for 2 hours, and the solvent was then removed under vacuum. A 1M HCl aqueous solution was added thereto, and the resulting product was extracted with EtOAc. The EtOAc layers were combined, dried over Na₂SO₄, and filtered, and the solvent was removed under vacuum. The residue was purified by column chromatography (SiO₂, SNAP ultra (10 g), EtOAc/CH₂Cl₂ = 13% → 21%) to give 4-(4-isopropylbenzamido)benzoic acid as a pale yellow solid (26.9 mg, 0.07 mmol, 42% yield).
¹H NMR (400 MHz, CD₃OD) δ 8.02 (2H, d, J = 8.9 Hz), 7.88 (2H, d, J = 8.5 Hz), 7.84 (2H, d, J = 8.9 Hz), 7.40 (1H, d, J = 8.5 Hz), 3.00 (1H, sep, J = 6.7 Hz), 1.29 (6H, d, J = 6.7 Hz)

### Production Example 18

### 4-(2-(1-(4-Isopropylphenyl)ethylidene)hydrazinyl)benzoic acid (compound 18)

### (1) Production of 1-(4-isopropylphenyl)ethan-1-one

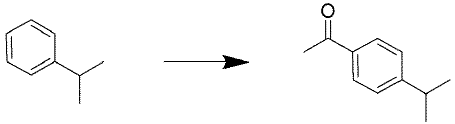

Aluminum chloride (3.6 g, 27.4 mmol, 3.3 equiv) was added at 0°C to a stirred solution of cumene (1.0 g, 8.3 mmol, 1 equiv) in CH₂Cl₂ (11 mL), followed by the addition of acetic anhydride (1.0 g, 10 mmol, 1.2 equiv). The reaction mixture was stirred at room temperature for 30 minutes, diluted with a saturated NaHCO₃ aqueous solution, and extracted with CH₂Cl₂. The organic layers were combined, washed with brine, dried over Na₂SO₄, and concentrated under vacuum. The residue was purified by column chromatography (SiO₂, SNAP ultra (10 g), EtOAc/hexane = 12% → 60%) to give 1-(4-isopropylphenyl)ethan-1-one as a colorless oil (1.19 g, 89% yield).
¹H NMR (400 MHz, CDCl₃) δ 7.89 (2H, d, J = 8.2 Hz), 7.30 (2H, d, J = 8.2 Hz), 2.95 (1H, sep, J = 6.7 Hz), 2.56 (3H, s), 1.26 (6H, d, J = 6.7 Hz)

### (2) Production of 4-(2-(1-(4-isopropylphenyl)ethylidene)hydrazineyl)benzoic acid

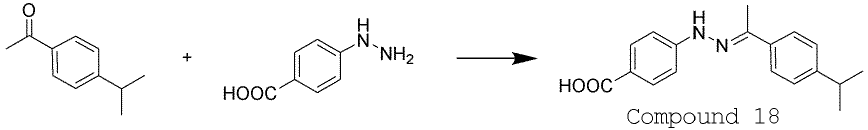

A solution of 1-(4-isopropylphenyl)ethan-1-one (79.1 mg, 0.488 mmol, 1.0 equiv) and 4-isopropylbenzaldehyde (74.2 mg, 0.488 mmol, 1.0 equiv) in EtOH (10 mL), and acetic acid (0.025 mL) were stirred under reflux for 2.5 hours. The solvent was removed under vacuum, and purification was performed by column chromatography (SiO₂, SNAP ultra (10 g), CH₂Cl₂/hexane = 12% → 60%) to give 4-(2-(1-(4-isopropylphenyl)ethylidene)hydrazineyl)benzoic acid as a white solid (36.4 g, 0.122 mmol, 25% yield).
¹H NMR (500 MHz, CD₃OD) δ 7.89 (2H, d, J = 8.5 Hz), 7.75 (2H, d, J = 8.5 Hz), 7.24-7.26 (4H, m), 2.91 (1H, sep, J = 6.8 Hz), 2.28 (3H, s), 1.26 (6H, d, J = 6.8 Hz)

### Production Example 19

### 4-(2-(4-(2-(Dimethylamino)ethoxy)benzylidene)hydrazineyl)benzoic acid (negative control compound 1)

### (1) Production of 4-(2-(dimethylamino)ethoxy)benzaldehyde

The title compound was produced according to the description in Kok-Fui L, Kit-Lam C, Chong-Yew L. Eur. J. Med. Chem. 2015, 94, 195 (NPL 5).

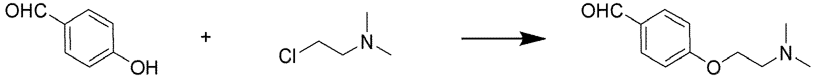

4-Hydroxybenzaldehyde (122.0 mg, 1.0 mmol, 1.0 equiv) was dissolved in acetone (4 mL), and K₂CO₃ (422.0 mg, 3.05 mmol, 3.05 equiv) was added. The resulting liquid mixture was vigorously stirred at 60°C for 2 hours. The resulting reaction liquid was allowed to cool to room temperature and then mixed with an acetone solution (13 mL) of 2-chloro-N,N'-dimethylethan-1-amine hydrochloride (144.0 mg, 1.4 mmol, 1.4 equiv). The reaction liquid was then stirred at 60°C for 2 hours, and the insoluble matter was removed by filtration. The resulting filtrate was concentrated to dryness, and the residue was separated by silica gel chromatography. The chromatography was performed using SiO₂ with SNAP ultra (10 g) as the stationary phase and MeOH/CH₂Cl₂ = 1% → 24% (concentration gradient) as the mobile phase. The fractions containing the target 4-(2-(dimethylamino)ethoxy)benzaldehyde were collected and concentrated under reduced pressure to give the target compound as a colorless oil (145.1 mg, 0.75 mmol, 75% yield).
¹H NMR (400 MHz, CD₃OD) δ 9.83 (1H, s), 7.87 (2H, d, J = 8.7 Hz), 7.11 (2H, d, J = 8.7 Hz), 4.21 (2H, t, J = 5.3 Hz), 2.80 (2H, t, J = 5.3 Hz), 2.34 (s, 6H)

### (2) 4-(2-(4-(2-(dimethylamino)ethoxy)benzylidene)hydrazineyl)benzoic acid

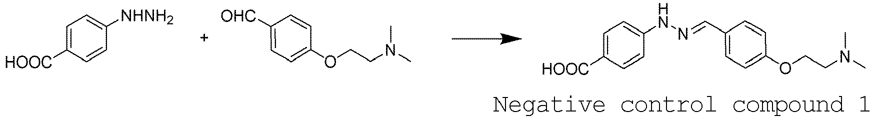

After reacting 4-(2-(dimethylamino)ethoxy)benzaldehyde (658.2 mg, 3.4 mmol) obtained above with 4-hydrazineylbenzoic acid (517.3 mg, 3.24 mmol) according to the method described in Production Example 1, purification was performed by silica gel chromatography (SiO₂, SNAP ultra (10 g), MeOH/EtOAc = 5% → 100%; concentration gradient elution) to give 4-(2-(4-(2-(dimethylamino)ethoxy)benzylidene)hydrazineyl)benzoic acid (negative control compound 1) as a brown solid (900.5 mg, 2.75 mmol). The yield was 80%.
¹H NMR (400 MHz, DMSO-d₆) δ 10.50 (1H, s), 7.76, (1H, s), 7.65 (2H, d, J = 8.5 Hz), 7.48 (2H, d, J = 8.0 Hz), 6.92 (2H, d, J = 8.5 Hz), 6.85 (2H, d, J = 8.0 Hz), 3.94 (2H, t, J = 5.3 Hz), 2.50 (2H, t, J = 5.3 Hz), 2.37 (6H, s)

### Production Example 20

### 4-(2-(4-(Dimethylamino)-2-hydroxybenzylidene)hydrazineyl)benzoic acid (negative control compound 2)

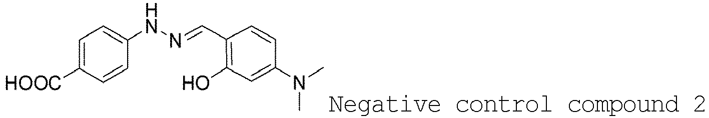

4-(2-(4-(Dimethylamino)-2-hydroxybenzylidene)hydrazineyl)benzoic acid was obtained as a yellow solid (31.3 mg, 0.104 mmol, 56% yield) after purification by column chromatography (SiO₂, SNAP ultra (10 g), MeOH/CH₂Cl₂ = 5%) in the same manner as in the procedure described in Production Example 1, except that 4-(dimethylamino)-2-hydroxybenzaldehyde (30.73 mg, 0.186 mmol) was used in place of 4-isopropylbenzaldehyde.

¹H NMR (400 MHz, DMSO-d₆) δ 10.55 (1H, br), 10.46 (1H, s), 8.09 (1H, s), 7.79 (2H, d, J = 8.7 Hz), 7.31 (1H, d, J = 8.5 Hz), 6.90 (2H, d, J = 8.7 Hz), 6.29 (1H, dd, J = 2.2, 8.5 Hz), 6.16 (1H, d, J = 2.2 Hz), 2.92 (6H, s) ;
¹³C NMR (500 MHz, DMSO-d₆) δ 167.5, 157.8, 152.1, 148.7, 131.5, 129.3, 119.7, 110.6, 108.7, 104.6, 98.6, 40.0;
FT-IR (KBr) 3321, 2847, 1671, 1599, 1522, 1272, 1168 cm⁻¹;
HRMS (ESI-TOF) m/z: [M + H]⁺ Calcd for C₁₆H₁₈N₃O₃ 300.1343; Found 300.1340.

### Production Example 21

### 4-(2-(4-((2,3-Dihydroxypropyl)(methyl)amino)benzylidene)hydrazineyl)benzoic acid (negative control compound 3)

### (1) Production of 4-((2,3-dihydroxypropyl)(methyl)amino)benzaldehyde

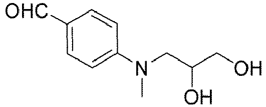

4-((2,3-Dihydroxypropyl)(methyl)amino)benzaldehyde was obtained as a beige solid (113.00 mg, 0.54 mmol, 14% yield) after purification by column chromatography (SiO₂, SNAP ultra (10 g), MeOH/CH₂Cl₂ = 5%) in the same manner as in the procedure described in Production Example 4(1), except that 3-(methylamino)propane-1,2-diol (603.50 mg, 5.74 mmol) was used in place of 2-(methylamino)ethanol.

¹H NMR (500 MHz, DMSO-d₆) δ 9.64 (1H, s), 7.65 (2H, d, J= 8.5 Hz), 6.80 (2H, d, J = 8.5 Hz), 4.88 (1H, s), 4.75 (1H, s), 3.72 (1H, d, J = 3.6 Hz), 3.62 (1H, dd, J = 3.6, 14.9 Hz), 3.26-3.41 (3H, m), 3.13 (3H, s);
¹³C NMR (500 MHz, DMSO-d₆) δ189.7, 153.7, 131.5, 124.2, 111.0, 69.5, 63.8, 55.0, 40.0; FT-IR (KBr) 3384, 2917, 1652, 1594, 1529, 1385, 1171 cm⁻¹;
HRMS (ESI-TOF) m/z: [M + Na]⁺ Calcd for C₁₁H₁₅NO₃Na 232.0944; Found 232.0957.

### (2) Production of 4-(2-(4-((2,3-dihydroxypropyl)(methyl)amino)benzylidene)hydrazineyl)benzoic acid

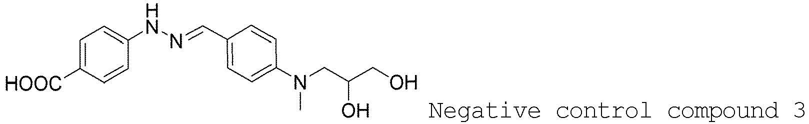

4-(2-(4-((2,3-Dihydroxypropyl)(methyl)amino)benzylidene)hydrazineyl)benzoic acid was obtained as a yellow solid (55.6 mg, 0.162 mmol, 87% yield) after purification by column chromatography (SiO₂, SNAP ultra (10 g), MeOH/CH₂Cl₂= 4% to 16%) in the same manner as in the procedure described in Production Example 1, except that 4-((2,3-dihydroxypropyl)(methyl)amino)benzaldehyde (38.90 mg, 0.186 mmol) produced in (1) above was used in place of 4-isopropylbenzaldehyde.

¹H NMR (500 MHz, DMSO-d₆) δ 10.44 (1H, s), 7.83 (1H, s), 7.78 (2H, d, J = 8.5 Hz), 7.47 (2H, d, J = 8.5 Hz), 7.02 (2H, d, J = 8.5 Hz), 6.72 (2H, d, J = 8.5 Hz), 4.77 (1H, br), 3.71 (1H, m), 3.54 (1H, m), 3.36 (2H, m), 3.19 (1H, m), 2.99 (3H, s);
¹³C NMR (500 MHz, DMSO-d₆) δ 167.6, 149.9, 149.4, 140.6, 131.4, 127.5, 122.4, 119.4, 111.6, 110.8, 69.8, 64.1, 55.4, 20.7;
FT-IR (KBr) 3312, 2926, 1671, 1600, 1518, 1267, 1166 cm⁻¹;
HRMS (ESI-TOF) m/z: [M + H]⁺ Calcd for C₁₈H₂₂N₃O₄ 344.1605; Found 344.1591.

### Production Example 22

### 3-(2-(4-Isopropylbenzylidene)hydrazineyl)benzoic acid (negative control compound 4)

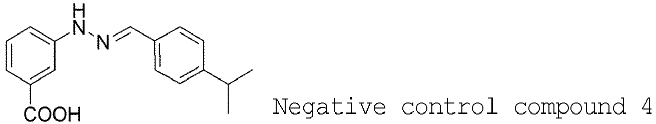

3-(2-(4-Isopropylbenzylidene)hydrazinyl)benzoic acid was obtained as a white solid (11.40 mg, 0.0403 mmol, 21% yield) by reacting with 4-isopropylbenzaldehyde as in the production method described in Production Example 1, except that 3-hydrazineylbenzoic acid (28.30 mg, 0.186 mmol) was used in place of 4-hydrazineylbenzoic acid. The spectroscopic properties were consistent with those reported in the literature.
¹H NMR (400 MHz, CD₃OD) δ 7.79 (1H, s), 7.74 (1H, s), 7.58 (2H, d, J = 8.2 Hz), 7.41 (1H, m), 7.30 (2H, d, J = 5.3 Hz), 7.25 (2H, d, J = 8.2 Hz), 2.91 (1H, sep, J = 6.7 Hz), 1.26 (6H, d, J = 6.7 Hz) .

### Production Example 23

### 2-(2-(4-Isopropylbenzylidene)hydrazineyl)benzoic acid (negative control compound 5)

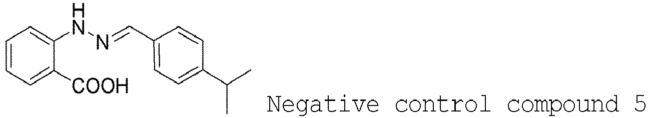

2-(2-(4-Isopropylbenzylidene)hydrazinyl)benzoic acid was obtained as a yellow solid (35.6 mg, 0.126 mmol, 67% yield) by reacting with 4-isopropylbenzaldehyde as in the production method described in Production Example 1, except that 2-hydrazineylbenzoic acid hydrochloride (35.08 mg, 0.186 mmol) was used in place of 4-hydrazineylbenzoic acid.

¹H NMR (400 MHz, CD₃OD) δ 7.91 (1H, d, J = 8.4 Hz), 7.90 (1H, s), 7.75 (1H, d, J = 8.5 Hz), 7.62 (2H, d, J = 8.0 Hz), 7.46 (1H, dd, J = 1.3, 8.5 Hz), 7.26 (2H, d, J = 8.0 Hz), 6.76 (1H, dd, J = 1.3, 8.4 Hz), 2.92 (1H, sep, J = 7.1 Hz), 1.26 (6H, d, J = 7.1 Hz) ;
¹³C NMR (500 MHz, DMSO-d₆) δ 169.8, 149.5, 147.3, 141.2, 134.7, 133.0, 131.4, 126.8, 126.5, 117.6, 113.2, 109.9, 33.5, 23.9; FT-IR (KBr) 3287, 2959, 2864, 1659, 1577, 1146 cm⁻¹; HRMS (ESI-TOF) m/z: [M + Na]⁺ Calcd for C₁₇H₁₈N₂O₂Na 305.1260; Found 305.1267.

### Production Example 24

### 1-(4-Isopropylbenzylidene)-2-phenylhydrazine (negative control compound 6)

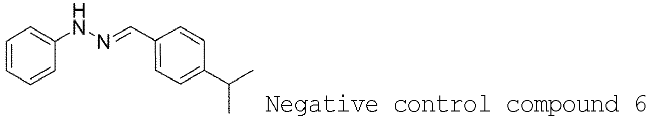

1-(4-Isopropylbenzylidene)-2-phenylhydrazine was obtained as a pink solid (2.5 mg, 0.0104 mmol, 5% yield) after purification by column chromatography (SiO₂, SNAP ultra (10 g), EtOAc/hexane = 3% → 7%) by reacting with 4-isopropylbenzaldehyde as in the production method described in Production Example 1, except that phenylhydrazine (20.08 mg, 0.186 mmol) was used in place of 4-hydrazineylbenzoic acid.
¹H NMR (500 MHz, DMSO-d₆) δ 10.24 (1H, s), 7.83 (1H, s), 7.55 (2H, d, J = 8.0 Hz), 7.25 (2H, d, J = 8.0 Hz), 7.20 (2H, dd, J = 7.4, 7.4 Hz), 7.04 (2H, d, J = 7.4 Hz), 6.73 (1H, t, J = 7.4 Hz), 2.88 (1H, sep, J = 6.8 Hz), 1.20 (6H, d, J = 6.8 Hz) ; ¹³C NMR (500 MHz, DMSO-d₆) δ 148.3, 145.4, 136.6, 133.5, 129.1, 126.6, 125.7, 118.5, 111.9, 33.3, 23.8; FT-IR (KBr) 3295, 3025, 2956, 1595, 1489, 1258, 1127 cm⁻¹; HRMS (ESI-TOF) m/z: [M + H]⁺ Calcd for C₁₆H₁₉N₂ 239.1543; Found 239.1557.

### Production Example 25

### 4-(2-(Cyclohexylmethylene)hydrazinyl)benzoic acid (negative control compound 7)

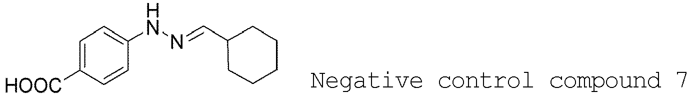

4-(2-(Cyclohexylmethylene)hydrazinyl)benzoic acid was obtained as a pink solid (39.6 mg, 0.161 mmol, 87% yield) after purification by column chromatography (SiO₂, SNAP ultra (10 g), EtOAc/hexane = 50%) by reacting with 4-hydrazineylbenzoic acid as in the production method described in Production Example 1, except that cyclohexanecarbaldehyde (20.83 mg, 0.186 mmol) was used in place of 4-isopropylbenzaldehyde.

¹H NMR (500 MHz, DMSO-d₆) δ 12.17 (1H, br), 10.15 (1H, s), 7.73 (2H, d, J = 8.8 Hz), 7.17 (1H, s), 6.90 (2H, d, J = 8.8 Hz), 2.21 (1H, m), 1.71 (5H, m), 1.24 (5H, m);
¹³C NMR (500 MHz, DMSO-d₆) δ 167.5, 149.7, 147.2, 131.3, 119.4, 110.7, 30.3, 25.8, 25.4, 25.3; FT-IR (KBr) 3319, 2929, 2851, 1670, 1602, 1423, 1289, 1167 cm⁻¹;
HRMS (ESI-TOF) m/z: [M + Na]⁺ Calcd for C₁₄H₁₈N₂O₂Na 269.1260; Found 269.1262.

### Production Example 26

### 4-(5-(4-(Dimethylamino)phenyl)oxazol-2-yl)benzoic acid (compound 19)

### (1) Production of N,N-dimethyl-4-(oxazol-5-yl)aniline

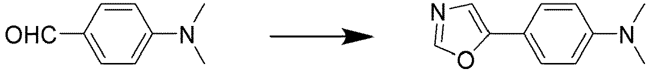

N,N-Dimethyl-4-(oxazol-5-yl)aniline was obtained as an orange solid (62.4 mg, 0.33 mmol, 66% yield) after purification by column chromatography (SiO₂, SNAP ultra (10 g), ethyl acetate/hexane = 20% → 100%) in the same manner as in the procedure described in Production Example 15(1), except that 4-(dimethylamino)benzaldehyde (75.4 mg, 0.5 mmol) was used in place of 2-chloro-5-(trifluoromethyl)benzaldehyde.
¹H NMR (500 MHz, CDCl₃) δ7.82 (1H, s), 7.51 (2H, d, J = 8.8 Hz), 7.14 (1H, s), 6.73 (2H, d, J = 8.8 Hz), 2.99 (6H, s)

### (2) Production of methyl 4-(5-(4-(dimethylamino)phenyl)oxazol-2-yl)benzoate

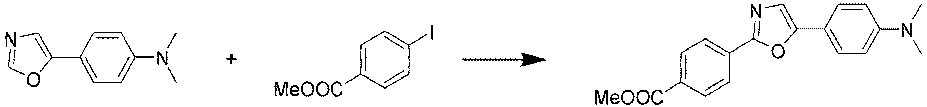

Methyl 4-(5-(4-(dimethylamino)phenyl)oxazol-2-yl)benzoate was obtained as an orange solid (12.2 mg, 0.03 mmol, 71% yield) after purification by column chromatography (SiO₂, SNAP ultra (10 g), ethyl acetate/hexane = 10% → 30%) in the same manner as in the procedure described in Production Example 15(2), except that N,N-dimethyl-4-(oxazol-5-yl)aniline (10.0 mg, 0.05 mmol) was used in place of 5-(2-chloro-5-(trifluoromethyl)phenyl)oxazole.
¹H NMR (400 MHz, CDCl₃) δ 8.13-8.15 (4H, m), 7.60 (2H, d, J = 8.5 Hz), 7.29 (1H, s), 6.76 (2H, d, J = 8.5 Hz), 3.94 (3H, s), 3.02 (6H, s)

### (3) Production of 4-(5-(4-(dimethylamino)phenyl)oxazol-2-yl)benzoic acid

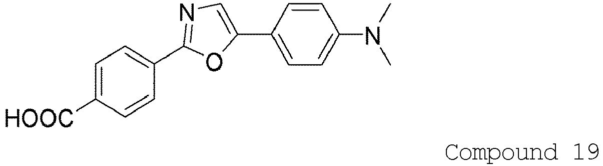

A solution of lithium hydroxide monohydrate (8 mg, 0.2 mmol) in water (0.2 mL) was added to a solution of methyl 4-(5-(4-(dimethylamino)phenyl)oxazol-2-yl)benzoate (12.2 mg, 0.03 mmol) in acetone (0.5 mL). The mixture was stirred under reflux for 3 hours. After the mixture was cooled to room temperature, a 1M hydrochloric acid aqueous solution was added to adjust the pH to 1 to 2, and the resulting precipitate was collected by filtration. The solid was washed with water to give 4-(5-(4-(dimethylamino)phenyl)oxazol-2-yl)benzoic acid as an orange solid (4.1 mg, 0.013 mmol, 35% yield).
¹H NMR (400 MHz, DMSO-d₆) δ 8.14 (2H, d, J = 8.3 Hz), 8.07 (2H, d, J = 8.3 Hz), 7.66 (2H, d, J = 8.9 Hz), 7.61 (1H, s), 6.81 (2H, d, J = 8.9 Hz), 2.96 (6H, s)

### Production Example 27

### 4-(2-(2-Chloro-5-(trifluoromethyl)phenyl)oxazol-5-yl)benzoic acid (compound 20)

### (1) Production of methyl 4-(2-bromoacetyl)benzoate

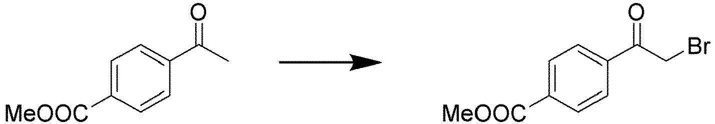

Methyl 4-acetylbenzoate (445.4 mg, 2.5 mmol, 1.0 equiv) was dissolved in ethyl acetate (21 mL), followed by the addition of copper(II) bromide (1.1 g, 5.0 mmol, 2.0 equiv). The liquid mixture was stirred under reflux for 3 hours. The resulting reaction liquid was allowed to cool to room temperature, and then the solid was collected by filtration. Further, washing was performed with water and hexane/ethyl acetate = 4:1 to give methyl 4-(2-bromoacetyl)benzoate as a yellow solid (511.5 mg, 1.9 mmol, 79% yield).
¹H NMR (400 MHz, CDCl₃) δ 8.15 (2H, d, J = 8.0 Hz), 8.06 (2H, d, J = 8.0 Hz), 4.46 (2H, s), 3.95 (3H, s)

### (2) Production of methyl 4-(2-azidoacetyl)benzoate

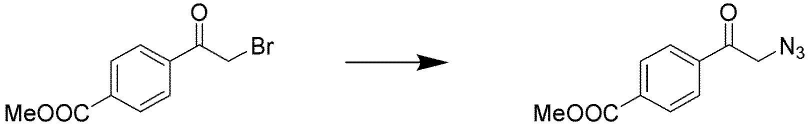

Methyl 4-(2-bromoacetyl)benzoate (363.0 mg, 1.4 mmol, 1.0 equiv) produced in (1) was dissolved in ethanol (12 mL) and acetic acid (0.48 mL), and the mixture was stirred at 4°C. Sodium azide (183.5 mg, 2.8 mmol, 2.0 equiv) was added, and the liquid mixture was stirred at 4°C overnight. The resulting reaction liquid was concentrated to dryness, and the residue was dissolved in ethyl acetate. The resulting solution was washed twice with a saturated aqueous sodium carbonate solution, dried over sodium sulfate, and filtered. The filtrate was concentrated under reduced pressure to give methyl 4-(2-azidoacetyl)benzoate (281.5 mg, 1.28 mmol, 90% yield).
¹H NMR (400 MHz, CDCl₃) δ 8.16 (2H, d, J = 8.7 Hz), 7.96 (2H, d, J = 8.7 Hz), 4.59 (2H, s), 3.96 (3H, s)

### (3) Production of methyl 4-glycylbenzoate hydrochloride

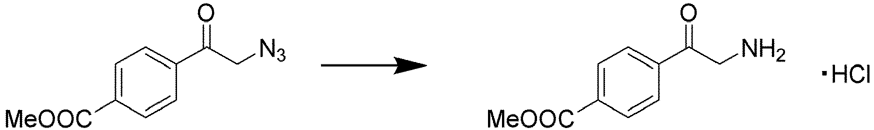

Methyl 4-(2-azidoacetyl)benzoate (281.5 mg, 1.2 mmol) produced in (2) was dissolved in methanol (5.8 mL) and 1M hydrochloric acid aqueous solution (1.28 mL), and the flask was purged with argon. Pd/C (28.0 mg, 10 w/w%) was added, and the flask was purged with hydrogen. The mixture was stirred at room temperature overnight. The resulting reaction liquid was filtered through Celite, and the filtrate was concentrated under reduced pressure to give methyl 4-glycylbenzoate hydrochloride (210.8 mg, 0.91 mmol, 76% yield).
¹H NMR (400 MHz, CD₃OD) δ 8.01 (2H, d, J = 8.3 Hz), 7.57 (2H, d, J = 8.3 Hz), 3.94 (2H, s), 3.89 (3H, s)

### (4) Production of 2-chloro-5-(trifluoromethyl)benzoic acid

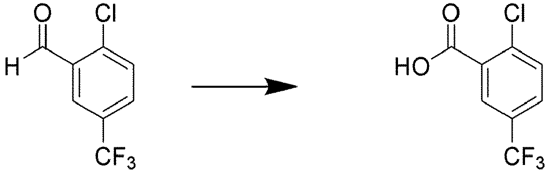

2-Chloro-5-(trifluoromethyl)benzaldehyde (522 mg, 2.5 mmol, 1.0 equiv) was dissolved in acetone (15 mL) and water (15 mL), followed by the addition of potassium permanganate (1.6 g, 10 mmol, 4.0 equiv). The mixture was stirred at room temperature for 3 and a half hours. Sodium carbonate (0.4 g, 3.75 mmol, 1.5 equiv) was added thereto, and the mixture was stirred at room temperature for 15 minutes. The resulting reaction liquid was filtered through Celite, and the filtrate was concentrated under reduced pressure to remove the solvent. The remaining aqueous solution was acidified with 2M hydrochloric acid, followed by extraction with ethyl acetate. The organic layer was washed with water and a saturated aqueous sodium chloride solution, dried over sodium sulfate, and filtered. The filtrate was concentrated under reduced pressure to give 2-chloro-5-(trifluoromethyl)benzoic acid (556.4 mg, 2.4 mmol, 99% yield).
¹H NMR (400 MHz, CD₃OD) δ 8.11 (1H, s), 7.77 (1H, d, J = 8.5 Hz), 7.69 (1H, d, J = 8.5 Hz)

### (5) Production of methyl 4-((2-chloro-5-(trifluoromethyl)benzoyl)glycyl)benzoate

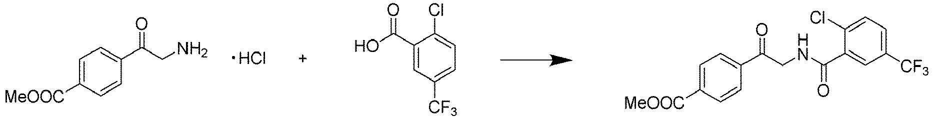

Methyl 4-glycylbenzoate hydrochloride (3.6 g, 16.1 mmol, 1.1 equiv) produced in (3), 2-chloro-5-(trifluoromethyl)benzoic acid (3.3 g, 14.7 mmol, 1.0 equiv) produced in (4), and anhydrous HOBt (2.1 g, 16.1 mmol, 1.1 equiv) were dissolved in dichloromethane (36.0 mL) and triethylamine (2.4 mL), followed by the addition of WSC·HCl (3.0 g, 16.1 mmol, 1.1 equiv). The mixture was stirred at room temperature overnight. Water (500 mL) was added to the resulting reaction liquid, followed by extraction with ethyl acetate. The resulting organic layer was washed with 1M hydrochloric acid, water, a saturated aqueous sodium hydrogencarbonate solution, and a saturated aqueous sodium chloride solution, dried over sodium sulfate, and filtered, and the filtrate was concentrated under reduced pressure. The residue was purified by column chromatography (SiO₂, SNAP ultra (10 g), ethyl acetate/hexane = 15% → 25%, methanol/ethyl acetate = 30%) to give methyl 4-((2-chloro-5-(trifluoromethyl)benzoyl)glycyl)benzoate as a white solid (1.8 g, 4.5 mmol, 30% yield).
¹H NMR (500 MHz, CDCl₃) δ 8.15 (2H, d, J = 8.0 Hz), 8.05 (2H, d, J = 8.0 Hz), 7.98 (1H, s), 7.62 (1H, d, J = 8.3 Hz), 7.48 (1H, d, J = 8.3 Hz), 5.00 (2H, s), 3.94 (3H, s)

### (6) Production of methyl 4-(2-(2-chloro-5-(trifluoromethyl)phenyl)oxazol-5-yl)benzoate

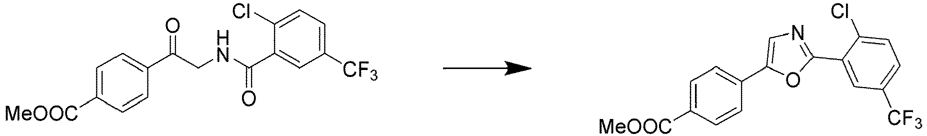

Methyl 4-((2-chloro-5-(trifluoromethyl)benzoyl)glycyl)benzoate (842.5 mg, 2.1 mmol) produced in (5) was dissolved in POCl₃ (14.0 mL), and the mixture was stirred under reflux for 6 hours. The resulting reaction liquid was allowed to cool to room temperature. Thereafter, ice-cold water (140 mL) was added, and a 2M sodium hydroxide aqueous solution was added to adjust the pH to 10, followed by extraction with ethyl acetate. The resulting organic layer was washed with water and a saturated aqueous sodium chloride solution, dried over sodium sulfate, and filtered. The filtrate was concentrated under reduced pressure. The residue was purified by column chromatography (SiO₂, SNAP ultra (10 g), ethyl acetate/hexane = 5% → 20%) to give methyl 4-(2-(2-chloro-5-(trifluoromethyl)phenyl)oxazol-5-yl)benzoate as a white solid (321.0 mg, 0.84 mmol, 40% yield).
¹H NMR (400 MHz, CDCl₃) δ 8.40 (1H, s), 8.13 (2H, d, J = 8.3 Hz), 7.81 (2H, d, J = 8.3 Hz), 7.65-7.70 (3H, m), 3.95 (3H, s)

### (7) Production of 4-(2-(2-chloro-5-(trifluoromethyl)phenyl)oxazol-5-yl)benzoic acid

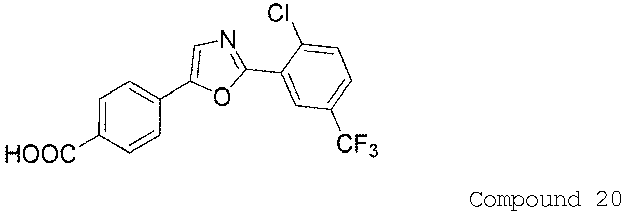

Methyl 4-(2-(2-chloro-5-(trifluoromethyl)phenyl)oxazol-5-yl)benzoate (321.0 mg, 0.84 mmol) produced in (6) was dissolved in THF (9.7 mL) and water (9.7 mL), followed by the addition of lithium hydroxide monohydrate (176.4 mg, 4.2 mmol). The mixture was stirred at room temperature overnight. A 1M hydrochloric acid aqueous solution was added to adjust the pH to 1 to 2, followed by extraction with ethyl acetate, drying over sodium sulfate, and filtration. The filtrate was concentrated under reduced pressure. The residue was purified by column chromatography (SiO₂, SNAP ultra (10 g), ethyl acetate/dichloromethane = 50% → 100%) to give 4-(2-(2-chloro-5-(trifluoromethyl)phenyl)oxazol-5-yl)benzoic acid as a white solid (160.3 mg, 0.43 mmol, 51% yield).
¹H NMR (500 MHz, CD₃OD) δ 8.41 (1H, s), 8.14 (2H, d, J = 8.5 Hz), 7.93 (2H, d, J = 8.5 Hz), 7.89 (1H, s), 7.83-7.84 (2H, m)

### Production Example 28

### 4-(3-(2-Chloro-5-(trifluoromethyl)phenyl)isoxazol-5-yl)benzoic acid (compound 21)

### (1) Production of 2-chloro-5-(trifluoromethyl)benzaldehyde oxime

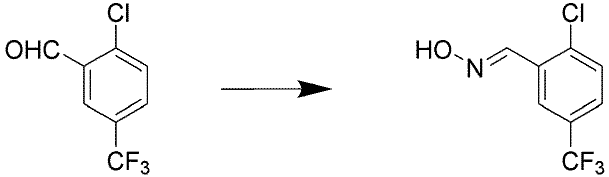

2-Chloro-5-(trifluoromethyl)benzaldehyde (522.0 mg, 2.5 mmol, 1.0 equiv) was dissolved in dehydrated ethanol (2.1 mL), followed by the addition of hydroxylamine hydrochloride (191.3 mg, 2.75 mmol, 1.1 equiv) and pyridine (296.5 mg, 3.75 mmol, 1.5 equiv). The liquid mixture was stirred under reflux for 3 hours. The resulting reaction liquid was allowed to cool to room temperature, and the solvent was removed under vacuum. Diethyl ether (15 mL) was added to the residue, and the mixture was washed with 1M hydrochloric acid and water, dried over sodium sulfate, and filtered. The filtrate was concentrated under reduced pressure to give 2-chloro-5-(trifluoromethyl)benzaldehyde oxime (476.9 mg, 2.1 mmol, 85% yield).
¹H NMR (400 MHz, CDCl₃) δ 8.57 (1H, s), 8.10 (1H, s), 7.56 (1H, d, J = 8.3 Hz), 7.53 (1H, d, J = 8.3 Hz)

### (2) Production of 2-chloro-N-hydroxy-5-(trifluoromethyl)benzimidoyl chloride

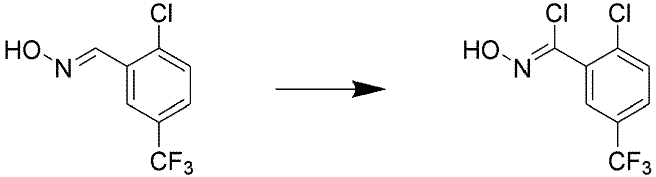

2-Chloro-5-(trifluoromethyl)benzaldehyde oxime (476.9 mg, 2.1 mmol, 1.0 equiv) produced in (1) was dissolved in DMF (0.54 mL), and the mixture was stirred at 0°C. NCS (298.6 mg, 2.23 mmol, 1.05 equiv) was added. About 1-2 mL of hydrogen chloride gas was added, and the reaction liquid was stirred from 0°C to room temperature overnight. Diethyl ether (33 mL) was added to the liquid mixture, and the mixture was washed with ice-cold water and saturated saline, dried with sodium sulfate, and filtered. The filtrate was concentrated under reduced pressure to give 2-chloro-N-hydroxy-5-(trifluoromethyl)benzimidoyl chloride (462.7 mg, 1.79 mmol, 84% yield).
¹H NMR (400 MHz, CDCl₃) δ 8.49 (1H, s), 7.74 (1H, s), 7.65 (1H, d, J = 8.5 Hz), 7.59 (1H, d, J = 8.5 Hz)

### (3) Ethyl 4-(3-(2-chloro-5-(trifluoromethyl)phenyl)isoxazol-5-yl)benzoate

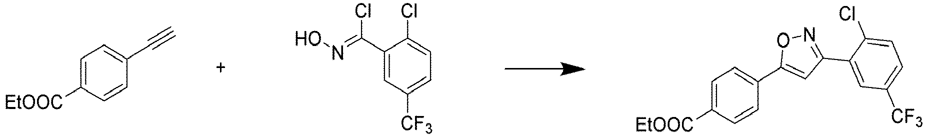

2-Chloro-N-hydroxy-5-(trifluoromethyl)benzimidoyl chloride (462.7 mg, 1.79 mmol, 1.0 equiv) produced in (2) and ethyl 4-ethynylbenzoate (327.4 mg, 1.87 mmol, 1.05 equiv) were dissolved in dichloromethane (14 mL), followed by the addition of triethylamine (271.5 mg, 2.68 mmol, 1.5 equiv). The mixture was stirred at room temperature for 48 hours. Dichloromethane (34 mL) was added to the liquid mixture, and the mixture was washed with a 1M sodium hydroxide aqueous solution and water, dried over sodium sulfate, and filtered. The filtrate was concentrated under reduced pressure. The residue was purified by column chromatography (SiO₂, SNAP ultra (10 g), ethyl acetate/hexane = 5% → 30%) to give ethyl 4-(3-(2-chloro-5-(trifluoromethyl)phenyl)isoxazol-5-yl)benzoate (259.8 mg, 0.65 mmol, 36% yield).
¹H NMR (400 MHz, CDCl₃) δ 8.17 (2H, d, J = 8.0 Hz), 8.09 (1H, s), 7.92 (2H, d, J = 8.0 Hz), 7.66 (2H, m), 7.11 (1H, s), 4.42 (2H, q, J = 7.1 Hz), 1.42 (3H, t, J = 7.1 Hz)

### (4) Production of 4-(3-(2-chloro-5-(trifluoromethyl)phenyl)isoxazol-5-yl)benzoic acid

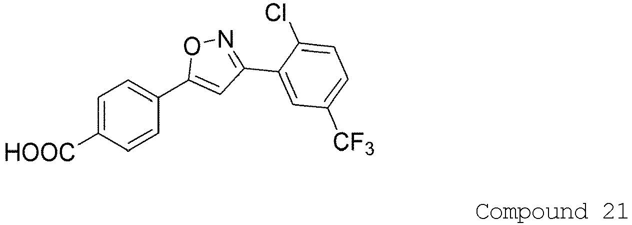

Ethyl 4-(3-(2-chloro-5-(trifluoromethyl)phenyl)isoxazol-5-yl)benzoate (259.8 mg, 0.65 mmol) produced in (3) was dissolved in THF (2.2 mL) and water (2.2 mL), followed by the addition of lithium hydroxide monohydrate (137.6 mg, 3.2 mmol). The mixture was stirred at room temperature overnight. A 1M hydrochloric acid aqueous solution was added to adjust the pH to 1 to 2, followed by extraction with ethyl acetate, drying over sodium sulfate, and filtration. The filtrate was concentrated under reduced pressure. The residue was purified by column chromatography (SiO₂, SNAP ultra (10 g), methanol/dichloromethane = 10%) to give 4-(3-(2-chloro-5-(trifluoromethyl)phenyl)isoxazol-5-yl)benzoic acid as a white solid (125.9 mg, 0.34 mmol, 52% yield).
¹H NMR (400 MHz, acetone-d₆) δ 8.22 (2H, d, J = 8.0 Hz), 8.11-8.13 (3H, m) 7.92 (2H, m), 7.59 (1H, s)

### Production Example 29

### 4-(1-(4-Isopropylphenyl)-1H-1,2,3-triazol-4-yl)benzoic acid (compound 22)

### (1) Production of 1-azido-4-isopropylbenzene

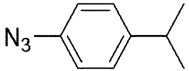

1-Azido-4-isopropylbenzene was obtained as a brown oily liquid (139.1 mg, 0.86 mmol, 86% yield) in the same manner as in the procedure described in Production Example 16(1), except that 4-isopropylaniline (135.1 mg, 1.0 mmol) was used in place of 4-(tert-butyl)aniline.
¹H NMR (400 MHz, CDCl₃) δ 7.27 (2H, d, J = 8.3 Hz), 7.02 (2H, d, J = 8.3 Hz), 2.96 (1H, sep, J = 7.1 Hz), 1.30 (6H, d, J = 7.1 Hz),

### (2) Production of 4-(1-(4-isopropylphenyl)-1H-1,2,3-triazol-4-yl)benzoic acid

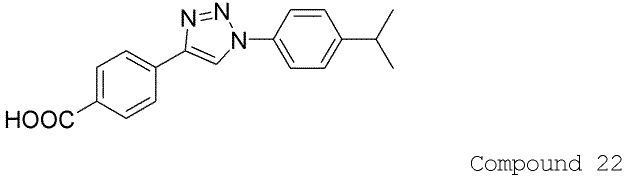

4-(1-(4-Isopropylphenyl)-1H-1,2,3-triazol-4-yl)benzoic acid was obtained as a light-brown solid (5.1 mg, 0.016 mmol, 23% yield) after purification by column chromatography (SiO₂, SNAP ultra (10 g), MeOH/CH₂Cl₂ = 5 → 14%) in the same manner as in the procedure described in Production Example 16(2), except that 1-azido-4-isopropylbenzene (11.3 mg, 0.07 mmol) was used in place of 1-azido-4-(tert-butyl)benzene.
¹H NMR (400 MHz, CD₃OD) δ 8.99 (1H, s), 8.11 (2H, d, J = 8.0 Hz), 8.01 (2H, d, J = 8.0 Hz), 7.83 (2H, d, J = 8.5 Hz), 7.49 (2H, d, J = 8.5 Hz), 3.03 (1H, sep, J = 7.1 Hz), 1.32 (6H, d, J = 7.1 Hz)

### Production Example 30

### 4-(1-(2-Chloro-5-(trifluoromethyl)phenyl)-1H-1,2,3-triazol-4-yl)benzoic acid (compound 23)

### (1) Production of 2-azido-1-chloro-4-(trifluoromethyl)benzene

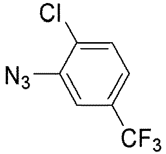

2-Azido-1-chloro-4-(trifluoromethyl)benzene was obtained as a brown oily liquid (191.3 mg, 0.86 mmol, 86% yield) in the same manner as in the procedure described in Production Example 16(1), except that 2-chloro-5-trifluoromethylaniline (195.5 mg, 1.0 mmol) was used in place of 4-(tert-butyl)aniline.
¹H NMR (400 MHz, CDCl₃) δ 7.36 (1H, d, J = 8.3 Hz), 7.26 (1H, s), 7.19 (1H, d, J = 8.3 Hz)

### (2) Production of 4-(1-(2-chloro-5-(trifluoromethyl)phenyl)-1H-1,2,3-triazol-4-yl)benzoic acid

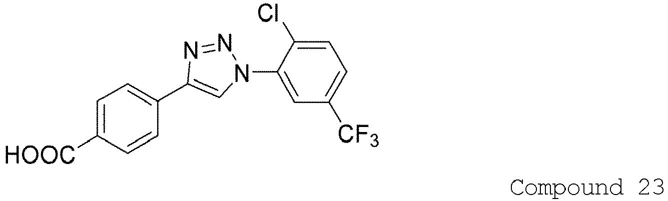

4-(1-(2-Chloro-5-(trifluoromethyl)phenyl)-1H-1,2,3-triazol-4-yl)benzoic acid was obtained as a white solid (6.1 mg, 0.017 mmol, 25% yield) after purification by column chromatography (SiO₂, SNAP ultra (10 g), MeOH/CH₂Cl₂ = 5 → 14%) in the same manner as in the procedure described in Production Example 16(2), except that 2-azido-1-chloro-4-(trifluoromethyl)benzene (17.7 mg, 0.07 mmol) was used in place of 1-azido-4-(tert-butyl)benzene.
¹H NMR (400 MHz, CD₃OD) δ 8.93 (1H, s), 8.14 (2H, d, J = 8.5 Hz), 8.11 (1H, s), 8.07 (2H, d, J = 8.5 Hz), 7.96-7.97 (2H, m)

### Production Example 31

### 4-(1-(2-Fluoro-5-(trifluoromethyl)phenyl)-1H-1,2,3-triazol-4-yl)benzoic acid (compound 24)

### (1) Production of 2-azido-1-fluoro-4-(trifluoromethyl)benzene

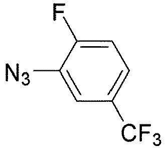

2-Azido-1-fluoro-4-(trifluoromethyl)benzene was obtained as a brown oily liquid (93.7 mg, 0.45 mmol, 45% yield) in the same manner as in the procedure described in Production Example 16(1), except that 2-fluoro-5-trifluoromethylaniline (179.1 mg, 1.0 mmol) was used in place of 4-(tert-butyl)aniline.
¹H NMR (400 MHz, CDCl₃) δ 7.36 (1H, s), 7.31 (1H, d, J = 8.5 Hz), 7.21 (1H, d, J = 8.5 Hz)

### (2) Production of 4-(1-(2-fluoro-5-(trifluoromethyl)phenyl)-1H-1,2,3-triazol-4-yl)benzoic acid

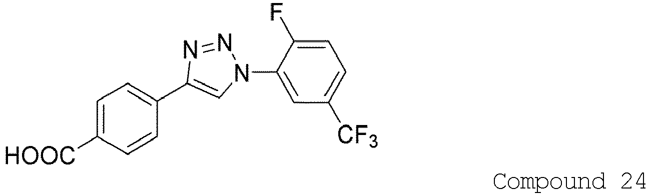

4-(1-(2-Fluoro-5-(trifluoromethyl)phenyl)-1H-1,2,3-triazol-4-yl)benzoic acid was obtained as a light-brown solid (72.7 mg, 0.20 mmol, 32% yield) after purification by column chromatography (SiO₂, SNAP ultra (10 g), ethyl acetate/chloroform = 5 → 8%) in the same manner as the procedure described in Production Example 16(2), except that 2-azido-1-fluoro-4-(trifluoromethyl)benzene (130.6 mg, 0.63 mmol) was used in place of 1-azido-4-(tert-butyl)benzene.
¹H NMR (400 MHz, CD₃OD) δ 8.98 (1H, s), 8.32 (1H, d, J = 8.0 Hz), 8.13 (2H, d, J = 8.0 Hz), 8.08 (2H, d, J = 8.0 Hz), 7.97 (1H, s), 7.73 (1H, d, J = 8.0 Hz); ESI-MS m/z 352.05 [M + H]⁺

### Production Example 32

### 4-(3-(2-Chloro-5-(trifluoromethyl)phenyl)-1H-1,2,4-triazol-5-yl)benzoic acid (compound 25)

### (1) Production of 2-chloro-5-(trifluoromethyl)benzohydrazide

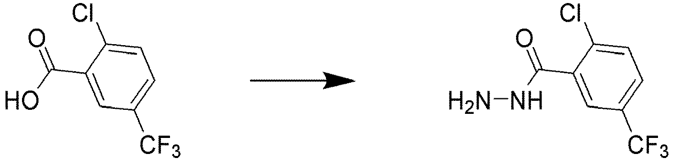

2-Chloro-5-(trifluoromethyl)benzoic acid (381.8 mg, 1.7 mmol, 1.0 equiv), dehydrated methanol (1.7 mL, 41.6 mmol, 24.5 equiv), and sulfuric acid (46 µL, 0.85 mmol, 0.5 equiv) were mixed, and the mixture was stirred under reflux for 4 hours. The resulting reaction liquid was allowed to cool to room temperature. Hydrazine monohydrate (537 µL, 11.0 mmol, 6.5 equiv) was added, and the mixture was vigorously stirred under reflux overnight. A saturated aqueous sodium carbonate solution was added, followed by extraction with ethyl acetate. The organic layer was washed with a saturated sodium chloride solution, dried with sodium sulfate, and filtered. The filtrate was concentrated to dryness. The residue was washed with hexane and diisopropyl ether and dried to obtain 2-chloro-5-(trifluoromethyl)benzohydrazide (160.9 mg, 0.67 mmol, 39% yield).
¹H NMR (400 MHz, CDCl₃) δ 7.89 (1H, s), 7.87 (1H, br), 7.63 (1H, d, J = 8.5 Hz), 7.55 (1H, d, J = 8.5 Hz), 4.17 (2H, br)

### (2) Production of ethyl 4-(3-(2-chloro-5-(trifluoromethyl)phenyl)-1H-1,2,4-triazol-5-yl)benzoate

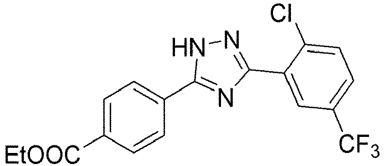

Methyl 4-cyanobenzoate (197.0 mg, 1.2 mmol) was dissolved in methanol (2.6 mL), followed by stirring at 0°C. Acetyl chloride (3.6 mL) was added dropwise. The liquid mixture was stirred from 0°C to room temperature overnight, and the solvent was removed under vacuum. The resulting solid was washed with diethyl ether, dried, and immediately used for the next reaction.

Sodium methoxide (5M methanol solution) (0.15 mL, 0.752 mmol, 1.28 equiv) was dissolved in ethanol (6.3 mL), followed by stirring. A solution of the residue described above in ethanol (2 mL) was added thereto. The mixture was stirred at room temperature for 30 minutes and filtered, and the filtrate was concentrated under reduced pressure. To the mixture, 2-chloro-5-(trifluoromethyl)benzohydrazide (140.1 mg, 0.58 mmol, 1.0 equiv) produced in (1) and dioxane (2 mL) were added, and the mixture was stirred under reflux overnight. A 1M sodium hydroxide aqueous solution was added, followed by extraction with ethyl acetate, drying over sodium sulfate, and filtration. The filtrate was concentrated under reduced pressure. The residue was purified by column chromatography (SiO₂, SNAP ultra (10 g), ethyl acetate/hexane = 5% → 30%) to give ethyl 4-(3-(2-chloro-5-(trifluoromethyl)phenyl)-1H-1,2,4-triazol-5-yl)benzoate (2.9 mg, 0.0073 mmol, 1.2% yield).
¹H NMR (400 MHz, CD₃OD) δ 8.15-8.22 (5H, m), 7.83 (2H, m), 4.41 (2H, q, J = 7.1 Hz), 1.42 (3H, t, J = 7.1 Hz); ESI-MS m/z 396.25 [M + H]⁺

### (3) Production of 4-(3-(2-chloro-5-(trifluoromethyl)phenyl)-1H-1,2,4-triazol-5-yl)benzoic acid

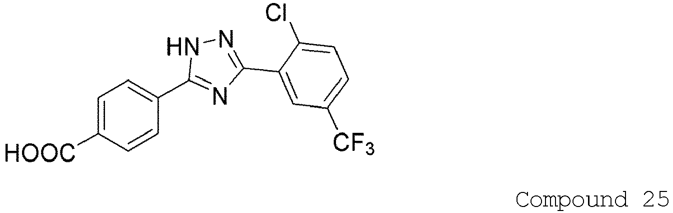

Ethyl 4-(3-(2-chloro-5-(trifluoromethyl)phenyl)-1H-1,2,4-triazol-5-yl)benzoate (2.9 mg, 0.0075 mmol) produced in (2) was dissolved in THF (0.1 mL) and water (0.1 mL), followed by the addition of lithium hydroxide monohydrate (1.6 mg, 0.037 mmol). The mixture was stirred at room temperature overnight and neutralized by the addition of a 1M HCl aqueous solution, followed by extraction with ethyl acetate, drying over sodium sulfate, and filtration. The filtrate was concentrated under reduced pressure to give 4-(3-(2-chloro-5-(trifluoromethyl)phenyl)-1H-1,2,4-triazol-5-yl)benzoic acid as a white solid (2.5 mg, 0.0067 mmol, 89% yield).
¹H NMR (400 MHz, CD₃OD) δ 8.17-8.21 (4H, m), 7.74-7.82 (3H, m); ESI-MS m/z 368.10 [M + H]⁺

### Production Example 33

### 4-(5-(4-Isopropylphenyl)-1H-imidazol-2-yl)benzoic acid (compound 26)

### (1) Production of methyl 4-((4-isopropylbenzoyl)glycyl)benzoate

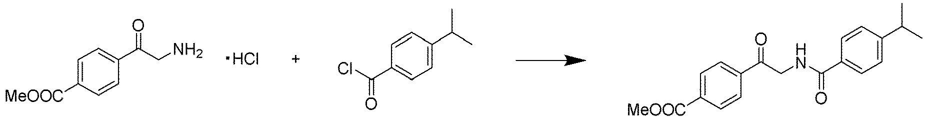

Methyl 4-glycylbenzoate hydrochloride (293.9 mg, 1.28 mmol, 1.0 equiv) produced in Production Example 20(3), 4-isopropylbenzoyl chloride (233.7 mg, 1.28 mmol, 1.0 equiv), and triethylamine (517.6 mg, 5.12 mmol, 4.0 equiv) were dissolved in dichloromethane (7.0 mL), and the mixture was stirred at room temperature for 30 minutes. The solvent was removed under vacuum, and the residue was purified by column chromatography (SiO₂, SNAP ultra (10 g), ethyl acetate/dichloromethane = 0% → 17%) to give methyl 4-((4-isopropylbenzoyl)glycyl)benzoate (87.1 mg, 0.25 mmol, 19% yield).
¹H NMR (400 MHz, CDCl₃) δ 8.17 (2H, d, J = 7.4 Hz), 8.08 (2H, d, J = 8.5 Hz), 7.81 (2H, d, J = 8.5 Hz), 7.32 (2H, t, J = 7.4 Hz), 7.22 (1H, s), 4.99 (2H, s), 3.96 (3H, s), 2.96 (1H, sep, J = 6.7 Hz), 1.27 (6H, d, J = 6.7 Hz)

### (2) Production of methyl 4-(5-(4-isopropylphenyl)-1H-imidazol-2-yl)benzoate

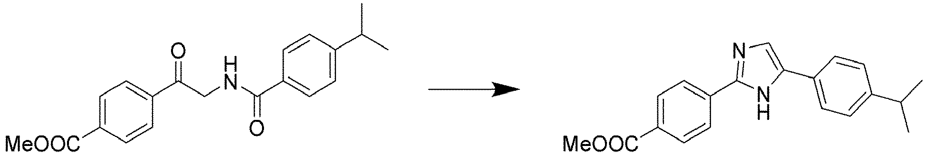

Methyl 4-((4-isopropylbenzoyl)glycyl)benzoate (87.1 mg, 0.25 mmol, 1.0 equiv) produced in (1) and ammonium acetate (157.8 mg, 2.04 mmol, 8.0 equiv) were dissolved in acetic acid (0.8 mL), and the mixture was stirred under reflux for 2 hours. The solvent was removed under vacuum, and the resulting solid was dissolved in ethanol/water/dichloromethane = 1:1:1. The dichloromethane layer was washed with a saturated aqueous sodium chloride solution, dried over sodium sulfate, and filtered. The filtrate was concentrated under reduced pressure to give methyl 4-(5-(4-isopropylphenyl)-1H-imidazol-2-yl)benzoate (7.6 mg, 0.023 mmol, 9% yield).
¹H NMR (400 MHz, CDCl₃) δ 7.97 (2H, d, J = 8.5 Hz), 7.84 (2H, d, J = 7.6 Hz), 7.71 (2H, d, J = 7.6 Hz), 7.20 (1H, s), 7.19 (2H, d, J = 8.5 Hz), 3.90 (3H, s), 2.82 (1H, sep, J = 7.1 Hz), 1.18 (6H, d, J = 7.1 Hz)

### (3) Production of 4-(5-(4-isopropylphenyl)-1H-imidazol-2-yl)benzoic acid

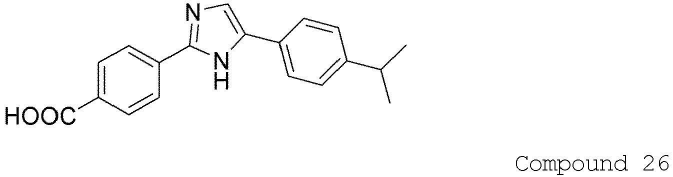

Methyl 4-(5-(4-isopropylphenyl)-1H-imidazol-2-yl)benzoate (7.6 mg, 0.023 mmol, 1.0 equiv) produced in (2) was dissolved in THF (0.3 mL) and water (0.3 mL), followed by the addition of lithium hydroxide monohydrate (5.0 mg, 0.11 mmol, 5 equiv). The mixture was stirred at room temperature overnight and neutralized by the addition of a 1M hydrochloric acid aqueous solution, followed by extraction with ethyl acetate, drying over sodium sulfate, and filtration. The filtrate was concentrated under reduced pressure. The residue was purified by column chromatography (SiO₂, SNAP ultra (10 g), ethyl acetate/dichloromethane = 80% → 100%) to give 4-(5-(4-isopropylphenyl)-1H-imidazol-2-yl)benzoic acid as a white solid (2.7 mg, 0.008 mmol, 34% yield).
¹H NMR (400 MHz, CD₃OD) δ 8.04 (2H, d, J = 8.5 Hz), 7.88 (2H, d, J = 8.3 Hz), 7.87 (2H, d, J = 8.3 Hz), 7.62 (1H, s) 7.37 (2H, d, J = 8.5 Hz), 2.96 (1H, sep, J = 7.1 Hz), 1.29 (2H, d, J = 7.1 Hz)

### Production Example 34

### 4-(5-(2-Chloro-5-(trifluoromethyl)phenyl)-1H-imidazol-2-yl)benzoic acid (compound 27)

### (1) Production of methyl 4-(5-(2-chloro-5-(trifluoromethyl)phenyl)-1H-imidazol-2-yl)benzoate

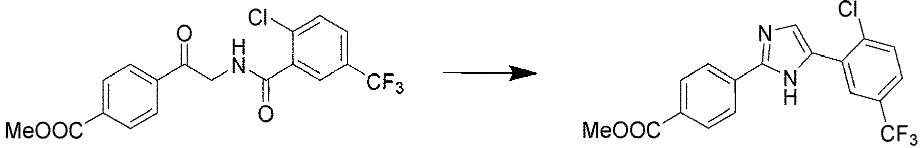

Methyl 4-((2-chloro-5-(trifluoromethyl)benzoyl)glycyl)benzoate (792.0 mg, 1.98 mmol, 1.0 equiv) produced in Production Example 20(5) and ammonium acetate (1.2 g, 15.5 mmol, 8.0 equiv) were dissolved in acetic acid (6.2 mL), and the mixture was stirred under reflux for 2 hours. The solvent was removed under vacuum, and the resulting solid was dissolved in ethanol/water/dichloromethane = 1:1:1. The dichloromethane layer was washed with a saturated aqueous sodium chloride solution, dried over sodium sulfate, and filtered. The filtrate was concentrated under reduced pressure to give methyl 4-(5-(2-chloro-5-(trifluoromethyl)phenyl)-1H-imidazol-2-yl)benzoate (267.2 mg, 0.7 mmol, 35% yield).
¹H NMR (400 MHz, CDCl₃) δ 10.49 (1H, br), 8.72 (1H, s), 8.08 (2H, d, J = 8.9 Hz), 7.89-7.91 (2H, m), 7.56 (2H, d, J = 8.9 Hz), 7.55 (1H, m), 3.93 (3H, s); ESI-MS m/z 380.80 [M + H]⁺

### (2) Production of 4-(5-(2-chloro-5-(trifluoromethyl)phenyl)-1H-imidazol-2-yl)benzoic acid

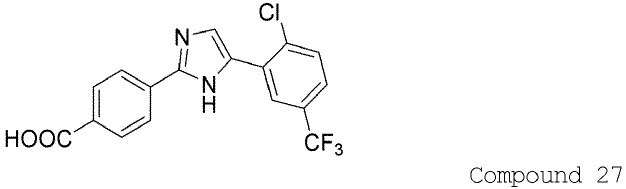

Methyl 4-(5-(2-chloro-5-(trifluoromethyl)phenyl)-1H-imidazol-2-yl)benzoate (267.2 mg, 0.7 mmol, 1.0 equiv) produced in (1) was dissolved in THF (8 mL) and water (8 mL), followed by the addition of lithium hydroxide monohydrate (146.8 mg, 3.5 mmol, 5.0 equiv). The mixture was stirred at room temperature overnight and neutralized by the addition of a 1M hydrochloric acid aqueous solution, followed by extraction with ethyl acetate, drying over sodium sulfate, and filtration. The filtrate was concentrated under reduced pressure. The residue was purified by column chromatography (SiO₂, SNAP ultra (10 g), ethyl acetate/dichloromethane = 80% → 100%) to give 4-(5-(2-chloro-5-(trifluoromethyl)phenyl)-1H-imidazol-2-yl)benzoic acid as a white solid (140.7 mg, 0.383 mmol, 54% yield).
¹H NMR (400 MHz, CD₃OD) δ 8.14 (1H, s), 8.06 (2H, d, J = 8.3 Hz), 7.90 (2H, d, J = 8.3 Hz), 7.75-7.81 (3H, m)

### Production Example 35

### 4-(5-(4-Isopropylphenyl)-1H-pyrazol-3-yl)benzoic acid (compound 28)

### (1) Production of methyl 4-(5-(4-isopropylphenyl)-1H-pyrazol-3-yl)benzoate

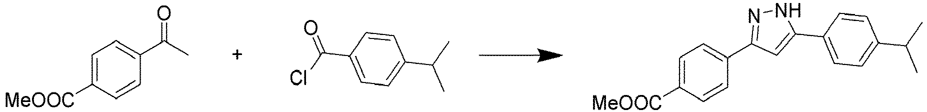

Methyl 4-acetylbenzoate (16.4 mg, 0.1 mmol, 1.0 equiv) was dissolved in toluene (0.25 mL), followed by stirring at 0°C. LiHMDS (1M THF solution, 105.0 µL, 0.105 mmol, 1.05 equiv) was quickly added. The liquid mixture was stirred at 0°C for 1 minute, and then 4-isopropylbenzoyl chloride (9.1 mg, 0.05 mmol, 0.5 equiv) was added with a syringe. The reaction vessel was removed from the ice bath, and the mixture was stirred for 1 minute, followed by the addition of acetic acid (0.1 mL), ethanol (0.5 mL), THF (0.25 mL), and hydrazine monohydrate (0.3 mL). Thereafter, the reaction liquid was stirred under reflux for one and a half hours, and then a 1M sodium hydroxide aqueous solution was added, followed by extraction with ethyl acetate. The organic layer was washed with a saturated sodium chloride solution, dried over sodium sulfate, and filtered. The filtrate was concentrated to dryness. The residue was purified by column chromatography (SiO₂, SNAP ultra (10 g), ethyl acetate/dichloromethane = 0% → 50%) to give methyl 4-(5-(4-isopropylphenyl)-1H-pyrazol-3-yl)benzoate (13.3 mg, 0.041 mmol, 83% yield).
¹H NMR (400 MHz, CD₃OD) δ 8.06 (2H, d, J = 7.1 Hz), 7.95 (2H, d, J = 6.7 Hz), 7.67 (2H, d, J = 6.7 Hz), 7.32 (2H, d, J = 7.1 Hz), 7.05 (1H, s), 2.94 (1H, sep, J = 7.1 Hz), 1.28 (6H, d, J = 7.1 Hz)

### (2) Production of 4-(5-(4-isopropylphenyl)-1H-pyrazol-3-yl)benzoic acid

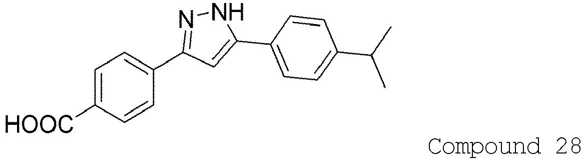

Methyl 4-(5-(4-isopropylphenyl)-1H-pyrazol-3-yl)benzoate (13.3 mg, 0.041 mmol, 1.0 equiv) produced in (1) was dissolved in THF (0.5 mL) and water (0.5 mL), followed by the addition of lithium hydroxide monohydrate (8.7 mg, 0.2 mmol, 5.0 equiv). The mixture was stirred at room temperature overnight and neutralized by the addition of a 1M hydrochloric acid aqueous solution, followed by extraction with ethyl acetate, drying over sodium sulfate, and filtration. The filtrate was concentrated under reduced pressure. The residue was purified by column chromatography (SiO₂, SNAP ultra (10 g), ethyl acetate/dichloromethane = 80% → 100%) to give 4-(5-(4-isopropylphenyl)-1H-pyrazol-3-yl)benzoic acid as a white solid (6.4 mg, 0.02 mmol, 50% yield).
¹H NMR (400 MHz, CD₃OD) δ 8.09 (2H, d, J = 8.3 Hz), 7.93 (2H, d, J = 8.0 Hz), 7.71 (2H, d, J = 8.0 Hz), 7.32 (2H, d, J = 8.3 Hz), 7.07 (1H, s), 2.95 (1H, sep, J = 6.7 Hz), 1.28 (6H, d, J = 6.7 Hz)

### Production Example 36

### 4-(5-(2-Chloro-5-(trifluoromethyl)phenyl)-1H-pyrazol-3-yl)benzoic acid (compound 29)

### (1) Production of methyl 4-(5-(2-chloro-5-(trifluoromethyl)phenyl)-1H-pyrazol-3-yl)benzoate

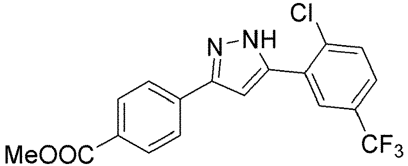

2-Chloro-5-(trifluoromethyl)benzoic acid (529.9 mg, 2.35 mmol, 1.0 equiv) produced in Production Example 27 was dissolved in dichloromethane (8.5 mL) and DMF (2.8 mL). The mixture was stirred at 0°C, and oxalyl dichloride (242 µL, 2.82 mmol, 1.2 equiv) was added dropwise. The reaction liquid was stirred at room temperature for 3 hours and concentrated under reduced pressure to give 2-chloro-5-(trifluoromethyl)benzoyl chloride. 2-Chloro-5-(trifluoromethyl)benzoyl chloride obtained was immediately used in the next reaction.

Methyl 4-(5-(2-chloro-5-(trifluoromethyl)phenyl)-1H-pyrazol-3-yl)benzoate (29.0 mg, 0.076 mmol, 100% yield) was obtained by performing column chromatography (SiO₂, SNAP ultra (10 g), ethyl acetate/dichloromethane = 0% → 50%) in the same manner as in the procedure described in Production Example 35(1), except that 2-chloro-5-(trifluoromethyl)benzoyl chloride (17.0 mg, 0.07 mmol) produced above was used in place of 4-isopropylbenzoyl chloride.
¹H NMR (400 MHz, CD₃OD) δ 8.10 (1H, s), 8.09 (2H, d, J = 9.4 Hz), 7.93 (2H, m), 7.72 (1H, d, J = 8.5 Hz), 7.67 (1H, m), 7.27 (1H, s), 3.93 (3H, s)

### (2) Production of 4-(5-(2-chloro-5-(trifluoromethyl)phenyl)-1H-pyrazol-3-yl)benzoic acid

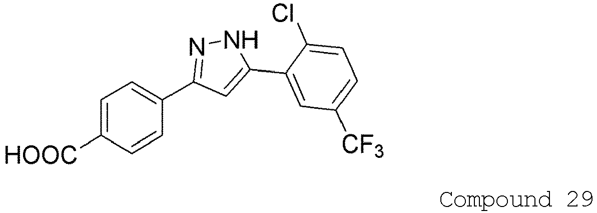

Methyl 4-(5-(2-chloro-5-(trifluoromethyl)phenyl)-1H-pyrazol-3-yl)benzoate (29.0 mg, 0.076 mmol, 1.0 equiv) produced in (1) was dissolved in THF (0.9 mL) and water (0.9 mL), followed by the addition of lithium hydroxide monohydrate (15.9 mg, 0.38 mmol, 5 equiv). The mixture was stirred at room temperature overnight and neutralized by the addition of a 1M hydrochloric acid aqueous solution, followed by extraction with ethyl acetate, drying over sodium sulfate, and filtration. The filtrate was concentrated under reduced pressure. The residue was purified by column chromatography (SiO₂, SNAP ultra (10 g), ethyl acetate/dichloromethane = 80% → 100%) to give 4-(5-(2-chloro-5-(trifluoromethyl)phenyl)-1H-pyrazol-3-yl)benzoic acid as a white solid (5.2 mg, 0.014 mmol, 18% yield).
¹H NMR (400 MHz, CD₃OD) δ 8.11 (2H, d, J = 8.3 Hz), 8.05 (1H, s), 7.91 (2H, d, J = 8.3 Hz), 7.76 (1H, d, J = 8.0 Hz), 7.68 (1H, d, J = 8.0 Hz), 7.24 (1H, s)

### Production Example 37

### 4-(3-(2-Chloro-5-(trifluoromethyl)phenyl)-1H-pyrazol-1-yl)benzoic acid (compound 30)

### (1) Production of 3-(2-chloro-5-(trifluoromethyl)phenyl)-1H-pyrazole

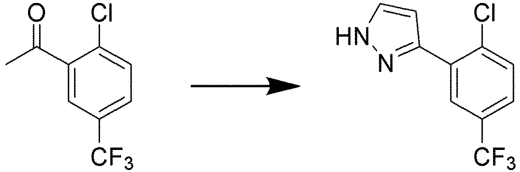

1-(2-Chloro-5-(trifluoromethyl)phenyl)ethan-1-one (556.4 mg, 2.5 mmol) and dimethylformamide dimethyl acetal (2.9 mL) were mixed, followed by stirring at 115°C overnight. The resulting reaction liquid was allowed to cool to room temperature, and then the solvent was removed under vacuum. Hydrazine monohydrate (0.5 mL) and acetic acid (4 mL) were added to the residue, and the mixture was stirred at 109°C overnight and then allowed to cool to room temperature. Water (40 mL) was added, followed by extraction with ethyl acetate. The organic layer was washed with a saturated sodium chloride solution, dried over sodium sulfate, and filtered. The filtrate was concentrated to dryness to give 3-(2-chloro-5-(trifluoromethyl)phenyl)-1H-pyrazole (652.7 mg, 2.5 mmol, 100% yield).
¹H NMR (400 MHz, CDCl₃) δ 7.91 (1H, s), 7.66 (1H, d, J = 2.0 Hz), 7.58 (1H, d, J = 8.2 Hz), 7.52 (1H, d, J = 8.2 Hz), 6.81 (1H, d, J = 2.0 Hz)

### (2) Production of methyl 4-(3-(2-chloro-5-(trifluoromethyl)phenyl)-1H-pyrazol-1-yl)benzoate

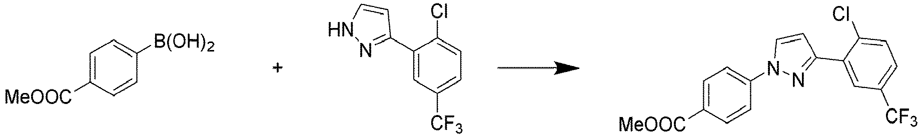

3-(2-Chloro-5-(trifluoromethyl)phenyl)-1H-pyrazole (100.0 mg, 0.4 mmol, 1.0 equiv) produced in (1), (4-(methoxycarbonyl)phenyl)boronic acid (216.0 mg, 1.2 mmol, 3.0 equiv), and copper(II) acetate (77.7 mg, 0.43 mmol, 1.07 equiv) were dissolved in pyridine (72 µL) and DMF (2 mL), followed by stirring at 60°C overnight. The resulting reaction liquid was allowed to cool to room temperature, and then a 1M hydrochloric acid aqueous solution (120 mL) was added, followed by filtration through a glass filter. The filtrate was removed, and ethyl acetate was passed through the solid on the glass filter. The resulting filtrate was concentrated under reduced pressure. The residue was purified by column chromatography (SiO₂, SNAP ultra (10 g), ethyl acetate/hexane = 0% → 5%) to give methyl 4-(3-(2-chloro-5-(trifluoromethyl)phenyl)-1H-pyrazol-1-yl)benzoate (35.8 mg, 0.094 mmol, 23% yield).
¹H NMR (400 MHz, CDCl₃) δ 8.23 (1H, s), 8.17 (2H, d, J = 8.9 Hz), 8.09 (1H, d, J = 2.6 Hz), 7.87 (2H, d, J = 8.9 Hz), 7.60 (1H, d, J = 8.3 Hz), 7.55 (1H, d, J = 8.3 Hz), 7.09 (1H, d, J = 2.6 Hz), 3.95 (3H, s)

### (3) Production of 4-(3-(2-chloro-5-(trifluoromethyl)phenyl)-1H-pyrazol-1-yl)benzoic acid

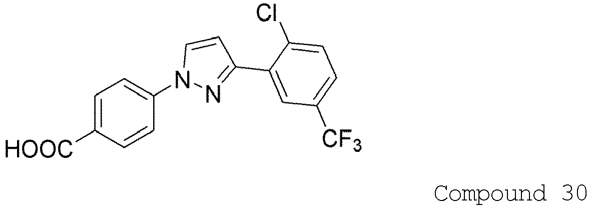

Methyl 4-(3-(2-chloro-5-(trifluoromethyl)phenyl)-1H-pyrazol-1-yl)benzoate (14.0 mg, 0.036 mmol) produced in (2) was dissolved in ethanol (1.0 mL), followed by the addition of a 1M sodium hydroxide aqueous solution (73.5 µL). The mixture was stirred under reflux for 2 hours. The resulting reaction liquid was allowed to cool to room temperature, and then the solvent was removed under vacuum. A 1M hydrochloric acid aqueous solution was added to the residue for acidification, followed by extraction with ethyl acetate. The organic layer was washed with a saturated aqueous sodium chloride solution, dried over sodium sulfate, and filtered. The filtrate was concentrated under reduced pressure to give 4-(3-(2-chloro-5-(trifluoromethyl)phenyl)-1H-pyrazol-1-yl)benzoic acid as a light yellow solid (7.0 mg, 0.019 mmol, 52% yield).
¹H NMR (400 MHz, CD₃OD) δ 8.48 (1H, d, J = 2.6 Hz), 8.23 (1H, s), 8.18 (2H, d, J = 8.9 Hz), 8.01 (2H, d, J = 8.9 Hz), 7.74 (1H, d, J = 8.5 Hz), 7.68 (1H, d, J = 8.5 Hz), 7.14 (1H, d, J = 2.6 Hz)

### Production Example 38

### 4-(5-(4-Isopropylphenyl)-1,3,4-oxadiazol-2-yl)benzoic acid (compound 31)

### (1) Production of 4-isopropylbenzohydrazide

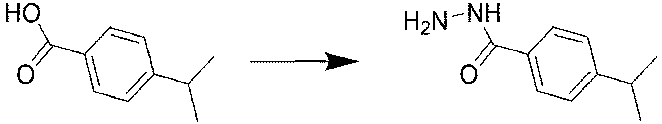

4-Isopropylbenzoic acid (246.3 mg, 1.5 mmol, 1.0 equiv), dehydrated methanol (1.5 mL, 36.8 mmol, 24.5 equiv), and sulfuric acid (40 µL, 0.75 mmol, 0.5 equiv) were mixed, followed by stirring under reflux for 4 hours. The resulting reaction liquid was allowed to cool to room temperature. Hydrazine monohydrate (473 µL, 9.75 mmol, 6.5 equiv) was added, and the mixture was vigorously stirred under reflux for 30 minutes. A saturated aqueous sodium carbonate solution was added, followed by extraction with ethyl acetate. The organic layer was washed with a saturated sodium chloride solution, dried over sodium sulfate, and filtered. The filtrate was concentrated to dryness to give 4-isopropylbenzohydrazide (146.7 mg, 0.82 mmol, 54% yield).
¹H NMR (400 MHz, CD₃OD) δ 7.71 (2H, d, J = 8.5 Hz), 7.31 (2H, d, J = 8.5 Hz), 2.93 (1H, sep, J = 7.1 Hz), 1.25 (6H, d, J = 7.1 Hz); ESI-MS m/z 178.90 [M + H]⁺

### (2) Production of methyl 4-((2-(4-isopropylbenzoyl)hydrazineylidene)methyl)benzoate

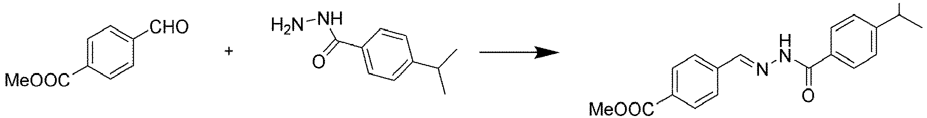

4-Isopropylbenzohydrazide (146.7 mg, 0.82 mmol, 1.0 equiv) produced in (1) and sodium acetate (135.0 mg, 1.64 mmol, 2.0 equiv) were dissolved in water (1.6 mL), and methyl 4-formylbenzoate (135.1 mg, 0.82 mmol, 1.0 equiv) was slowly added, followed by stirring at 40°C overnight. The resulting liquid mixture was filtered, and the resulting solid was washed with water and dried to give methyl 4-((2-(4-isopropylbenzoyl)hydrazineylidene)methyl)benzoate (260.4 mg, 0.80 mmol, 97% yield).
¹H NMR (400 MHz, CD₃OD) δ 8.37 (1H, s), 8.05 (2H, d, J = 8.5 Hz), 7.92 (2H, d, J = 8.0 Hz), 7.88 (2H, d, J = 8.0 Hz), 7.39 (2H, d, J = 8.5 Hz), 3.91 (3H, s), 2.99 (1H, sep, J = 7.1 Hz), 1.28 (6H, d, J = 7.1 Hz)

### (3) Production of methyl 4-(5-(4-isopropylphenyl)-1,3,4-oxadiazol-2-yl)benzoate

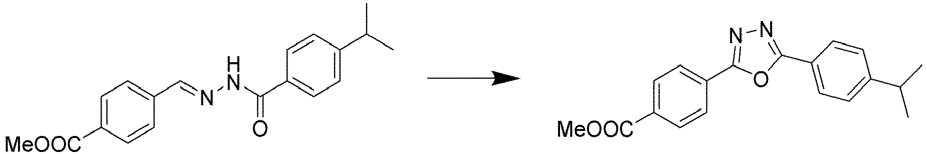

Methyl 4-((2-(4-isopropylbenzoyl)hydrazineylidene)methyl)benzoate (260.4 mg, 0.80 mmol, 1.0 equiv) produced in (2) and sodium acetate (131.5 mg, 1.60 mmol, 2.0 equiv) were dissolved in acetic acid (2.7 mL). A solution of bromine (142.2 mg, 0.89 mmol, 1.1 equiv) in acetic acid (0.9 mL) was added dropwise, and the mixture was stirred at 60°C for 2 hours. The resulting reaction liquid was allowed to cool to room temperature, and then ice was added. The liquid mixture was filtered. The resulting solid was washed with water, dried, and purified by column chromatography (SiO₂, SNAP ultra (10 g), ethyl acetate/hexane = 0% → 17%) to give methyl 4-(5-(4-isopropylphenyl)-1,3,4-oxadiazol-2-yl)benzoate (13.5 mg, 0.041 mmol, 5% yield).
¹H NMR (400 MHz, CD₃OD) δ 8.23 (2H, d, J = 8.3 Hz), 8.23 (2H, d, J = 8.5 Hz), 8.08 (2H, d, J = 8.5 Hz), 7.49 (2H, d, J = 8.3 Hz), 3.95 (3H, s), 3.00 (1H, sep, J = 7.1 Hz), 1.31 (6H, d, J = 7.1 Hz); ESI-MS m/z 323.05 [M + H]+

### (4) Production of 4-(5-(4-isopropylphenyl)-1,3,4-oxadiazol-2-yl)benzoic acid

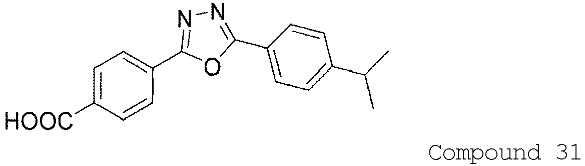

Methyl 4-(5-(4-isopropylphenyl)-1,3,4-oxadiazol-2-yl)benzoate (13.5 mg, 0.041 mmol, 1.0 equiv) produced in (3) was dissolved in THF (0.9 mL) and water (0.9 mL), followed by the addition of lithium hydroxide monohydrate (8.7 mg, 0.20 mmol, 5 equiv). The mixture was stirred at room temperature for 3 hours, acidified by the addition of a 1M hydrochloric acid aqueous solution, ice-cooled, and filtered. The resulting solid was washed with water and dried to give 4-(5-(4-isopropylphenyl)-1,3,4-oxadiazol-2-yl)benzoic acid as a white solid (8.0 mg, 0.025 mmol, 62% yield).
¹H NMR (400 MHz, CD₃OD) δ 8.26 (2H, d, J = 8.5 Hz), 8.22 (2H, d, J = 8.5 Hz), 8.10 (2H, d, J = 8.5 Hz), 7.50 (2H, d, J = 8.5 Hz), 3.03 (1H, sep, J = 6.7 Hz), 1.31 (6H, d, J = 6.7 Hz)

### Production Example 39

### 4-(5-(2-Chloro-5-(trifluoromethyl)phenyl)-1,3,4-oxadiazol-2-yl)benzoic acid (compound 32)

### (1) Production of methyl 4-((2-(4-isopropylbenzoyl)hydrazineylidene)methyl)benzoate

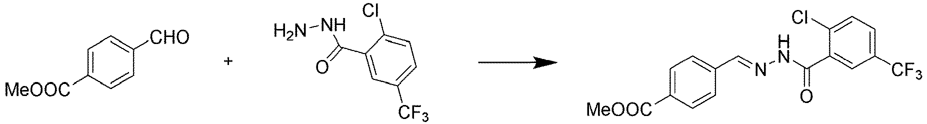

Methyl 4-((2- (4-isopropylbenzoyl)hydrazineylidene)methyl)benzoate (219.3 mg, 0.56 mmol, 85% yield) was obtained in the same manner as in the procedure described in Production Example 38(2), except that 2-chloro-5-(trifluoromethyl)benzohydrazide (156.5 mg, 0.65 mmol) produced in Production Example 32(1) was used in place of 4-isopropylbenzohydrazide.
¹H NMR (400 MHz, CD₃OD) δ 8.27 (1H, s), 8.09 (2H, d, J = 8.5 Hz), 7.95 (2H, d, J = 8.5 Hz), 7.84 (1H, d, J = 8.3 Hz), 7.78 (1H, s), 7.50 (2H, d, J = 8.3 Hz), 3.93 (3H, s)

### (2) Production of methyl 4-(5-(2-chloro-5-(trifluoromethyl)phenyl)-1,3,4-oxadiazol-2-yl)benzoate

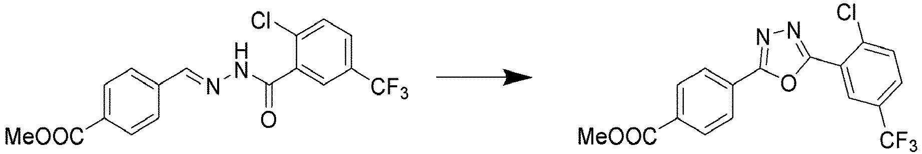

Methyl 4-(5-(2-chloro-5-(trifluoromethyl)phenyl)-1,3,4-oxadiazol-2-yl)benzoate (17.3 mg, 0.045 mmol, 11% yield) was obtained in the same manner as in the procedure described in Production Example 38(3), except that methyl 4-((2-(4-isopropylbenzoyl)hydrazineylidene)methyl)benzoate (153.8 mg, 0.40 mmol) produced in (1) was used in place of methyl 4-((2-(4-isopropylbenzoyl)hydrazineylidene)methyl)benzoate.
¹H NMR (400 MHz, CD₃OD) δ 8.45 (1H, s), 8.29 (2H, d, J = 8.7 Hz), 8.24 (2H, d, J = 8.7 Hz), 8.20 (1H, d, J = 8.0 Hz), 7.58 (1H, d, J = 8.0 Hz), 3.96 (3H, s)

### (3) Production of 4-(5-(2-chloro-5-(trifluoromethyl)phenyl)-1,3,4-oxadiazol-2-yl)benzoic acid

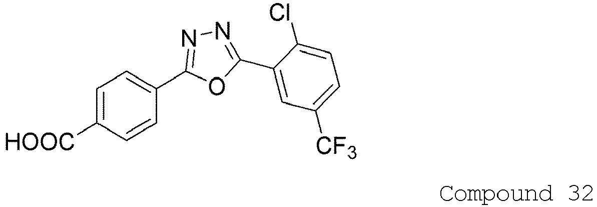

Methyl 4-(5-(2-chloro-5-(trifluoromethyl)phenyl)-1,3,4-oxadiazol-2-yl)benzoate (10.5 mg, 0.027 mmol, 1.0 equiv) produced in (2) was dissolved in THF (0.3 mL) and water (0.3 mL), followed by the addition of lithium hydroxide monohydrate (5.7 mg, 0.13 mmol, 5.0 equiv). The mixture was stirred at room temperature for 3 hours, acidified by the addition of a 1M hydrochloric acid aqueous solution, ice-cooled, and filtered. The resulting solid was washed with water and dried to give 4-(5-(2-chloro-5-(trifluoromethyl)phenyl)-1,3,4-oxadiazol-2-yl)benzoic acid as a white solid (6.5 mg, 0.017 mmol, 38% yield).
¹H NMR (500 MHz, CD₃OD) δ 8.44 (1H, s), 8.26 (2H, d, J = 8.3 Hz), 8.23 (2H, d, J = 8.3 Hz), 7.93 (2H, m)

### Production Example 40

### 4-(3-(2-Chloro-5-(trifluoromethyl)phenyl)-1,2,4-oxadiazol-5-yl)benzoic acid (compound 33)

### (1) Production of 2-chloro-N'-hydroxy-5-(trifluoromethyl)benzimidamide

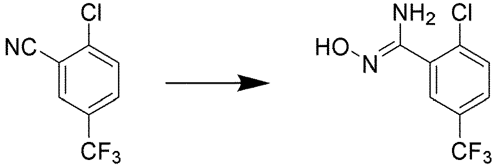

Hydroxylamine hydrochloride (260 mg, 3.75 mmol, 1.5 equiv) and sodium hydroxide (157.5 mg, 3.75 mmol, 1.5 equiv) were mixed in ethanol (2.1 mL) and water (2.1 mL), followed by stirring. 2-Chloro-5-(trifluoromethyl)benzonitrile (513.9 mg, 2.5 mmol, 1.0 equiv) was added, and the mixture was stirred at 90°C overnight and then allowed to cool to room temperature. The solvents were removed under vacuum, and ethanol/hexane = 9:1 (4.2 mL) was added to the residue, followed by stirring at room temperature for 30 minutes. The resulting reaction liquid was filtered, and the filtrate was concentrated to dryness to give 2-chloro-N'-hydroxy-5-(trifluoromethyl)benzimidamide (59.3 mg, 0.24 mmol, 10% yield).
¹H NMR (400 MHz, CDCl₃) δ 7.80 (1H, s), 7.61 (1H, d, J = 8.0 Hz), 7.56 (1H, d, J = 8.0 Hz)

### (2) Production of methyl 4-(3-(2-chloro-5-(trifluoromethyl)phenyl)-1,2,4-oxadiazol-5-yl)benzoate

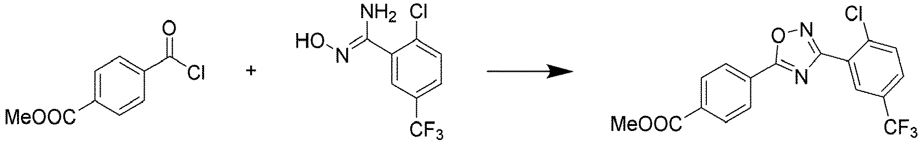

2-Chloro-N'-hydroxy-5-(trifluoromethyl)benzimidamide (59.3 mg, 0.24 mmol, 1.0 equiv) produced in (1) and triethylamine (41.4 µE, 0.29 mmol, 1.2 equiv) were dissolved in dichloromethane (1.6 mL), and the mixture was stirred at 0°C. Methyl 4-(chlorocarbonyl)benzoate (51.7 mg, 0.26 mmol, 1.05 equiv) was added, and the mixture was stirred at room temperature for 4 hours. The resulting reaction liquid was washed with water and a saturated aqueous sodium chloride solution, dried over sodium sulfate, and filtered. The filtrate was concentrated under reduced pressure. Toluene (1.6 mL) was added to the residue, and the mixture was stirred at 130°C overnight and allowed to cool to room temperature. Thereafter, the solvent was removed under vacuum, and the residue was purified by column chromatography (SiO₂, SNAP ultra (10 g), ethyl acetate/hexane = 5% → 30%) to give methyl 4-(3-(2-chloro-5-(trifluoromethyl)phenyl)-1,2,4-oxadiazol-5-yl)benzoate (27.0 mg, 0.070 mmol, 29% yield).
¹H NMR (400 MHz, CDCl₃) δ 8.35 (1H, s), 8.32 (2H, d, J = 8.5 Hz), 8.23 (2H, d, J = 8.5 Hz), 7.72 (2H, m), 3.98 (3H, s)

### (3) Production of 4-(3-(2-chloro-5-(trifluoromethyl)phenyl)-1,2,4-oxadiazol-5-yl)benzoic acid

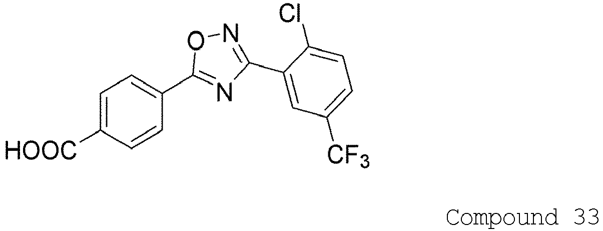

Methyl 4-(3-(2-chloro-5-(trifluoromethyl)phenyl)-1,2,4-oxadiazol-5-yl)benzoate (27.0 mg, 0.070 mmol, 1.0 equiv) produced in (2) was dissolved in THF (0.8 mL) and water (0.8 mL), followed by the addition of lithium hydroxide monohydrate (14.8 mg, 0.35 mmol, 5.0 equiv). The mixture was stirred at room temperature overnight and acidified by the addition of a 1M hydrochloric acid aqueous solution, followed by extraction with ethyl acetate, drying over sodium sulfate, and filtration. The filtrate was concentrated under reduced pressure to give 4-(3-(2-chloro-5-(trifluoromethyl)phenyl)-1,2,4-oxadiazol-5-yl)benzoic acid as a white solid (2.4 mg, 0.006 mmol, 9% yield).
¹H NMR (400 MHz, DMSO-d₆) δ 8.37 (1H, s), 8.32 (2H, m), 8.18 (2H, m), 8.05 (1H, d, J = 8.7 Hz), 8.00 (1H, d, J = 8.7 Hz)

### Production Example 41

### 4-(5-(4-Isopropylbenzamido)-1,3,4-thiadiazol-2-yl)benzoic acid (compound 34)

### (1) Production of methyl 4-(5-amino-1,3,4-thiadiazol-2-yl)benzoate

The title compound was produced as described below.

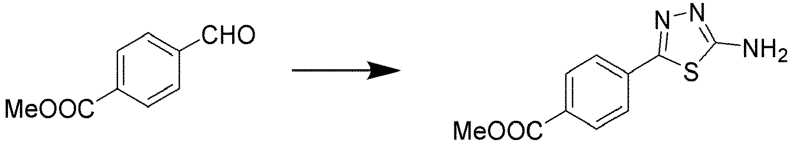

Thiosemicarbazide (136.7 mg, 1.5 mmol, 1.0 equiv) was added to warm water (1.5 mL), followed by stirring. A mixture of methyl 4-formylbenzoate and ethanol (8 mL) was slowly added, and the mixture was stirred at room temperature for 2 hours. A mixture of iron(III) chloride hexahydrate (810.9 mg, 3.0 mmol, 2.0 equiv) and water (1.5 mL) was added, and the mixture was stirred at 75°C for 30 minutes and allowed to cool to room temperature. Ethanol was removed under vacuum, followed by filtration. Pyridine (2.3 mL) was added to the resulting solid, and the mixture was stirred at room temperature for 30 minutes. Ice water was added to the reaction liquid, followed by filtration. The resulting residue was washed with warm ethanol, warm methanol, and ethyl acetate and dried to give methyl 4-(5-amino-1,3,4-thiadiazol-2-yl)benzoate (129.4 mg, 0.55 mmol, 33% yield).
¹H NMR (400 MHz, DMSO-d₆) δ 8.02 (2H, d, J = 8.2 Hz), 7.89 (2H, d, J = 8.2 Hz), 7.59 (2H, s), 3.86 (3H, s)

### (2) Production of 4-(5-(4-isopropylbenzamido)-1,3,4-thiadiazol-2-yl)benzoic acid

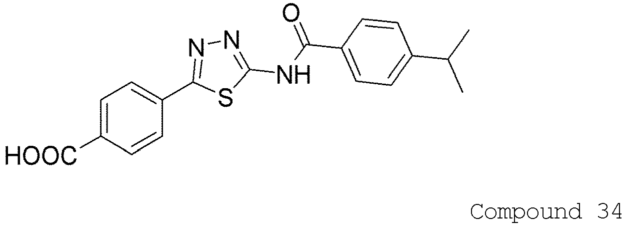

Methyl 4-(5-amino-1,3,4-thiadiazol-2-yl)benzoate (31.2 mg, 0.13 mmol, 1.0 equiv) produced in (1) and triethylamine (50.0 µE, 0.39 mmol, 3.0 equiv) were dissolved in THF (1.8 mL), followed by the addition of 4-isopropylbenzoyl chloride (43.6 mg, 0.26 mmol, 2.0 equiv). The mixture was stirred at room temperature overnight. Lithium hydroxide monohydrate (15.2 mg, 0.39 mmol, 3.0 equiv) and water (1.8 mL) were added to the reaction liquid, and the mixture was stirred at room temperature overnight. The solvents were removed under vacuum, and the residue was purified by column chromatography (SiO₂, SNAP ultra (10 g), methanol/ethyl acetate = 0% → 20%) to give 4-(5-(4-isopropylbenzamido)-1,3,4-thiadiazol-2-yl)benzoic acid as a light yellow solid (5.3 mg, 0.014 mmol, 11% yield).
¹H NMR (400 MHz, CD₃OD) δ 8.05 (2H, d, J = 8.5 Hz), 7.98 (2H, d, J = 8.0 Hz), 7.94 (2H, d, J = 8.5 Hz), 7.41 (2H, d, J = 8.0 Hz), 2.99 (1H, sep, J = 6.9 Hz), 1.26 (6H, d, J = 6.9 Hz); ESI-MS m/z 368.35 [M + H]+

### Experimental Examples

### I. Experimental materials and preparation methods threrefor

### (1) Animals

Dock8^{-/-} AND Tg mice (DOCK8-deficient mice expressing AND TCR) and Dock8^{-/-} OTIITg mice (DOCK8-deficient mice expressing OTII TCR) described in NPL 4 were used. B10.BR and C57BL/6J mice were purchased from Japan SLC (Japan) and Japan Clea (Japan), respectively. All of the mice were maintained under specific-pathogen-free conditions in the animal facility of Kyushu University. All animal experiments were conducted in accordance with relevant national and international guidelines contained in the Act on Welfare and Management of Animals (Ministry of the Environment, Japan) and Rules on Laboratory Animals (Kyushu University). Animal experiment protocols were pre-approved by the Ethics Committee of Animal Experiments, Kyushu University.

### (2) Cell line and transfectant

### (i) Creation of cell line used for primary screening (luciferase reporter assay)

Two plasmids were transfected into immortalized mouse embryonic fibroblasts (MEFs) using Lipofectamine 2000 (Thermo Fisher Scientific; hereinafter also referred to as "TFS"), a pTetOne system vector (Takara Bio Inc.) encoding mouse Epas1, and a pWhere system vector (Takara Bio Inc.) encoding luciferase under the control of mouse Il31 promoter (see Fig. 1). For selection, the puromycin and hygromycin markers (both produced by Takara Bio Inc.) were cotransfected into these vectors, respectively.A clone was isolated by limiting dilution after selection with hygromycin (600 µg/ml) and puromycin (3 µg/ml).

This was treated with doxycycline (100 ng/ml) for 24 hours and then cultured on a 96-well plate for 4 hours in the presence or absence of a test compound (10 µM). Luciferase activity was measured using the One-Glo Luciferase Assay System (produced by Promega).

### (ii) Human sarcoma cell line HT1080

Human sarcoma cell line HT1080 was provided by Y. Matsumoto (Kyushu University). For hypoxic induction, HT1080 (3 × 10⁵ cells) was cultured in 1% O₂ for 12 hours before assays.

### (3) Preparation and activation of mouse CD4⁺ T cells

Mouse CD4⁺ T cells were separated from the spleen and peripheral lymph nodes (PLNs) with a Dynabeads^{™} FlowComp^{™} Mouse CD4 Kit (TFS) and suspended in RPMI 1640 medium (FUJI FILM Wako Pure Chemical Corporation) containing 10% heat-inactivated fetal calf serum (TFS), 50 µM 2-mercaptoethanol (Nacalai Tesque, Inc.), 2 mM L-glutamine, 100 U/ml penicillin, 100 µg/ml streptomycin, 1 mM sodium pyruvate, and MEM non-essential amino acids (all from TFS). For primary stimulation, CD4⁺ T cells (3 × 10⁵ cells) from Dock8^{-/-} AND Tg mice were activated by culturing them in a 24-well plate with T cell-depleted irradiated spleen cells (5 × 10⁶ cells) in the presence of MCC88-103 (ANERADLIAYLKQATK (SEQ ID NO: 1), 3 µg/ml) for 24 hours. Similarly, CD4⁺ T cells (3 × 10⁵ cells) from Dock8^{-/-} OTII Tg mice were activated by stimulating them in a 24-well plate with OVA323-339 (ISQAVHAAHAEINEAGR (SEQ ID NO:2), 1 µg/ml) for 72 hours.

CD4⁺ T cells from Dock8^{-/-} OTII Tg mice are known to produce large amounts of IL-31 when stimulated with an antigen, as with CD4⁺ T cells from Dock8^{-/-} AND Tg mice (NPL 4) .

### (4) Preparation and activation of human CD4⁺ T cells

Human peripheral blood mononuclear cells (PBMCs) were separated by gradient centrifugation with Percoll (GE Healthcare), and CD4⁺ T cells were isolated from the PBMCs by magnetic sorting with Dynabeads human CD4 (TFS) followed by treatment with DETACHaBEAD human CD4 (TFS). Human peripheral blood samples were obtained from AD patients and healthy volunteers in compliance with Institutional Review Board protocols. AD was diagnosed according to the criteria provided by the Japanese Dermatological Association. The experiments were pre-approved by the Ethics Committee of Kyushu University Hospital, and written informed consent was obtained from all of the patients and the healthy volunteers.

To measure the amount of cytokine production, the isolated CD4⁺ T cells (1 × 10⁵ cells) were suspended in complete RPMI 1640 medium and then cultured in a 96-well plate with T cell-depleted irradiated PBMCs (1 × 10⁶ cells) in the presence of staphylococcal enterotoxin B (SEB: 100 ng/ml; Sigma-Aldrich) for 72 hours.

### (5) Real-time PCR

Total RNA was isolated using ISOGEN (Nippon Gene Co., Ltd.). For amplification by PCR, RNA samples treated with RNase-free DNase I (Thermo Fisher Scientific) were reverse-transcribed with oligo(dT) primers (TFS) and SuperScript III reverse transcriptase (TFS). Real-time PCR was performed in a CFX Connect^{™} thermal cycler (BIO-RAD) using SYBR Green PCR Master Mix (Thermo Fisher Scientific). The expression of mouse and human target genes was normalized to expression of Hprt and PPIB genes, respectively. CFX Manager software (ver.3.1) supplied with the instrument was used for analysis.

The following primers were used for real-time PCR.

For IL-31 gene amplification
   (1) 5'-AAACAAGAGTCTCAGGATCTTTATAACAAC-3' (SEQ ID NO: 3)
   (2) 5'-ACGGCAGCTGTATT GATTCGT-3' (SEQ ID NO: 4)
For IL-2 gene amplification
   (1) 5'-CCTGAGCAGGATGGAGAATTACA-3' (SEQ ID NO: 5)
   (2) 5'-TCCAGA ACATGCCGCAGAG-3' (SEQ ID NO: 6)
For IL-4 gene amplification
   (1) 5'-CGAAGAACACCACAGAGAGTGAGCT-3' (SEQ ID NO: 7)
   (2) 5'-GACTCATTCATGGTGCAGCTTATCG-3' (SEQ ID NO: 8)
For Hprt gene amplification
   (1) 5'-CTGGTGAAAAGGA CCTCTCG-3' (SEQ ID NO: 9)
   (2) 5'-TGAAGTACTCATTATAGTCAAGGGCA-3' (SEQ ID NO: 10)
For VEGFA gene amplification
   (1) 5'-CATCACCATGCAGATTATGCGG-3' (SEQ ID NO: 11)
   (2) 5'-CCCACAGGGACGGGATTTC-3' (SEQ ID NO: 12)
For GLUT1 gene amplification
   (1) 5'-CTTTTCTGTTGGGGGCATGAT-3' (SEQ ID NO: 13)
   (2) 5'-CCGCAGTACACA CCGATGAT-3' (SEQ ID NO: 14)
For PPIB (cyclophilin B) gene amplification
   (1) 5'-ATGTGGTTTTCGGCAAAG TTCTA-3' (SEQ ID NO: 15)
   (2) 5'-GGCTTGTCCCGGCTGTCT-3' (SEQ ID NO: 16)

### II. Experimental methods

### (1) Primary screening of chemical library (luciferase reporter assay)

A mouse IL-31 gene reporter plasmid (pGL4.10 Il31) into which the promoter region of the mouse IL-31 gene from position -1,367 to -1 is introduced is described in NPL 4.

The promoter region of the human IL-31 gene from position -1,308 to -1 was amplified by PCR and subcloned into a pGL4.10 [luc2] vector (Promega) to generate a human IL-31 gene promoter reporter plasmid (human pGL4.10 IL-31). Deletions and/or mutations in the mouse and human IL-31 gene promoter regions were created by PCR.

Expression vectors encoding FLAG-tagged mouse and human EPAS1 (pcDNA-EPAS1) were developed using pcDNA vectors.

For the luciferase reporter assay, immortalized MEFs (NPL 4) were co-transfected with a pRL-SV40-Renilla luciferase plasmid (0.1 µg; Promega), and pGL4.10 Il31 or human pGL4.10 IL-31 (2 ug) in the presence of a pcDNA vector encoding mouse or human EPAS1 (2 µg). Briefly, these plasmid DNAs were mixed with a Lipofectamine 2000 transfection reagent (5 ul: TFS) in Opti-MEM medium (500 ul: TFS) at room temperature for 20 minutes, and the mixture was added dropwise to MEFs (3 × 10⁵ cells) cultured in 1.5 ml of DMEM medium containing 1% FCS. Six hours after transfection, the cells were suspended in 10% FCS-containing DMEM containing a test compound (2.5 µM) or a vehicle alone (0.1% DMSO) for another 24 hours. Luciferase activity was measured with a Dual-Luciferase Reporter Assay System (Promega) according to the manufacturer's protocol.

Approximately 10,000 test compounds were subjected to this screening to select test compounds that showed 50% or more inhibition relative to the luciferase activity in the absence of the test compounds (control) (100%).

### (2) Secondary screening

CD4⁺ T cells (3 × 10⁵ cells) from Dock8^{-/-} AND Tg mice were cultured with T cell-depleted irradiated spleen cells (5 × 10⁶ cells) containing a test compound (2.5 µM) or a vehicle alone (0.1% DMSO), in the presence of MCC88-103 (3 µg/ml) for 24 hours.

The expression level of the IL-31 gene was measured by real-time PCR described above, and test compounds that showed 60% or more inhibition relative to the expression level in the absence of the test compounds (control) (100%) were selected.

### (3) ELISA and AlphaLISA

To measure the amounts of cytokines (IL-31, IL-2) in a mouse CD4⁺ T cell culture supernatant, CD4⁺ T cells (1 × 10⁵ cells) from Dock8^{-/-} AND Tg mice were stimulated with MCC88-103 (1 µg/ml) in the presence of a test compound (compound 1 (20 µM) or negative control compound 1 (20 µM)) for 60 hours. The mouse cytokine concentration in the culture supernatant was measured with an ELISA kit (Cloud-Clone Corp., Katy, TX).

Similarly, to measure the amounts of cytokines (IL-31, IL-2) in a human CD4⁺ T cell culture supernatant, human CD4⁺ T cells (1 × 10⁵ cells) isolated as described above were suspended in complete RPMI 1640 medium and then cultured with T cell-depleted irradiated PBMCs (1 × 10⁶ cells) in the presence of SEB (100 ng/ml; Sigma-Aldrich) in a 96-well plate for 72 hours. The human cytokine concentration in the culture supernatant was measured using an AlphaLISA Detection Kit (PerkinElmer) according to the manufacturer's instructions.

### (4) T cell proliferation assay

A T cell proliferation assay was performed by culturing CD4⁺ T cells (5 × 10⁴ cells) with T cell-depleted irradiated spleen cells (1 × 10⁶ cells) in the presence of phorbol 12-myristate 13-acetate (PMA: 100 ng/ml; Sigma-Aldrich) and ionomycin (1 µg/ml; Sigma-Aldrich) for 66 hours. For the last 18 hours of culture, ³H-thymidine (0.037 MBq) was added, and the incorporated radioactivity was measured with MicroBeta² (PerkinElmer, Inc., Waltham, MS).

### (5) Measurement of scratch behavior

Mice were put into an acrylic cage (11 × 14 × 20 cm) for at least 1 hour for acclimation, and their behaviors were videotaped. Playback of the video was used for determination of the total number of scratching bouts per 2 hours. When mice scratch, they stretch their hind paw toward the itchy spot, lean their head toward the hind paw, rapidly move the paw several times, and then lower it back to the floor. A series of these movements was counted as one bout of scratching.

### (6) Chromatin immunoprecipitation assay (ChIP assay)

A ChIP assay was performed using activated mouse CD4⁺ T cells and immortalized MEFs. Briefly, CD4⁺ T cells from Dock8^{-/-} AND Tg mice were stimulated with MCC88-103 in the presence of a test compound (2.5 µM) or a vehicle alone (0.1% DMSO) for 24 hours. Immortalized MEFs were co-transfected with pcDNA-human EPAS1 (2 ug) in the presence of pGL4.10 IL31 (2 µg). Six hours after transfection, the MEFs were suspended in 10% FCS-containing DMEM that contains a test compound (10 µM) or a vehicle alone (0.1% DMSO) for another 24 hours.

The activated mouse CD4⁺ cells and the transfected MEFs were treated with 0.5% formaldehyde at room temperature for 5 minutes and then neutralized with glycine at room temperature for 5 minutes. The resulting cells were washed twice with PBS containing a protease inhibitor, and then nuclei were isolated using a Magna ChIP^{™} HiSens kit (Millipore, Burlington, MS) according to the manufacturer's protocol. The isolated nuclei were resuspended in sonication buffer and sonicated using an ultrasonic processor (VCX-130; Sonics&Materials Inc., Newtown, CN) at 30% amplitude setting for 90 × 10 seconds on ice for shearing chromatin DNA. After centrifugation at 10,000 g at 4°C for 10 minutes, the sheared chromatin DNA was obtained and treated with RNase and proteinase K for quantifying DNA content. For immunoprecipitation, 5 to 10 µg of chromatin DNA was mixed with magnetic Protein A/G beads preincubated with a rabbit polyclonal anti-EPAS1 antibody (ab199; Abcam, Cambridge, UK) or a rabbit polyclonal anti-FLAG (registered trademark) M2 antibody (#14793; CST, Danvers, MS) and incubated at 4°C overnight.

Magnetic beads preincubated with rabbit polyclonal IgG (ab171870; Abcam) were used for control studies. The magnetic beads were washed three times with buffer containing physiologic salt and once with low salt buffer. Subsequently, the magnetic beads were treated with proteinase K at 65°C for 2 hours and heated at 95°C for 15 minutes for inactivation of proteinase K, and elution of bound chromatin DNA was performed. Eluted DNA and input chromatin DNA were analyzed with a CFX Connect^{™} thermal cycler (BIO-RAD) using SYBR Green PCR Master Mix (Thermo Fisher Scientific) and the following primers. Data were normalized for the amount of the input chromatin DNA.

For mouse IL-31 gene (-1336/-1042) amplification
   (1) 5'-TCAGTGATCAATTAGCCC AGCC-3' (SEQ ID NO: 17)
   (2) 5'-ATCTGACACTCTCGAGCCTCT-3' (SEQ ID NO: 18)
For human IL-31 gene (-280/-4) amplification
   (1) 5'-TGCATTCATGTGCCTTCTTGTG-3' (SEQ ID NO: 19)
   (2) 5'-AGAGAGAGCAAGGCCA GCTTC-3' (SEQ ID NO: 20)

### Experimental Example 1

### Selection of compound by screening and structure-activity relationship of selected compound

By the primary screening and secondary screening described above, 4-(2-(4-isopropylbenzylidene)hydrazinyl)benzoic acid (compound 1) (Production Example 1) was selected as a compound that significantly inhibited antigen-induced Il-31 gene expression when CD4⁺ T cells from Dock8^{-/-}AND Tg mice were treated with 2.5 µM of the compound, from a group of approximately 10,000 compounds.

In contrast, when CD4⁺ T cells from Dock8^{-/-} AND Tg mice were treated, in the same manner as compound 1, with a compound having a p-(2-dimethylamino)ethoxy substituent in place of the p-isopropyl substituent on the right-hand-side aromatic ring of compound 1 as a hydrazine compound structurally similar to compound 1 (negative control compound 1), IL-31 gene expression was not inhibited (see Fig. 2A). On the other hand, neither of these compounds had an effect on the expression of other cytokine (IL-2 and IL-4) genes in the CD4⁺ T cells (see Fig. 2B).

When the CD4⁺ T cells were treated with compound 1 or negative control compound 1 (20 µM) in the presence of MCC88-103, and the amounts of IL-31 and IL-2 produced were measured by ELISA, the same results as above were obtained (Fig. 3). However, it was confirmed that neither of these compounds had an adverse effect on non-specific T cell proliferation induced by PMA and ionomycin (see Fig. 4).

These results suggest that compound 1 is a compound that selectively inhibits the induction of IL-31 gene expression via a TCR (T-cell receptor) in CD4⁺ T cells of Dock8^{-/-} AND Tg mice and specifically inhibits IL-31 production, and that the p-isopropyl substituent attached to the right-hand-side aromatic ring of compound 1 is largely responsible for its action. Further, compound 1 did not affect non-specific T-cell proliferation response in vitro even when used at 50 µM. In addition, compound 1 showed no histological or biochemical abnormalities in a single-dose toxicity study of 100-mg oral administration, and the compound 1 treatment group did not differ from the non-treated group in body weight gain. From these, it is determined that compound 1 is a highly safe compound with low cytotoxicity.

### Experimental Example 2

### Confirmation of in vivo action of compound 1

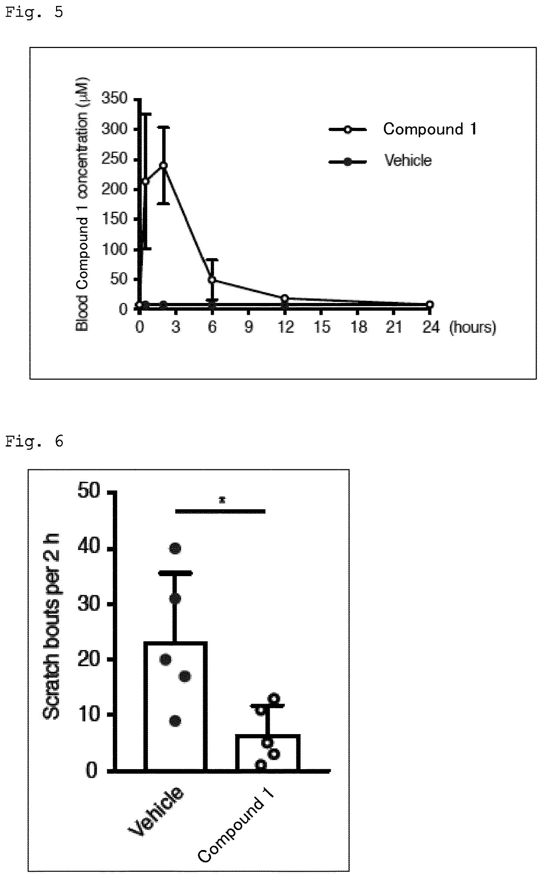

After compound 1 (100 mg) was orally administered to C57BL/6 mice (n = 3), its blood concentration was monitored for 24 hours. It was found that compound 1 was eluted into the blood within 12 hours after oral administration (see Fig. 5).

### (1) Itch inhibitory action

To investigate whether compound 1 has an itch inhibitory action, mice expressing ovalbumin (OVA) under the control of the cytomegalovirus immediate early enhancer-chicken β-actin hybrid promoter (hereafter referred to as "CAG-OVA mice") were used. Activated CD4⁺ T cells (8.0 × 10⁶ per mouse) from Dock8^{-/-} OTII Tg mice were stimulated with OVA323-339 (ISQAVHAAHAEINEAGR (SEQ ID NO: 2), 1 µg/ml) for 72 hours and intravenously injected into CAG-OVA mice, and the scratch behavior of the mice was measured 1 hour later. As a result, when the activated CD4⁺ T cells were injected into CAG-OVA mice, scratch behavior was induced (see the results of the vehicle in Fig. 6). However, when compound 1 was orally administered to CAG-OVA mice, the scratch behavior was significantly suppressed (see the results of compound 1 in Fig. 6).

From the results of Experimental Examples 1 and 2, it is believed that orally administered compound 1 functions in vivo to inhibit IL-31 production and suppress itch.

### Experimental Example 3

### Mechanism of suppression of expression of IL-31 gene by compound 1

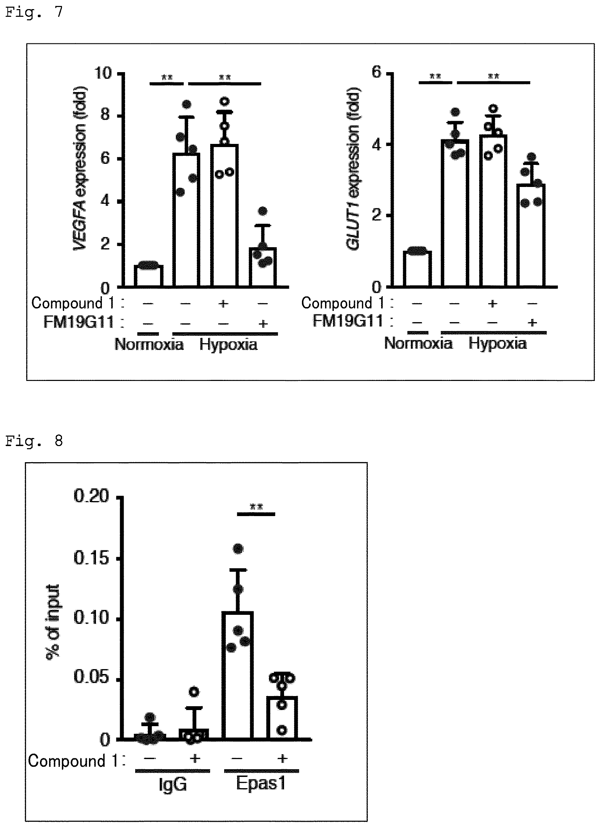

(1) Although EPAS1 is known to control hypoxic responses through complex formation with aryl hydrocarbon receptor nuclear translocator (ARNT) (NPL 6), EPAS1-mediated Il31 promoter activation is independent of ARNT (NPL 4). FM19G11 is not a direct inhibitor of EPAS1, but is known to suppress the expression of the EPAS1 gene (Epas1) by acting on an unidentified target (NPL 7). Treatment of human cancer cell line HT1080 with FM19G11 suppressed VEGFA expression and GLUT1 expression induced by hypoxia (Fig. 7). In contrast, compound 1 did not show such an effect of suppressing expression (Fig. 7). This suggests that ARNT-dependent EPAS1 function is not affected by compound 1.
(2) To understand the mechanism by which compound 1 controls IL-31 production in CD4⁺ T cells, a chromatin immunoprecipitation (ChIP) assay was performed using CD4⁺ T cells of Dock8^{-/-} AND Tg mice. The results showed that antigen stimulation recruited EPAS1 to the IL-31 gene promoter region (-1336 to -1042), but the recruitment was greatly reduced in the presence of compound 1 (Fig. 8). The results suggest that compound 1 suppresses TCR-mediated induction of IL-31 production by inhibiting recruitment of EPAS1 to the IL-31 gene promoter region.

### Experimental Example 4

### IL-31 production induction inhibitory action in humans

As shown in Experimental Example 1, compound 1 was found to selectively inhibit the induction of IL-31 gene expression in mouse CD4⁺ T cells; thus, in this study, whether compound 1 acts similarly on human CD4⁺ T cells was investigated.

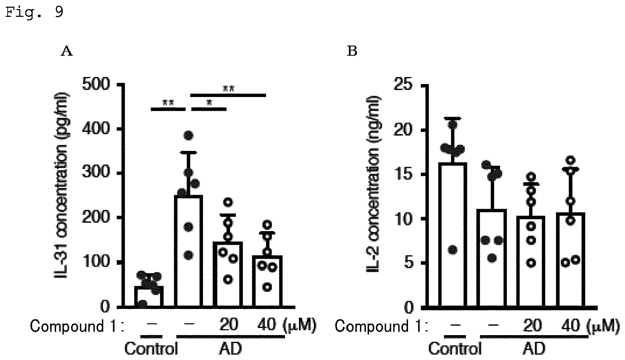

When stimulated with staphylococcal enterotoxin B (SEB), CD4⁺ T cells of AD patients produced larger amounts of IL-31 than CD4⁺ T cells of healthy subjects (control example). In contrast, treatment of CD4⁺ T cells of AD patients with compound 1 reduced IL-31 production in a dose-dependent manner (Fig. 9A). On the other hand, IL-2 production was not affected (Fig. 9B).

To understand the effect of compound 1 on IL-31 gene promoter activation, a reporter construct containing the human IL-31 gene promoter sequence (-1,308 to -1) was made. When the reporter construct was expressed in MEFs, activation of the IL-31 gene promoter was induced in the presence of human EPAS1, but was significantly suppressed by compound 1. From this, it is believed that compound 1 also suppresses the induction of IL-31 production in human CD4⁺ T cells by inhibiting the recruitment of EPAS1 to the IL-31 gene promoter region.

The above experiments confirmed that compound 1 is useful as a small molecule inhibitor that targets the induction of IL-31 production by EPAS1 in mouse and human CD4⁺ T cells. Small molecule inhibitors of Janus kinase (JAK) and phosphodiesterase 4 (PDE4) are in development as therapeutic agents for AD by oral administration (NPL 8). Although these inhibitors are effective in the treatment of AD, they all act on multiple cytokine pathways, and their effects are nonspecific. In contrast to these, compound 1 selectively inhibits the induction of IL-31 production without affecting the production of cytokines other than IL-31 or hypoxic responses. Compound 1 is therefore useful as an active ingredient in therapeutic agents for AD with a low risk of adverse effects, in particular, in therapeutic agents for AD with an excellent itch-reducing effect.

### Experimental Example 5

### Evaluation of IL-31 production inhibitory action and toxicity of compounds structurally similar to compound 1

The IL-31 production inhibitory action and cell proliferation action of compounds 1 to 18 and negative control compounds 1 to 7, which were produced in the methods described in Production Examples 1 to 25, and compounds 19 to 34, which were produced in the methods described in Production Examples 26 to 41, were measured by the following methods.

### (1) Measurement of IL-31 production inhibitory action

CD4⁺ T cells (3 × 10⁵ cells) from Dock8^{-/-} AND Tg mice were cultured with T cell-depleted irradiated spleen cells (5 × 10⁶ cells) containing a test compound (various concentrations) or a vehicle alone (0.1% DMSO) in the presence of MCC88-103 (3 µg/ml) for 24 hours. The expression level of the mouse IL-31 gene was measured by real-time PCR described above, and the inhibition rate relative to the expression level of the IL-31 gene in the absence of the test compound (control) (100%) was calculated.

### (2) Measurement of cell proliferation action

A T cell proliferation assay was performed by culturing CD4⁺ T cells (5 × 10⁴ cells) with T cell-depleted irradiated spleen cells (1 × 10⁶ cells) in the presence of phorbol 12-myristate 13-acetate (PMA: 100 ng/ml; Sigma-Aldrich) and ionomycin (1 µg/ml; Sigma-Aldrich) for 66 hours. Test compounds were added at various final concentrations at the start of culture. For the last 18 hours of culture, ³H-thymidine (0.037 MBq) was added, and the incorporated radioactivity was measured with MicroBeta² (PerkinElmer, Inc., Waltham, MS). Specifically, the inhibitory activities of the test compounds were calculated based on the IL-31 gene expression and the CD4⁺ T cell proliferation response in CD4⁺ T cells from Dock8^{-/-} AND Tg mice when treated with a solvent alone (vehicle alone; 0.1% DMSO) taken as 100%.

Table 1 shows the results of compounds 1 to 18 and negative control compounds 1 to 7.

**Table 1**

| | | IL-31 production inhibitory action | | Cell proliferation action | | |
|---|---|---|---|---|---|---|
| | | Inhibition rate (%) based on the case using only solvent taken as 100% | | Inhibition rate (%) based on the case using only solvent taken as 100% | | |
| NO. | KLogP of phenyl group with substituent B | Concentration of test compound | | Concentration of test compound | | |
| | | 2.5 µM | 5 µM | 5 µM | 20 µM | 50 µM |
| Compound 1 | 3.569 | 20.9 | 19.6 | 137.8 | 124.7 | 80.9 |
| Compound 2 | 3.968 | 11.5 | 35.2 | ND | 115.6 | ND |
| Compound 3 | 2.307 | 16.8 | 21.9 | 148.7 | 176.3 | 124.1 |
| Compound 4 | 1.5666 | 62.6 | 48.8 | 99.6 | 134.2 | 114.4 |
| Compound 5 | 1.93 | 67.4 | 31.9 | ND | 135.2 | 96.6 |
| Compound 6 | 2.436 | 43.4 | 16.5 | ND | 158.7 | 142.0 |
| Compound 7 | 3.395 | 13.4 | 3.1 | 105.2 | 67.0 | 42.0 |
| Compound 8 | 2.784 | 15.3 | 30.1 | ND | 88.6 | ND |
| Compound 9 | 2.647 | 26.4 | 39.0 | ND | 111.4 | 46.2 |
| Compound 10 | 3.738 | 8.4 | 16.2 | 146.6 | 143.3 | 18.0 |
| Compound 11 | 3.168 | 11.1 | 18.9 | ND | 89.0 | ND |
| Compound 12 | 2.412 | 40.0 | 38.2 | ND | 94.3 | ND |
| Compound 13 | 1.885 | 43.9 | 46.3 | ND | 109.6 | ND |
| Compound 14 | 3.968 | 15.1 | 19.6 | 90.9 | ND | ND |
| Compound 15 | 3.738 | 25.3 | 24.9 | ND | 117.0 | 34.2 |
| Compound 16 | 3.968 | 17.9 | 11.3 | 89.4 | 61.7 | 64.0 |
| Compound 17 | 3.569 | 39.8 | 53.5 | ND | 82.5 | 61.7 |
| Compound 18 | 3.569 | 11.6 | 27.1 | ND | 83.7 | 31.6 |
| Negative control compound 1 | 2.1598 | 111.5 | 109.1 | 98.1 | 99.9 | 88.9 |
| Negative control compound 2 | 1.64 | 53.8 | 57.2 | ND | ND | ND |
| Negative control compound 3 | 1.2934 | 88.3 | 61.1 | 99.6 | ND | ND |
| Negative control compound 4 | 3.569 | 124 | 126.6 | ND | ND | ND |
| Negative control compound 5 | 3.569 | 120.5 | 124.5 | ND | ND | ND |
| Negative control compound 6 | 3.569 | 91.5 | 98.4 | ND | ND | ND |
| Negative control compound 7 | 3.354 | 157.4 | 115.3 | ND | ND | ND |

| | | | | | | |
|---|---|---|---|---|---|---|
| "ND" means not tested. | | | | | | |

The results of negative control compounds 4 to 6 confirmed that the IL-31 production inhibitory action of compound 1 is completely lost when the left-hand-side aromatic ring of the two aromatic rings of compound 1 has no substituents (negative control compound 6) or when the position of the carboxy group located is a position other than the para position (negative control compounds 4 and 5). From the results, the carboxy group located at the para position in the left-hand-side aromatic ring is considered to be essential for exhibiting the IL-31 production inhibitory action.

The results of compounds 14 to 16 and 18 confirmed that regarding the connection site between the two aromatic rings of compound 1, the -NH-N=CH- group (hydrazone moiety) of compound 1 can be replaced by a -NH-N=C(CH₃)- group (compound 18) or a divalent N-containing 5-membered heterocyclic group, such as an oxazole (compounds 14 and 15) or a triazole (compound 16). This indicates that the bond length in the connection site is important for maintaining the IL-31 production inhibitory action and that the rigidity is a minor factor.

As shown in negative control compound 7, when the right-hand-side aromatic ring of the two aromatic rings of compound 1 is replaced by a cyclohexane ring, the IL-31 production inhibitory action was completely lost. The results revealed that the right-hand-side aromatic ring is important for exhibiting the IL-31 production inhibitory action. However, it was confirmed that the IL-31 production inhibitory action is maintained even when the isopropyl group located at the para position in the aromatic ring is replaced by a tert-butyl group (compound 2) or a dimethylamino group (compound 3). The results of compounds 4 to 7 confirmed that the methyl groups in the dimethylamino group may be alkyl groups having about 1 to 6 carbon atoms (i.e., di-C₁₋₆ alkylamino group), that each alkyl group may be bonded to another carbon atom of the aromatic ring to which the di-C₁₋₆ alkylamino group is attached, to form a fused ring (compound 7), and that the alkyl groups may have up to one hydroxyl group, although the IL-31 production inhibitory action tends to be slightly reduced (compounds 4 to 6). Further, it was confirmed that at least one of the five hydrogen atoms of the aromatic ring may be replaced by a group of the following groups other than the above substituents: a halogen atom (e.g., a chlorine atom or a fluorine atom), a halo C₁₋₆ alkyl group (e.g., a fluorocarbon), a C₁₋₆ alkyl group (e.g., a methyl group), a C₂₋₆ alkynylene group (e.g., an ethynylene group), and a carboxy group; the substituents with which the hydrogen atoms of the aromatic ring may be replaced may be the same or different (compounds 8 to 13 and 15). However, in all of the cases in which the aromatic ring had a di-C₁₋₆ alkylamino group having two or more hydroxyl groups (negative control compound 3), a (2-dimethylamino)ethoxy group at the para position (negative control compound 1), or a hydroxyl group (negative control compound 2) in place of the above substituents, the IL-31 production inhibitory action tended to notably decrease.

These results suggest that it is important for exhibiting the IL-31 production inhibitory effect that the right-hand-side aromatic ring moiety, as a whole, including the substituent(s), is hydrophobic.

Table 2 shows the results of compounds 19 to 34.

**Table 2**

| | | | qPCR results (IL-31 production (%)): based on the case using 0.1% DMSO taken as 100% | | | | | | Cell proliferation (%): based on the case using 0.2% DMSO taken as 100% | | | |
|---|---|---|---|---|---|---|---|---|---|---|---|---|
| NO. | | KLogP of phenyl group with substituent B | 0.078 µM | 0.156 µM | 0.312 µM | 0.625 µM | 1.25 µM | 2.5 µM | 5 µM | 10 µM | 20 µM | 40 µM |
| Compound 1 | IPHBA | 3.569 | 57.5 | 40.1 | 38.7 | 23.9 | 16.4 | ND | ND | ND | ND | ND |
| Compound 19 | KS-276 | 2.307 | ND | ND | 67.6 | 69.9 | 54.5 | 16.9 | 86.3 | 79.4 | 66.0 | 67.4 |
| Compound 20 | KS-290 | 3.738 | 16.9 | 17.7 | 23.3 | 24.7 | 22.1 | 32.6 | ND | 91.2 | 87.5 | 86.7 |
| Compound 21 | KS-293 | 3.738 | 23.7 | 29.9 | 25.3 | 23.0 | 38.2 | 13.1 | ND | 95.5 | 98.2 | 106.5 |
| Compound 22 | KS-277 | 3.569 | ND | 60.4 | 47.5 | 64.9 | 30.6 | 17.4 | 102.7 | 56.7 | 6.1 | ND |
| Compound 23 | KS-278 | 5.514 | ND | 32.5 | 46.4 | 31.4 | 22.7 | ND | 110.1 | 92.9 | 88.5 | 68.9 |
| Compound 24 | KS-281 | 4.943 | ND | 31.2 | 18.1 | 32.0 | 31.5 | ND | 88.1 | 81.5 | 71.3 | 62.9 |
| Compound 25 | KS-292 | 3.738 | ND | ND | 82.7 | 85.6 | 72.2 | 81.9 | ND | 82.9 | 81.3 | 80.5 |
| Compound 26 | KS-284 | 3.569 | ND | ND | ND | 45.1 | 26.2 | ND | ND | 77.1 | 70.6 | 64.6 |
| Compound 27 | KS-288 | 3.738 | 28.6 | 35.1 | 39.6 | 43.1 | 46.8 | ND | 86.2 | 78.0 | 70.0 | ND |
| Compound 28 | KS-285 | 3.569 | ND | 73.2 | 70.6 | 42.6 | 28.0 | ND | ND | 99.9 | 89.5 | 69.1 |
| Compound 29 | KS-287 | 3.738 | ND | 59.1 | 52.3 | 61.9 | 40.7 | 63.6 | ND | 88.6 | 77.7 | 86.0 |
| Compound 30 | KS-294 | 3.738 | 41.5 | 39.9 | 29.4 | 23.4 | 26.1 | 30.9 | ND | 98.3 | 90.3 | 76.4 |
| Compound 31 | KS-282 | 3.569 | ND | ND | ND | 53.6 | 24.0 | ND | ND | 97.5 | 79.3 | 65.6 |
| Compound 32 | KS-289 | 3.738 | ND | 35.5 | 52.1 | 39.9 | 53.5 | ND | ND | 115.3 | 92.7 | 91.8 |
| Compound 33 | KS-291 | 3.738 | 57.7 | 48.9 | 37.9 | 25.1 | 28.0 | 29.7 | ND | 73.5 | 66.7 | 64.3 |
| Compound 34 | KS-283 | 3.569 | ND | ND | ND | 48.3 | 52.3 | ND | ND | 79.0 | 90.8 | 87.8 |

| | | | | | | | | | | | | |
|---|---|---|---|---|---|---|---|---|---|---|---|---|
| "ND" means not detected. | | | | | | | | | | | | |

### Sequence Listing Free Text

SEQ ID NO: 1 shows the amino acid sequence of MCC88-103; SEQ ID NO: 2 shows the amino acid sequence of OVA323-339; SEQ ID NOs: 3 and 4 show the base sequences of the forward primer and reverse primer for IL-31 gene amplification, respectively; SEQ ID NOs: 5 and 6 show the base sequences of the forward primer and reverse primer for IL-2 gene amplification, respectively; SEQ ID NOs: 7 and 8 show the base sequences of the forward primer and reverse primer for IL-4 gene amplification, respectively; SEQ ID NOs: 9 and 10 show the base sequences of the forward primer and reverse primer for Hert gene amplification, respectively; SEQ ID NOs: 11 and 12 show the base sequences of the forward primer and reverse primer for VEGFA gene amplification, respectively; SEQ ID NOs: 13 and 14 show the base sequences of the forward primer and reverse primer for GLUT1 gene amplification, respectively; SEQ ID NOs: 15 and 16 show the base sequences of the forward primer and reverse primer for PPIB (cyclophilin B) gene amplification, respectively; SEQ ID NOs: 17 and 18 show the base sequences of the forward primer and reverse primer for mouse IL-31 gene (-1336/-1042) amplification, respectively; and SEQ ID NOs: 19 and 20 show the base sequences of the forward primer and reverse primer for human IL-31 gene (-280/-4) amplification, respectively.

## Claims

1. An interleukin-31 production inhibitor comprising a compound represented by the following formula (1) or a salt thereof as an active ingredient: 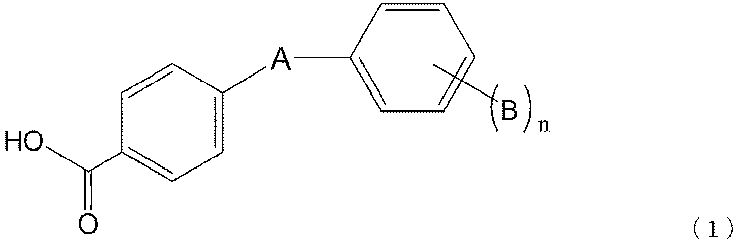 wherein
A is a -NH-N=C(R₁)- group, a divalent N-containing 5-membered heterocyclic group, a -NH-CO- group, or a group in which a divalent N-containing 5-membered heterocyclic group is linked to a -NH-CO- group, wherein R₁ is a hydrogen atom or a C₁₋₄ alkyl group,
B is the same or different and is a C₁₋₆ alkyl group optionally having one hydroxyl group, a mono or di(C₁₋₆ alkyl)amino group optionally having one hydroxyl group, a halogen atom, a halo C₁₋₆ alkyl group, a C₂₋₆ alkynylene group, or a carboxy group, wherein at least one C₁₋₆ alkyl group in the mono or di(C₁₋₆ alkyl)amino group is optionally attached to a carbon atom adjacent to a carbon atom in the phenyl group to which it is attached, to form a 5- to 6-membered ring, and
n is an integer of 1 to 5.

2. The interleukin-31 production inhibitor according to claim 1, wherein in the compound (1), the phenyl group with a substituent represented by B in the formula (1) has an octanol-water partition coefficient (KLogP) of 1.5 or more.

3. The interleukin-31 production inhibitor according to claim 1 or 2, wherein the compound (1) is a compound represented by the formula (1) in which
A is a -NH-N=C(R₁)- group, wherein R₁ is a hydrogen atom,
B is the same or different and is a C₁₋₆ alkyl group, a di(C₁₋₆ alkyl)amino group optionally having one hydroxyl group, a halogen atom, or a halo C₁₋₆ alkyl group, wherein at least one C₁₋₆ alkyl group in the di(C₁₋₆ alkyl)amino group is optionally attached to a carbon atom adjacent to a carbon atom in the phenyl group to which it is attached, to form a 6-membered ring, and
n is an integer of 1 to 5.

4. The interleukin-31 production inhibitor according to claim 1 or 2, wherein the compound (1) is a compound represented by the formula (1) in which
A is a divalent N-containing 5-membered heterocyclic group,
B is the same or different and is a C₁₋₆ alkyl group, a di (C₁₋₆ alkyl) amino group, a halogen atom, or a halo C₁₋₆ alkyl group, and
n is an integer of 1 to 5.

5. A pharmaceutical composition comprising the interleukin-31 production inhibitor of any one of claims 1 to 4 and a pharmaceutically acceptable carrier or excipient.

6. The pharmaceutical composition according to claim 5, which is in an oral administration form.

7. The pharmaceutical composition according to claim 5 or 6, which is an anti-pruritic agent.

8. The pharmaceutical composition according to claim 7, which is a therapeutic agent for an IL-31-mediated pruritic disease.

9. A compound represented by the following formula (1) or a salt thereof: 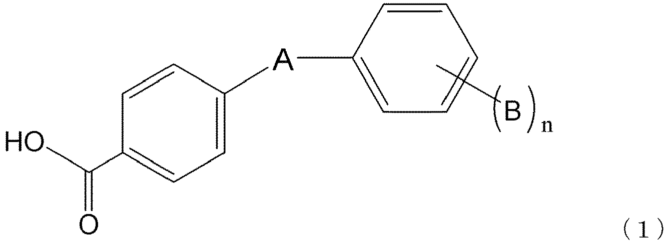 wherein
A is a -NH-N=C(R₁)- group, a divalent N-containing 5-membered heterocyclic group, a -NH-CO- group, or a group in which a divalent N-containing 5-membered heterocyclic group is linked to a -NH-CO- group, wherein R₁ is a hydrogen atom or a C₁₋₄ alkyl group,
B is the same or different and is a C₁₋₆ alkyl group optionally having one hydroxyl group, a mono or di(C₁₋₆ alkyl)amino group optionally having one hydroxyl group, a halogen atom, a halo C₁₋₆ alkyl group, a C₂₋₆ alkynylene group, or a carboxy group, wherein at least one C₁₋₆ alkyl group in the mono or di(C₁₋₆ alkyl)amino group is optionally attached to a carbon atom adjacent to a carbon atom in the phenyl group to which it is attached, to form a 5- to 6-membered ring, and
n is an integer of 1 to 5.

10. The compound or a salt thereof according to claim 9, wherein in the formula (1),
A is a -NH-N=C(R₁)- group, wherein R₁ is a hydrogen atom,
B is the same or different and is a C₁₋₆ alkyl group, a di(C₁₋₆ alkyl)amino group optionally having one hydroxyl group, a halogen atom, or a halo C₁₋₆ alkyl group, wherein at least one C₁₋₆ alkyl group in the di(C₁₋₆ alkyl)amino group is optionally attached to a carbon atom adjacent to a carbon atom in the phenyl group to which it is attached, to form a 6-membered ring, and
n is an integer of 1 to 5.

11. The compound or a salt thereof according to claim 9, wherein in the formula (1),
A is a divalent N-containing 5-membered heterocyclic group,
B is the same or different and is a C₁₋₆ alkyl group, a di (C₁₋₆ alkyl) amino group, a halogen atom, or a halo C₁₋₆ alkyl group, and
n is an integer of 1 to 5.

12. A screening system for an IL-31 production inhibitor showing suppression of expression of a reporter protein, wherein a system of expression of the reporter protein induced by an IL-31 gene promoter is introduced in a mouse MEF cell or human cell line in which expression of a transcription factor EPAS1 (endothelial PAS domain-containing protein 1) is induced in the presence of doxycycline.
